# EUROPEAN PATENT APPLICATION

(11) **EP 4 682 138 A1**
(43) Date of publication of application: **21.01.2026**
(21) Application number: 24770922.3
(22) Date of filing: 13.03.2024
(51) Int. Cl.: C07C 219/06, A61K 31/711, A61K 31/7105, A61K 38/02, A61K 45/00, A61K 47/18, A61K 47/28, A61K 47/44, A61K 48/00, A61P 43/00

(54) **COMPOUND OR SALT THEREOF, LIPID COMPOSITION, PHARMACEUTICAL COMPOSITION, AND DELIVERY CARRIER**

(30) Priority: 13.03.2023 JP 2023038537
(71) Applicant: FUJIFILM Corporation, Tokyo 106-8620 (JP)
(72) Inventor: NITABARU, Tatsuya, Ashigarakami-gun, Kanagawa 258-8577 (JP); YAMAMOTO, Masahiko, Ashigarakami-gun, Kanagawa 258-8577 (JP); TANABE, Shintaro, Ashigarakami-gun, Kanagawa 258-8577 (JP)
(74) Representative: Klunker IP Patentanwälte PartG mbB
(86) International application number: PCT/JP2024/009719
(87) International publication number: WO 2024/190817

(57) **Abstract**

An object of the present invention is to provide a compound or a salt thereof, which constitutes a lipid composition capable of realizing a high nucleic acid encapsulation rate and excellent nucleic acid delivery in a case where mRNA is used; and a lipid composition, a pharmaceutical composition, and a delivery carrier, which are capable of realizing a high nucleic acid encapsulation rate and excellent nucleic acid delivery in a case where mRNA is used. According to the present invention, a compound represented by Formula (1) or a salt thereof is provided.

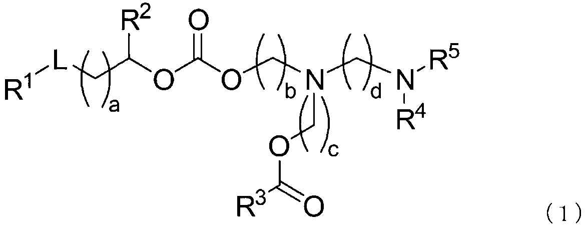

In the formula, R¹ represents a hydrocarbon group having 1 to 24 carbon atoms, R² represents a hydrocarbon group having 1 to 6 carbon atoms, R³ represents a hydrocarbon group having 6 to 24 carbon atoms and having a branched chain, R⁴ and R⁵ represent a hydrocarbon group having 1 to 6 carbon atoms, which may be substituted, R⁶ represents a hydrogen atom or a hydrocarbon group having 1 to 18 carbon atoms, L represents *-O-C(O)- or *-C(O)O-, a represents an integer of 1 to 12, and b, c, and d represent an integer of 1 to 4.

## Description

### Technical Field

The present invention relates to a compound or a salt thereof, which facilitates the introduction of a nucleic acid into a cell or the like. The present invention further relates to a lipid composition, a pharmaceutical composition, and a delivery carrier, which contain the above-described compound or a salt thereof.

### Background Art

Nucleic acid drugs have a clear mechanism of action on diseases, have few side effects, and are regarded as promising next-generation medicines. For example, a nucleic acid drug using small interfering RNA (siRNA) can inhibit the expression of a target gene in a cell in a sequence-specific manner. As a result, the nucleic acid drug can relieve or treat the diseases and symptoms caused by the abnormal expression of a specific gene or gene group. To express the function of these nucleic acids, the nucleic acid drugs need to be delivered into cells.

One of the methods for efficiently delivering nucleic acids into cells is a method using a viral vector such as a retrovirus or an adenovirus. The method using a viral vector brings a high gene introduction efficiency. However, the size of genes to be introduced by this method is limited, and there is a concern over immunogenicity and safety of this method. On the other hand, in a case where a lipid composition is used for gene introduction, any gene can be introduced without limitation, and the above issues can be solved. Therefore, the lipid composition is being developed vigorously.

Patent Document 1 describes an amino lipid represented by a predetermined formula (1) or a salt thereof, and also describes a lipid composition containing the amino lipid or a salt thereof. Patent Document 1 describes delivering a nucleic acid to mouse cells using the above-described lipid composition.

Patent Document 2 describes a lipid composition containing an amino lipid represented by a predetermined formula (1) or a salt thereof, a nonionic lipid, a lipid having a nonionic hydrophilic polymer structure, and a nucleic acid, and containing or not containing a zwitterionic lipid. Patent Document 2 describes delivering a nucleic acid to mouse cells using the above-described lipid composition.

Patent Document 3 describes a lipid composition containing a first lipid which is a lipid represented by Formula (1) or a salt thereof, sterols, and nucleic acid, in which a ratio of the number of moles of the first lipid in the lipid composition to the number of moles of sterols in the lipid composition is 0.300 or more and less than 1.299. Patent Document 3 describes delivering a nucleic acid to mouse cells using the above-described lipid composition.

### Prior Art Documents

### Patent Documents

Patent Document 1: WO2019/235635A
Patent Document 2: WO2020/246581A
Patent Document 3: WO2021/095876A

### Summary of Invention

### Object to be solved by the invention

In the related art, it has been difficult to express proteins in a case of using mRNA and to produce proteins in a dose-dependent manner at high doses. An object to be solved by the present invention is to provide a compound or a salt thereof, which constitutes a lipid composition capable of realizing a high nucleic acid encapsulation rate and excellent nucleic acid delivery in a case where mRNA is used. Furthermore, an object to be solved of the present invention is to provide a lipid composition, a pharmaceutical composition, and a delivery carrier, which can realize a high nucleic acid encapsulation rate and excellent nucleic acid delivery in a case where mRNA is used, using the compound or a salt thereof.

### Means for solving the object

To solve the above object, the inventors of the present invention have conducted intensive studies. As a result, the inventors have found that a lipid composition prepared using a compound represented by Formula (1) or a salt thereof can realize a high nucleic acid encapsulation rate and excellent nucleic acid delivery in a case where mRNA is used. Based on the finding, the inventors have accomplished the present invention. According to the present invention, the following inventions are provided.
<1> A compound represented by Formula (1) or a salt thereof, 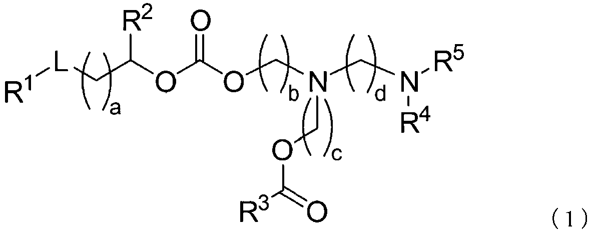
   in the formula, R¹ represents a hydrocarbon group having 1 to 24 carbon atoms,
   R² represents a hydrocarbon group having 1 to 6 carbon atoms,
   R³ represents a hydrocarbon group having 6 to 24 carbon atoms and having a branched chain,
   R⁴ and R⁵ each independently represent a hydrocarbon group having 1 to 6 carbon atoms, which may be substituted, and substituents on the hydrocarbon group having 1 to 6 carbon atoms, which may be substituted, represented by R⁴ and R⁵ each independently represent -O-R⁶, where R⁶ represents a hydrogen atom or a hydrocarbon group having 1 to 18 carbon atoms,
   L is absent or represents *-O-C(O)- or *-C(O)O-, where * represents a bonding position to R¹,
   a represents an integer of 1 to 12,
   b represents an integer of 1 to 4,
   c represents an integer of 1 to 4, and
   d represents an integer of 1 to 4.
<2> The compound or a salt thereof according to <1>,
   in which R¹ represents a hydrocarbon group having 6 to 14 carbon atoms,
   R² represents a hydrocarbon group having 6 carbon atoms,
   R³ represents a hydrocarbon group having 11 to 17 carbon atoms and having a branched chain,
   R⁴ and R⁵ each independently represent a hydrocarbon group having 2 to 4 carbon atoms which may be substituted, and substituents on the hydrocarbon group having 2 to 4 carbon atoms which may be substituted, represented by R⁴ and R⁵, each independently represent -O-R⁶, where R⁶ represents a hydrogen atom or a benzyl group,
   L represents *-O-C(O)-, where * represents a bonding position to R¹,
   a represents an integer of 3 to 8,
   b represents 2,
   c represents 2, and
   d represents 2.
<3> The compound or a salt thereof according to <2>, in which R¹ represents a linear hydrocarbon group having 6 to 8 carbon atoms.
<4> A compound or a salt thereof selected from the following compounds:
   octyl
      6-(2-((2-butyloctanoyl)oxy)ethyl)-3-ethyl-12-hexyl-10-oxo-9,11-dioxa-3,6-diazahenicosan-21 -oate 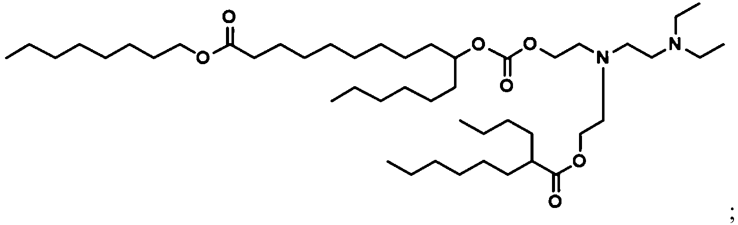
   hexyl
      3-ethyl-12-hexyl-6-(2-((2-hexyloctanoyl)oxy)ethyl)-10-oxo-9,11-dioxa-3,6-diazahenicosan-21 -oate 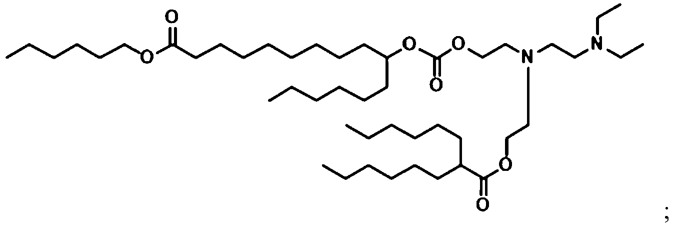
   heptyl
      3-ethyl-6-(2-((2-heptylnonanoyl)oxy)ethyl)-12-hexyl-10-oxo-9,11-dioxa-3,6-diazahexadecan-16-oate 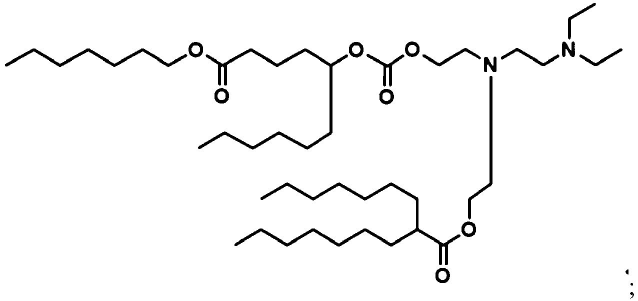
   heptyl
      3-ethyl-12-hexyl-6-(2-((2-octyldecanoyl)oxy)ethyl)-10-oxo-9,11-dioxa-3,6-diazahexadecan-1 6-oate 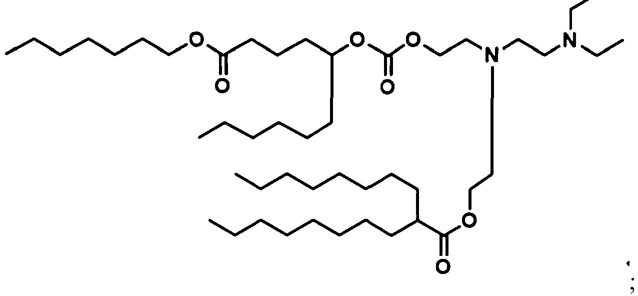
   heptyl
      3-ethyl-12-hexyl-6-(2-((2-hexyloctanoyl)oxy)ethyl)-10-oxo-9,11-dioxa-3,6-diazahexadecan-1 6-oate 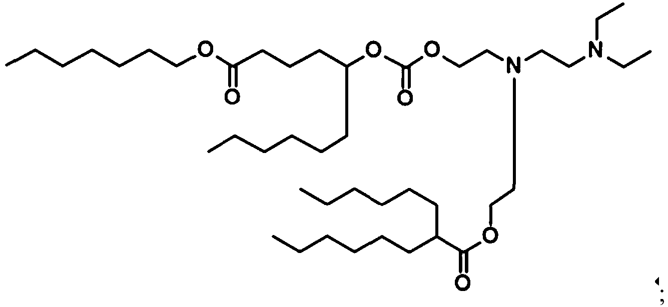
   heptyl
      3-ethyl-6-(2-((3-heptyldecanoyl)oxy)ethyl)-12-hexyl-10-oxo-9,11-dioxa-3,6-diazahexadecan-16-oate 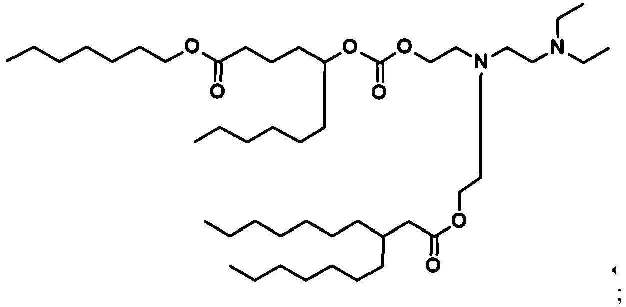
   octyl
      3-ethyl-6-(2-((2-heptylnonanoyl)oxy)ethyl)-12-hexyl-10-oxo-9,11-dioxa-3,6-diazahexadecan-16-oate 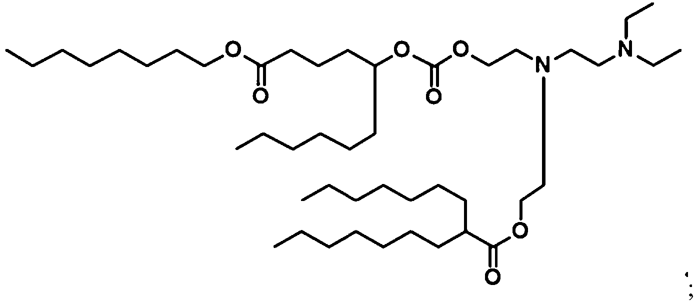
   octyl
      3-ethyl-6-(2-((3-heptyldecanoyl)oxy)ethyl)-12-hexyl-10-oxo-9,11-dioxa-3,6-diazahexadecan-16-oate 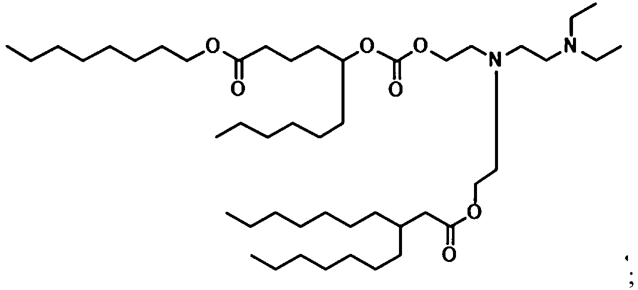
   2-pentylheptyl
      3-ethyl-6-(2-((3-heptyldecanoyl)oxy)ethyl)-12-hexyl-10-oxo-9,11-dioxa-3,6-diazahexadecan-16-oate 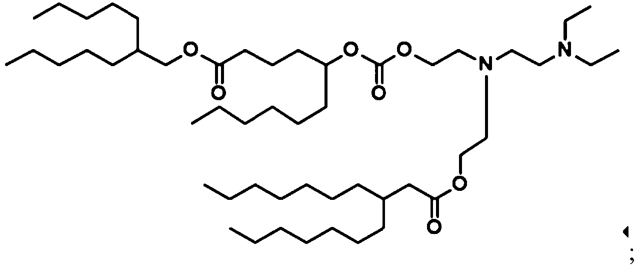
   2-hexyloctyl
      3-ethyl-6-(2-((3-heptyldecanoyl)oxy)ethyl)-12-hexyl-10-oxo-9,11-dioxa-3,6-diazahexadecan-16-oate 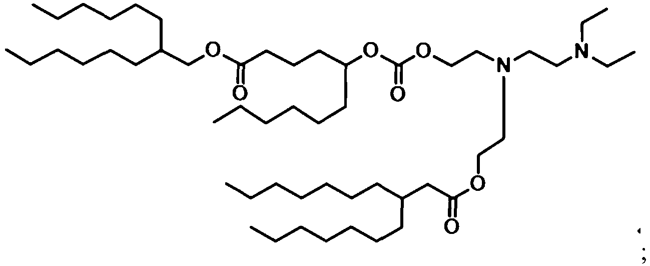
   octyl
      5-ethyl-8-(2-((3-heptyldecanoyl)oxy)ethyl)-14-hexyl-12-oxo-1-phenyl-2,11,13-trioxa-5,8-diaz aoctadecan-18-oate 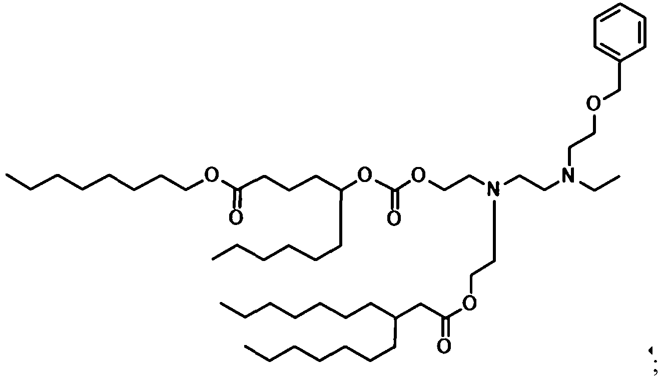
   octyl
      6-ethyl-9-(2-((3-heptyldecanoyl)oxy)ethyl)-15-hexyl-13-oxo-1-phenyl-2,12,14-trioxa-6,9-diaz anonadecan-19-oate 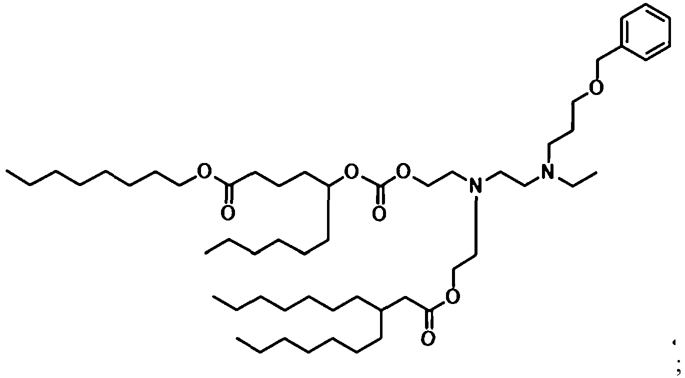
   octyl
      7-ethyl-10-(2-((3-heptyldecanoyl)oxy)ethyl)-16-hexyl-14-oxo-1-phenyl-2,13,15-trioxa-7,10-di azaicosan-20-oate 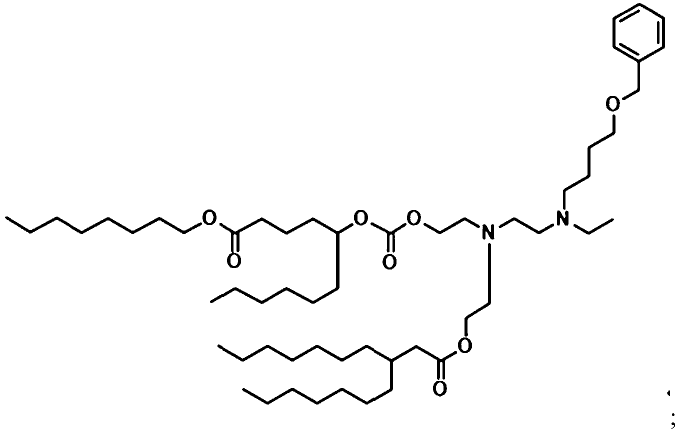
   octyl
      3-ethyl-6-(2-((3-heptyldecanoyl)oxy)ethyl)-12-hexyl-1-hydroxy-10-oxo-9,11-dioxa-3,6-diazah exadecan-16-oate 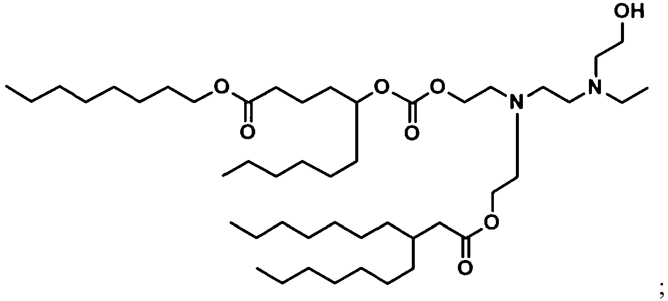
   octyl
      4-ethyl-7-(2-((3-heptyldecanoyl)oxy)ethyl)-13-hexyl-1-hydroxy-11-oxo-10,12-dioxa-4,7-diaza heptadecan-17-oate 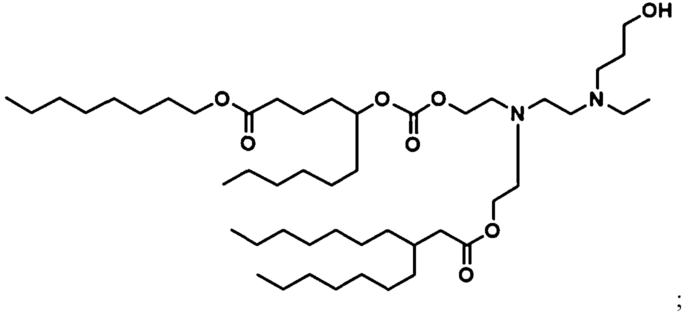
   octyl
      5-ethyl-8-(2-((3-heptyldecanoyl)oxy)ethyl)-14-hexyl-1-hydroxy-12-oxo-11,13-dioxa-5,8-diaza octadecan-18-oate 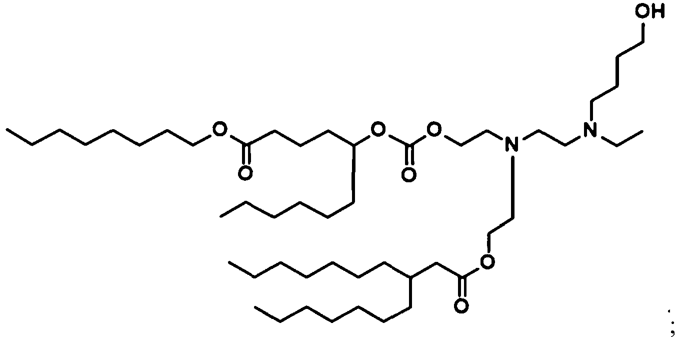
   heptyl
      3-ethyl-12-hexyl-6-(2-((2-nonylundecanoyl)oxy)ethyl)-10-oxo-9,11-dioxa-3,6-diazahexadecan -16-oate 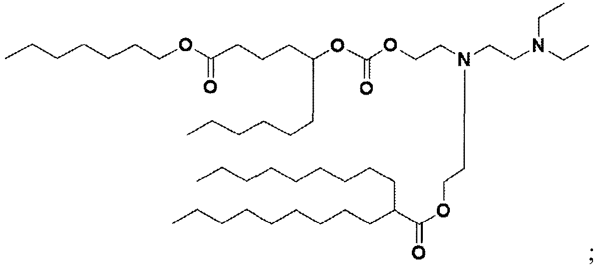
   heptyl
      6-(2-((2-decyldodecanoyl)oxy)ethyl)-3-ethyl-12-hexyl-10-oxo-9,11-dioxa-3,6-diazahexadecan -16-oate 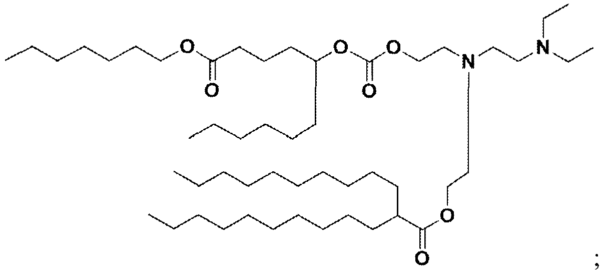
   heptyl
      3-ethyl-12-hexyl-6-(2-((2-hexyldecanoyl)oxy)ethyl)-10-oxo-9,11-dioxa-3,6-diazahexadecan-1 6-oate 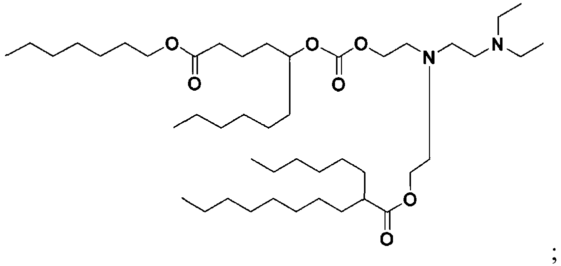
   heptyl
      3-ethyl-13-hexyl-7-(2-((2-hexyloctanoyl)oxy)ethyl)-11-oxo-10,12-dioxa-3,7-diazaheptadecan-17-oate 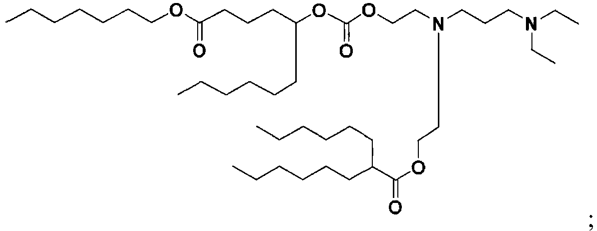
   heptyl
      3-ethyl-13-hexyl-7-(2-((2-hexyloctanoyl)oxy)ethyl)-11-oxo-10,12-dioxa-3,7-diazaoctadecan-1 8-oate 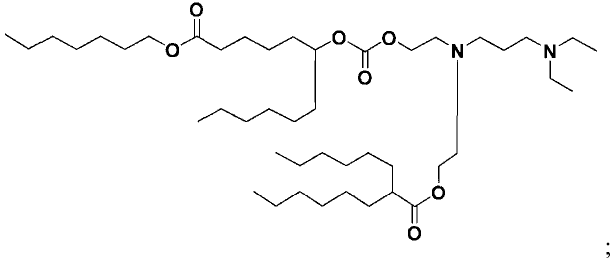
   heptyl
      3-ethyl-7-(2-((2-heptylnonanoyl)oxy)ethyl)-13-hexyl-11-oxo-10,12-dioxa-3,7-diazaoctadecan-18-oate 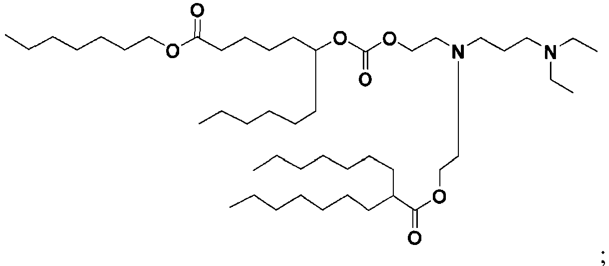
   heptyl
      3-ethyl-13-hexyl-7-(2-((2-octyldecanoyl)oxy)ethyl)-11-oxo-10,12-dioxa-3,7-diazaoctadecan-1 8-oate 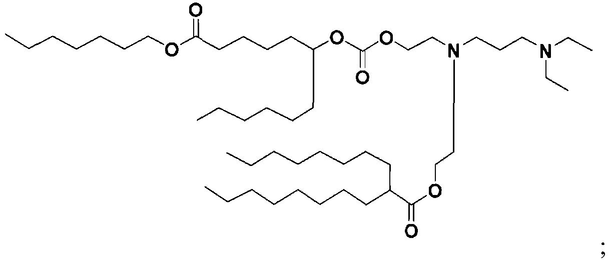
   heptyl
      3-ethyl-13-hexyl-7-(2-((2-hexyldecanoyl)oxy)ethyl)-11-oxo-10,12-dioxa-3,7-diazaoctadecan-18-oate 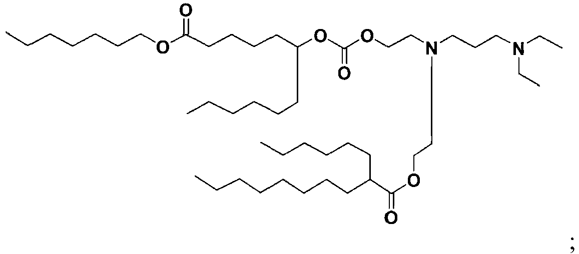
   heptyl
      3-ethyl-7-(2-((3-heptyldecanoyl)oxy)ethyl)-13-hexyl-11-oxo-10,12-dioxa-3,7-diazaoctadecan-18-oate 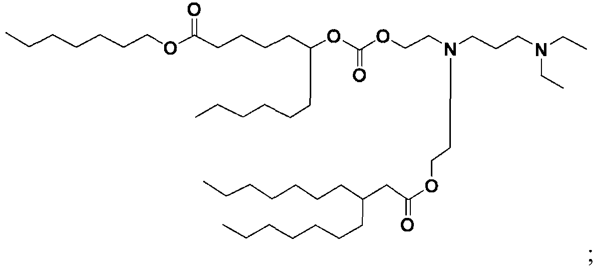
   heptyl
      3-ethyl-13-hexyl-6-(3-((2-hexyloctanoyl)oxy)propyl)-11-oxo-10,12-dioxa-3,6-diazaoctadecan-18-oate 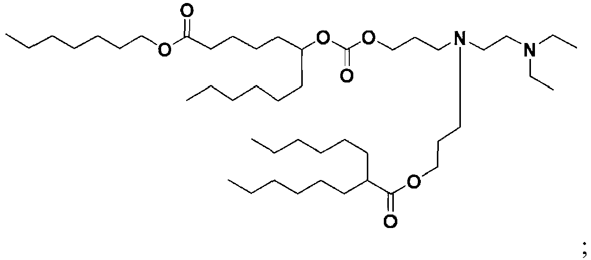
   heptyl
      3-ethyl-6-(3-((2-heptylnonanoyl)oxy)propyl)-13-hexyl-11-oxo-10,12-dioxa-3,6-diazaoctadeca n-18-oate 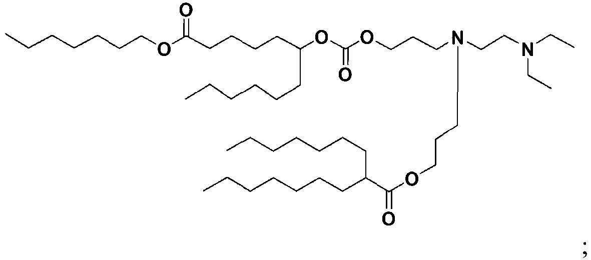
   heptyl
      3-ethyl-13-hexyl-6-(3-((2-octyldecanoyl)oxy)propyl)-11-oxo-10,12-dioxa-3,6-diazaoctadecan-18-oate 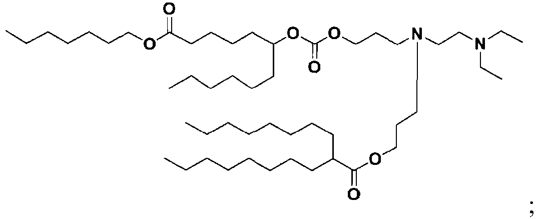
   heptyl
      3-ethyl-13-hexyl-6-(3-((2-nonylundecanoyl)oxy)propyl)-11-oxo-10,12-dioxa-3,6-diazaoctadec an-18-oate 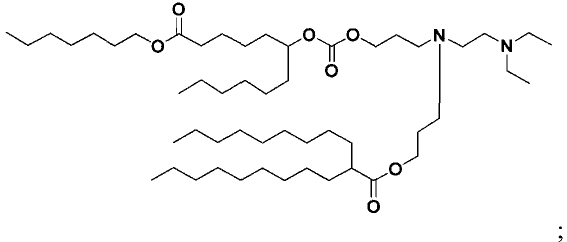
   heptyl
      3-ethyl-13-hexyl-6-(3-((2-hexyldecanoyl)oxy)propyl)-11-oxo-10,12-dioxa-3,6-diazaoctadecan -18-oate 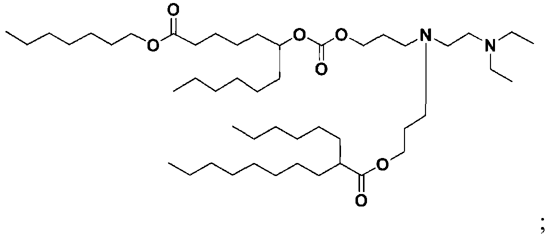
   heptyl
      3-ethyl-6-(3-((3-heptyldecanoyl)oxy)propyl)-13-hexyl-11-oxo-10,12-dioxa-3,6-diazaoctadeca n-18-oate 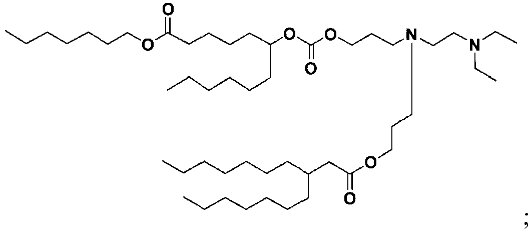
   heptyl
      3-ethyl-14-hexyl-7-(3-((2-hexyloctanoyl)oxy)propyl)-12-oxo-11,13-dioxa-3,7-diazanonadecan -19-oate 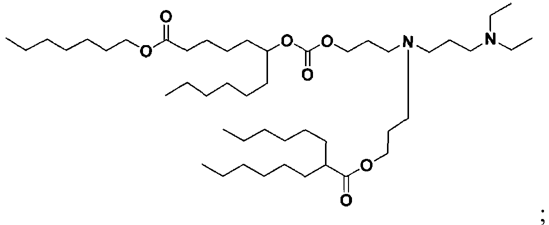
   heptyl
      3-ethyl-7-(3-((2-heptylnonanoyl)oxy)propyl)-14-hexyl-12-oxo-11,13-dioxa-3,7-diazanonadeca n-19-oate 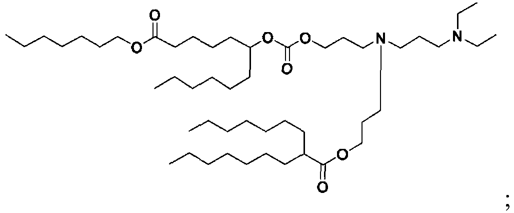
   heptyl
      3-ethyl-14-hexyl-7-(3-((2-octyldecanoyl)oxy)propyl)-12-oxo-11,13-dioxa-3,7-diazanonadecan -19-oate 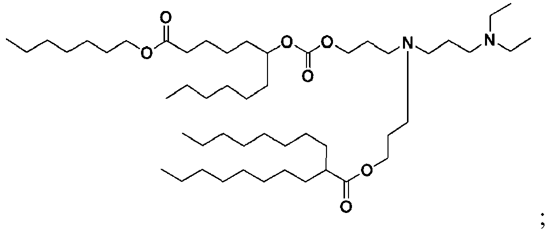
   heptyl
      3-ethyl-14-hexyl-7-(3-((2-nonylundecanoyl)oxy)propyl)-12-oxo-11,13-dioxa-3,7-diazanonade can-19-oate 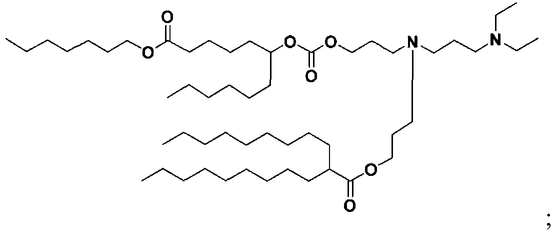
   heptyl
      3-ethyl-14-hexyl-7-(3-((2-hexyldecanoyl)oxy)propyl)-12-oxo-11,13-dioxa-3,7-diazanonadeca n-19-oate 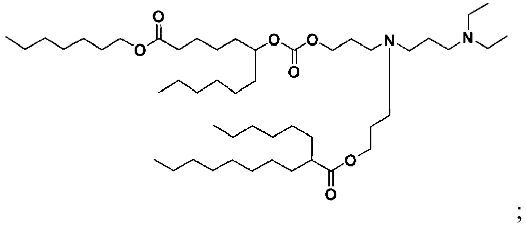 and
   heptyl
      3-ethyl-7-(3-((3-heptyldecanoyl)oxy)propyl)-14-hexyl-12-oxo-11,13-dioxa-3,7-diazanonadeca n-19-oate 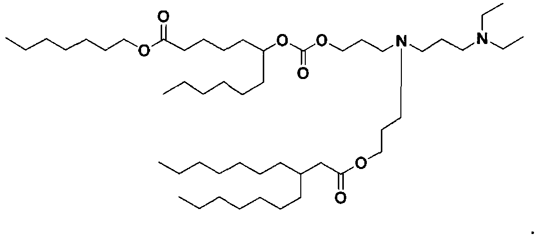
<5> A **lipid** composition comprising:
   the compound or a salt thereof according to any one of <1> to <4>; and
   a lipid.
<6> The lipid composition according to <5>, in which the lipid is at least one lipid selected from the group consisting of a neutral lipid and a lipid having a nonionic hydrophilic polymer chain.
<7> The lipid composition according to <5> or <6>, further comprising a sterol.
<8> The lipid composition according to any one of <5> to <7>, further comprising:
   at least one selected from the group consisting of a nucleic acid, a protein, a peptide, and a low-molecular-weight compound.
<9> A pharmaceutical composition comprising:
   the lipid composition according to any one of <5> to <8>.
<10> A delivery carrier comprising:
   the lipid composition according to any one of <5> to <8>.

### Effect of the invention

By using the compound according to the embodiment of the present invention, it is possible to produce a lipid composition, a pharmaceutical composition, and a delivery carrier, which are capable of realizing a high nucleic acid encapsulation rate and excellent nucleic acid delivery in a case where mRNA is used. The lipid composition, the pharmaceutical composition, and the delivery carrier according to the embodiment of the present invention can realize a high nucleic acid encapsulation rate and excellent nucleic acid delivery in a case where mRNA is used.

### BRIEF DESCRIPTION OF THE DRAWINGS

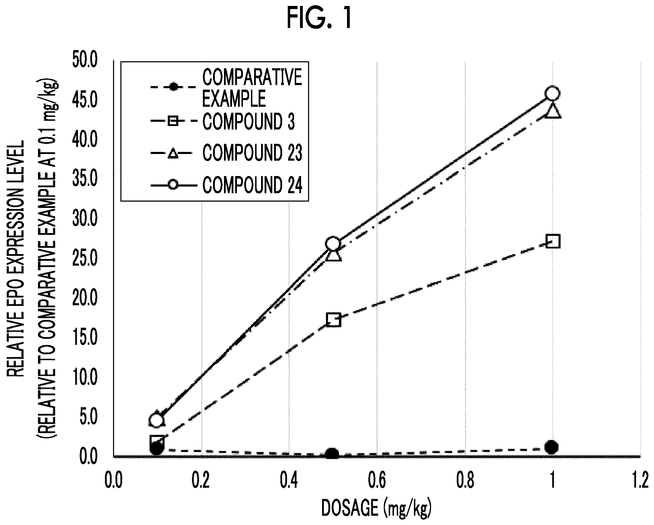

Fig. 1 shows an erythropoietin (EPO) expression level (dose dependency) of nucleic acid-encapsulating lipid nanoparticles.

### Embodiments for carrying out the invention

Hereinafter, the present invention is specifically described.

In the present specification, "to" shows a range including numerical values described before and after "to" as a minimum value and a maximum value, respectively.

### <Compound according to embodiment of present invention>

The present invention relates to a compound represented by Formula (1) or a salt thereof. By using the compound represented by Formula (1) or a salt thereof as a constituent component of the lipid composition, a high nucleic acid encapsulation rate and excellent nucleic acid delivery in a case where mRNA is used. Furthermore, according to the present invention, it is possible to realize protein production corresponding to a high dose. The dose dependency can also be achieved.

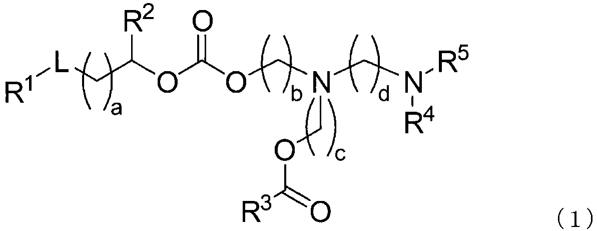

In the formula, R¹ represents a hydrocarbon group having 1 to 24 carbon atoms,
R² represents a hydrocarbon group having 1 to 6 carbon atoms,
R³ represents a hydrocarbon group having 6 to 24 carbon atoms and having a branched chain,
R⁴ and R⁵ each independently represent a hydrocarbon group having 1 to 6 carbon atoms, which may be substituted, and substituents on the hydrocarbon group having 1 to 6 carbon atoms, which may be substituted, represented by R⁴ and R⁵ each independently represent -O-R⁶, where R⁶ represents a hydrogen atom or a hydrocarbon group having 1 to 18 carbon atoms,
L is absent or represents *-O-C(O)- or *-C(O)O-, where * represents a bonding position to R¹,
a represents an integer of 1 to 12,
b represents an integer of 1 to 4,
c represents an integer of 1 to 4, and
d represents an integer of 1 to 4.

R¹ preferably represents a hydrocarbon group having 3 to 18 carbon atoms, more preferably represents a hydrocarbon group having 6 to 14 carbon atoms, and still more preferably represents a hydrocarbon group having 6 to 8 carbon atoms.

R² preferably represents a hydrocarbon group having 3 to 6 carbon atoms, more preferably represents a hydrocarbon group having 4 to 6 carbon atoms, and particularly preferably represents a hydrocarbon group having 6 carbon atoms.

R³ preferably represents a hydrocarbon group having 8 to 20 carbon atoms and having a branched chain, and more preferably represents a hydrocarbon group having 11 to 17 carbon atoms and having a branched chain.

The "hydrocarbon group having 6 to 24 carbon atoms and having a branched chain" represented by R³ is preferably a group represented by R³¹(R³²)CH- or R³³(R³⁴)CH-CH₂-.

R³¹ and R³² each independently represent a hydrocarbon group, and the total number of carbon atoms in the hydrocarbon groups represented by R³¹ and R³² is 5 to 23. R³¹ and R³² each independently preferably represent a linear hydrocarbon group, and more preferably represent a linear alkyl group.

The number of carbon atoms in the hydrocarbon group represented by R³¹ and R³² may be the same as or different from each other. The difference in the number of carbon atoms between the hydrocarbon groups represented by R³¹ and R³² is preferably 5 or less, more preferably 3 or less, and still more preferably 1 or less. It is preferable that the number of carbon atoms in the hydrocarbon group represented by R³¹ and R³² is the same.

R³¹ and R³² are each independently preferably a linear hydrocarbon group (preferably an alkyl group) having 4 to 10 carbon atoms, more preferably a linear hydrocarbon group (preferably an alkyl group) having 4 to 8 carbon atoms, and still more preferably a linear hydrocarbon group (preferably an alkyl group) having 6 to 8 carbon atoms.

R³³ and R³⁴ each independently represent a hydrocarbon group, and the total number of carbon atoms in the hydrocarbon groups represented by R³³ and R³⁴ is 4 to 22. R³³ and R³⁴ each independently preferably represent a linear hydrocarbon group, and more preferably represent a linear alkyl group.

The number of carbon atoms in the hydrocarbon group represented by R³³ and R³⁴ may be the same as or different from each other. The difference in the number of carbon atoms between the hydrocarbon groups represented by R³³ and R³⁴ is preferably 5 or less, more preferably 3 or less, and still more preferably 1 or less. It is preferable that the number of carbon atoms in the hydrocarbon group represented by R³³ and R³⁴ is the same.

R³³ and R³⁴ are each independently preferably a linear hydrocarbon group (preferably an alkyl group) having 4 to 10 carbon atoms, more preferably a linear hydrocarbon group (preferably an alkyl group) having 4 to 8 carbon atoms, still more preferably a linear hydrocarbon group (preferably an alkyl group) having 6 to 8 carbon atoms, and particularly preferably a linear hydrocarbon group (preferably an alkyl group) having 7 carbon atoms.

R⁴ and R⁵ each independently preferably represent a hydrocarbon group having 2 to 4 carbon atoms, which may be substituted.

The substituents on the hydrocarbon group having 1 to 6 carbon atoms, which may be substituted, represented by R⁴ and R⁵ each independently represents -O-R⁶, where R⁶ preferably represents a hydrogen atom or a hydrocarbon group having 1 to 12 carbon atoms, more preferably represents a hydrogen atom or a hydrocarbon group having 7 to 12 carbon atoms, and particularly preferably represents a hydrogen atom or a benzyl group.

L preferably represents *-O-C(O)-, where * represents a bonding position to R¹.
a preferably represents an integer of 2 to 10 and more preferably an integer of 3 to 8.
b preferably represents 2 or 3 and more preferably represents 2.
c preferably represents 2 or 3 and more preferably represents 2.
d preferably represents 2 or 3 and more preferably represents 2.

In Formula (1), preferably,
R¹ represents a hydrocarbon group having 6 to 14 carbon atoms,
R² represents a hydrocarbon group having 6 carbon atoms,
R³ represents a hydrocarbon group having 11 to 17 carbon atoms and having a branched chain,
R⁴ and R⁵ each independently represent a hydrocarbon group having 2 to 4 carbon atoms which may be substituted, and substituents on the hydrocarbon group having 2 to 4 carbon atoms which may be substituted, represented by R⁴ and R⁵, each independently represent -O-R⁶, where R⁶ represents a hydrogen atom or a benzyl group,
L represents *-O-C(O)-, where * represents a bonding position to R¹,
a represents an integer of 3 to 8,
b represents 2,
c represents 2, and
d represents 2.

In Formula (1), R¹ more preferably represents a linear hydrocarbon group having 6 to 8 carbon atoms.

The hydrocarbon group is preferably an alkyl group, an alkenyl group, or an alkynyl group.

The alkyl group may be linear or branched, or may be chainlike or cyclic. Specifically, examples of the alkyl group include a methyl group, an ethyl group, a propyl group, an isopropyl group, a cyclopropyl group, a butyl group, an isobutyl group, a tert-butyl group, a cyclobutyl group, a pentyl group, a cyclopentyl group, a hexyl group, a cyclohexyl group, a heptyl group, an octyl group, a nonyl group, a decyl group, an undecyl group, a dodecyl group, a tridecyl group, a trimethyldodecyl group (preferably a 3,7,11-trimethyldodecyl group), a tetradecyl group, a pentadecyl group, a hexadecyl group, a tetramethylhexadecyl group (preferably a 3,7,11,15-tetramethylhexadecyl group), a heptadecyl group, an octadecyl group, a 2-butylhexyl group, a 2-butyloctyl group, a 1-pentylhexyl group, a 2-pentylheptyl group, a 3-pentyloctyl group, a 1-hexylheptyl group, a 1-hexylnonyl group, a 2-hexyloctyl group, a 2-hexyldecyl group, a 3-hexylnonyl group, a 1-heptyloctyl group, a 2-heptylnonyl group, a 2-heptylundecyl group, a 3-heptyldecyl group, a 1-octylnonyl group, a 2-octyldecyl group, a 2-octyldodecyl group, a 3-octylundecyl group, a 2-nonylundecyl group, a 3-nonyldodecyl group, a 2-decyldodecyl group, a 2-decyltetradecyl group, a 3-decyltridecyl group, a 2-(4,4-dimethylpentan-2-yl)-5,7,7-trimethyloctyl group, and the like.

The alkenyl group may be linear or branched, or may be chainlike or cyclic. Specifically, examples of the alkenyl group include an allyl group, a prenyl group, a pentenyl group, a hexenyl group, a heptenyl group, an octenyl group, a nonenyl group (preferably a (Z)-2-nonenyl group or an (E)-2-nonenyl group), a decenyl group, an undecenyl group, a dodecenyl group, a dodecadienyl group, a tridecenyl group (preferably a (Z)-tridec-8-enyl group), a tetradecenyl group (preferably a tetradec-9-enyl group), a pentadecenyl group (preferably a (Z)-pentadec-8-enyl group), a hexadecenyl group (preferably a (Z)-hexadec-9-enyl group), a hexadecadienyl group, a heptadecenyl group (preferably a (Z)-heptadec-8-enyl group), a heptadecadienyl group (preferably a (8Z,11Z)-heptadeca-8,11-dienyl group), an octadecenyl group (preferably a (Z)-octadec-9-enyl group), an octadecadienyl group (preferably a (9Z,12Z)-octadeca-9,12-dienyl group), and the like.

The alkynyl group may be linear or branched, or may be chainlike or cyclic. Specifically, examples of alkynyl group include a propargyl group, a butynyl group, a pentynyl group, a hexynyl group, a heptynyl group, an octynyl group, a nonynyl group, a decynyl group, an undecynyl group, a dodecynyl group, a tetradecynyl group, a pentadecynyl group, a hexadecynyl group, a heptadecynyl group, an octadecynyl group, and the like.

All of the above alkenyl groups preferably have one double bond or two double bonds. All of the above alkynyl groups preferably have one triple bond or two triple bonds.

The compound according to the embodiment of the present invention may form a salt.

Examples of the salt in a basic group include salts with mineral acids such as hydrochloric acid, hydrobromic acid, nitric acid, and sulfuric acid; salts with organic carboxylic acids such as formic acid, acetic acid, citric acid, oxalic acid, fumaric acid, maleic acid, succinic acid, malic acid, tartaric acid, aspartic acid, trichloroacetic acid, and trifluoroacetic acid; and salts with sulfonic acids such as methanesulfonic acid, benzenesulfonic acid, p-toluenesulfonic acid, mesitylenesulfonic acid, and naphthalenesulfonic acid.

Examples of the salt in an acidic group include salts with alkali metals such as sodium and potassium; salts with alkaline earth metals such as calcium and magnesium; ammonium salts; salts with nitrogen-containing organic bases such as trimethylamine, triethylamine, tributylamine, pyridine, N,N-dimethylaniline, N-methylpiperidine, N-methylmorpholine, diethylamine, dicyclohexylamine, procaine, dibenzylamine, N-benzyl-β-phenethylamine, 1-ephenamine, and N,N'-dibenzylethylenediamine; and the like.

Among the above-described salts, for example, pharmacologically acceptable salts are preferable.

Specifically, as the compound according to the embodiment of the present invention, for example, Compounds 1 to 37 described in Examples which will be described later are preferable. However, the present invention is not limited thereto.

Among Compounds 1 to 16, Compound 1, Compound 2, Compound 3, Compound 4, Compound 5, Compound 6, Compound 7, Compound 8, Compound 9, and Compound 10 are preferable. Furthermore, Compound 3, Compound 4, Compound 5, Compound 6, Compound 7, Compound 8, and Compound 9 are preferable, and particularly, Compound 3, Compound 4, Compound 6, Compound 7, and Compound 8 are preferable.

Among Compounds 17 to 37, Compounds 17 to 25 are preferable. Furthermore, Compound 19 and Compounds 22 to 25 are preferable.

### <Manufacturing method>

The manufacturing method of the compound according to the embodiment of the present invention is described.

The compound according to the embodiment of the present invention can be manufactured by a combination of known methods. For example, the compound can be manufactured by the following manufacturing method.

### [Manufacturing method 1]

A method for manufacturing the compound represented by Formula [1] from the compound represented by Formula [2].

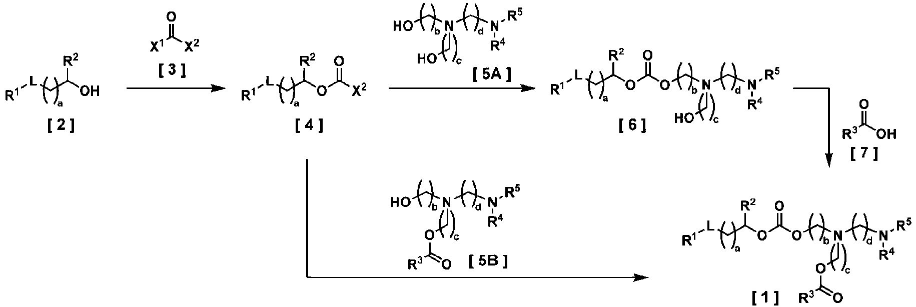

In the formula, R¹, R², R³, R⁴, R⁵, L, a, b, c, and d have the same meanings as above; and X¹ and X² each represent a leaving group.

Examples of the leaving group include a chloro group, a fluoro group, a bromo group, a trichloromethoxy group, a 4-nitro-phenoxy group, a 2,4-dinitrophenoxy group, a 2,4,6-trichlorophenoxy group, a pentafluorophenoxy group, a 2,3,5,6-tetrafluorophenoxy group, an imidazolyl group, a triazolyl group, a 3,5-dioxo-4-methyl-1,2,4-oxadiazolidinyl group, an N-hydroxysuccinimidyl group, a methanesulfonyl group, a 4-toluenesulfonyl group, and the like.

(1-1)
The compound represented by Formula [4] can be manufactured by reacting the compound represented by Formula [2] with the compound represented by Formula [3] in the presence or absence of a base.

As the compound represented by Formula [3], for example, 1,1'-carbonyldi(1,2,4-triazole), 1,1'-carbonyldiimidazole, 4-nitrophenyl chloroformate, triphosgene, phosgene, and the like are known.

The solvent used in this reaction is not particularly limited as long as the solvent does not affect the reaction. Examples of the solvent include halogenated hydrocarbons, ethers, esters, amides, nitriles, sulfoxides, and aromatic hydrocarbons. These solvents may be used by being mixed together.

Preferred examples of the solvent include ethers, and tetrahydrofuran is more preferable.

The used amount of the solvent is not particularly limited, but is only required to be 1 to 500 times (v/w) the amount of the compound represented by Formula [2].

Examples of the base used in this reaction include an inorganic base and an organic base. As the base, an organic base is preferable. Specifically, examples thereof include triethylamine, N,N-diisopropylethylamine, 4-methylmorpholine, pyridine, 1,8-diazabicyclo[5.4.0]-7-undecene, 4-dimethylaminopyridine, and the like.

The used amount of the base is only required to be 1 to 50 times and preferably 1 to 10 times the molar amount of the compound represented by Formula [2].

The used amount of the compound represented by Formula [3] is not particularly limited, but is only required to be 1 to 10 times the molar amount of the compound represented by Formula [2].

This reaction is only required to carry out at -30°C to 150°C, preferably at 0°C to 100°C for 5 minutes to 48 hours.

(1-2)
The compound represented by Formula [6] can be manufactured by reacting the compound represented by Formula [4] with the compound represented by Formula [5A] in the presence of a base.

The solvent used in this reaction is not particularly limited as long as the solvent does not affect the reaction. Examples of the solvent include halogenated hydrocarbons, ethers, esters, amides, nitriles, sulfoxides, and aromatic hydrocarbons. These solvents may be used by being mixed together.

Preferred examples of the solvent include ethers, and tetrahydrofuran is more preferable.

The used amount of the solvent is not particularly limited, but is only required to be 1 to 500 times (v/w) the amount of the compound represented by Formula [4].

Examples of the base used in this reaction include an inorganic base and an organic base. Specifically, examples thereof include potassium carbonate, sodium carbonate, lithium carbonate, potassium phosphate, sodium phosphate, lithium phosphate, triethylamine, N,N-diisopropylethylamine, 4-methylmorpholine, pyridine, 1,8-diazabicyclo[5.4.0]-7-undecene, 4-dimethylaminopyridine, and the like. These bases may be used in combination.

The used amount of the base is only required to be 1 to 50 times and preferably 1 to 10 times the molar amount of the compound represented by Formula [4].

The used amount of the compound represented by Formula [5A] is not particularly limited, but is only required to be 0.5 to 10 times the molar amount of the compound represented by Formula [4].

This reaction is only required to carry out at -30°C to 150°C, preferably at 0°C to 100°C for 5 minutes to 48 hours.

(1-3)
The compound represented by Formula [1] can be manufactured by reacting the compound represented by Formula [6] with the compound represented by Formula [7] in the presence or absence of an acid, in the presence or absence of a condensing agent or an acid halide, and in the presence or absence of a base.

The solvent used in this reaction is not particularly limited as long as the solvent does not affect the reaction. Examples of the solvent include halogenated hydrocarbons, ethers, esters, amides, nitriles, sulfoxides, and aromatic hydrocarbons. These solvents may be used by being mixed together.

Preferred examples of the solvent include halogenated hydrocarbons, ethers, and aromatic hydrocarbons, and dichloromethane, tetrahydrofuran, and toluene are more preferable.

The used amount of the solvent is not particularly limited, but is only required to be 1 to 500 times (v/w) the amount of the compound represented by Formula [6].

Examples of the acid used in this reaction include an inorganic acid and an organic acid. As the acid, sulfonic acids are preferable. Specifically, examples thereof include sulfuric acid, 4-toluenesulfonic acid, methanesulfonic acid, and the like.

Examples of the condensing agent used in this reaction include carbodiimides such as N,N'-dicyclohexylcarbodiimide and 1-ethyl-3-(3-dimethylaminopropyl)carbodiimide; carbonyls such as carbonyldiimidazole; acid azides such as diphenylphosphoryl azide; acid cyanides such as diethylphosphoryl cyanide; uroniums such as 2-ethoxy-1-ethoxycarbonyl-1,2-dihydroquinoline; O-benzotriazol-1-yl-1,1,3,3-tetramethyluronium=hexafluorophosphate; O-(7-azabenzotriazol-1-yl)-1,1,3,3-tetramethyluronium=hexafluorophosphate; and the like.

Examples of the acid halide used in this reaction include carboxylic acid halides such as acetyl chloride and trifluoroacetyl chloride; sulfonic acid halides such as methanesulfonyl chloride and tosyl chloride; chloroformic acid esters such as ethyl chloroformate and isobutyl chloroformate; and the like.

Examples of the base used in this reaction include an inorganic base and an organic base. An organic base is preferable. Specifically, examples thereof include triethylamine, N,N-diisopropylethylamine, 4-methylmorpholine, pyridine, 1,8-diazabicyclo[5.4.0]-7-undecene, 4-dimethylaminopyridine, and the like. These bases may be used in combination.

The used amount of the base is only required to be 1 to 50 times and preferably 1 to 10 times the molar amount of the compound represented by Formula [6].

The used amount of the compound represented by Formula [7] is not particularly limited, but is only required to be 0.8 to 10 times the molar amount of the compound represented by Formula [6].

This reaction is only required to carry out at -30°C to 150°C, preferably at 0°C to 100°C for 5 minutes to 48 hours.

(1-4)
The compound represented by Formula [1] can be manufactured in the same method as in the manufacturing method 1 (1-2) using the compound represented by Formula [4] and the compound represented by Formula [5B] as raw materials.

### [Manufacturing method 2]

A method for manufacturing the compound represented by Formula [2] from the compound represented by Formula [8].

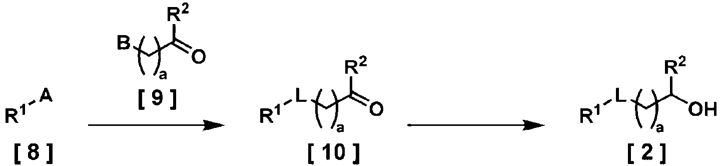

In the formulae, R¹, R², L, and a have the same meanings as above; and A and B represent a hydroxyl group or a carboxyl group.

(2-1)
The compound represented by Formula [10] can be manufactured by reacting the compound represented by Formula [8] with the compound represented by Formula [9] in the presence or absence of an acid, in the presence or absence of a condensing agent or an acid halide, and in the presence or absence of a base.

As the compound represented by Formula [8], for example, 1-heptanol is known and can be obtained as a commercially available product.

As the compound represented by Formula [9], for example, 10-oxohexadecanoic acid or 5-oxoundecanoic acid is known, and it can be obtained by a known method or as a commercially available product.

The solvent used in this reaction is not particularly limited as long as the solvent does not affect the reaction. Examples of the solvent include halogenated hydrocarbons, ethers, esters, amides, nitriles, sulfoxides, and aromatic hydrocarbons. These solvents may be used by being mixed together. Preferred examples of the solvent include aromatic hydrocarbons and ethers, and toluene and tetrahydrofuran are more preferable.

The used amount of the solvent is not particularly limited, but is only required to be 1 to 500 times (v/w) the amount of the compound represented by Formula [8].

Examples of the acid used in this reaction include an inorganic acid and an organic acid. As the acid, sulfonic acids are preferable. Specifically, examples thereof include sulfuric acid, 4-toluenesulfonic acid, methanesulfonic acid, and the like.

Examples of the condensing agent used in this reaction include carbodiimides such as N,N'-dicyclohexylcarbodiimide and 1-ethyl-3-(3-dimethylaminopropyl)carbodiimide; carbonyls such as carbonyldiimidazole; acid azides such as diphenylphosphoryl azide; acid cyanides such as diethylphosphoryl cyanide; uroniums such as 2-ethoxy-1-ethoxycarbonyl-1,2-dihydroquinoline; O-benzotriazol-1-yl-1,1,3,3-tetramethyluronium=hexafluorophosphate; O-(7-azabenzotriazol-1-yl)-1,1,3,3-tetramethyluronium=hexafluorophosphate; and the like.

Examples of the acid halide used in this reaction include carboxylic acid halides such as acetyl chloride and trifluoroacetyl chloride; sulfonic acid halides such as methanesulfonyl chloride and tosyl chloride; chloroformic acid esters such as ethyl chloroformate and isobutyl chloroformate; and the like.

Examples of the base used in this reaction include an inorganic base and an organic base. An organic base is preferable. Specifically, examples thereof include triethylamine, N,N-diisopropylethylamine, 4-methylmorpholine, pyridine, 1,8-diazabicyclo[5.4.0]-7-undecene, 4-dimethylaminopyridine, and the like. These bases may be used in combination.

The used amount of the base is only required to be 1 to 50 times and preferably 1 to 10 times the molar amount of the compound represented by Formula [8].

The used amount of the compound represented by Formula [9] is not particularly limited, but is only required to be 0.8 to 10 times (v/w) the amount of the compound represented by Formula [8].

This reaction is only required to carry out at -30°C to 200°C, preferably at 0°C to 150°C for 5 minutes to 48 hours.

### (2-2)

The compound represented by Formula [2] can be manufactured by reacting the compound represented by Formula [10] with a reducing agent.

The solvent used in this reaction is not particularly limited as long as the solvent does not affect the reaction. Examples of the solvent include halogenated hydrocarbons, alcohols, ethers, esters, amides, nitriles, sulfoxides, and aromatic hydrocarbons. These solvents may be used by being mixed together.

Preferred examples of the solvent include alcohols, ethers, and aromatic hydrocarbons, and a mixed solvent of alcohols and aromatic hydrocarbons is more preferable.

The used amount of the solvent is not particularly limited, but is only required to be 1 to 500 times (v/w) the amount of the compound represented by Formula [10].

Examples of the reducing agent used in this reaction include sodium borohydride, lithium borohydride, calcium borohydride, sodium cyanoborohydride, sodium triacetoxyborohydride, and lithium aluminum hydride, and sodium borohydride is more preferable.

The used amount of the reducing agent is only required to be 0.1 to 100 times and preferably 0.5 to 10 times the molar amount of the compound represented by Formula [10].

This reaction is only required to carry out at -30°C to 150°C, preferably at 0°C to 100°C for 5 minutes to 48 hours.

### [Manufacturing method 3]

A method for manufacturing the compound represented by Formula [5A] and the compound represented by Formula [5B] from the compound represented by Formula [11].

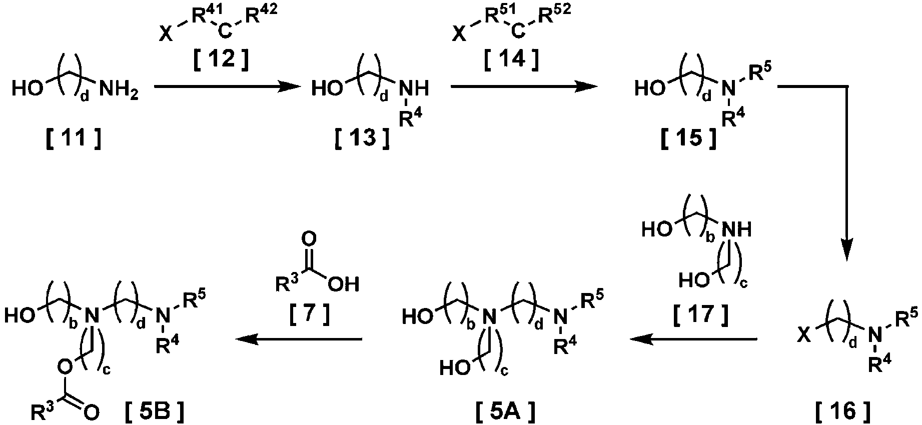

In the formulae, R³, R⁴, R⁵, b, c, and d have the same meanings as above; X represents the same leaving group as above; R⁴¹ and R⁵¹ represent hydrocarbon groups having 1 to 6 carbon atoms; R⁴² and R⁵² represent a hydrogen atom or a hydrocarbon group having 1 to 18 carbon atoms; and C represent an oxygen atom or is absent.

(3-1)
The compound represented by Formula [13] can be manufactured by reacting the compound represented by Formula [11] with the compound represented by Formula [12] in the presence or absence of a base and in the presence or absence of an additive.

As the compound represented by Formula [11], for example, 2-aminoethanol is known, and as the compound represented by Formula [12], for example, benzyl 2-bromoethyl ether is known.

The solvent used in this reaction is not particularly limited as long as the solvent does not affect the reaction. Examples of the solvent include halogenated hydrocarbons, alcohols, ethers, esters, amides, nitriles, sulfoxides, and aromatic hydrocarbons. These solvents may be used by being mixed together. Preferred examples of the solvent include alcohols, and ethanol is more preferable.

The used amount of the solvent is not particularly limited, but is only required to be 1 to 500 times (v/w) the amount of the compound represented by Formula [11].

Examples of the base used in this reaction include an inorganic base and an organic base. Specifically, examples thereof include potassium hydroxide, sodium hydroxide, lithium hydroxide, potassium carbonate, sodium carbonate, lithium carbonate, potassium phosphate, sodium phosphate, lithium phosphate, triethylamine, N,N-diisopropylethylamine, 4-methylmorpholine, pyridine, 1,8-diazabicyclo[5.4.0]-7-undecene, 4-dimethylaminopyridine, and the like, and the inorganic salts are preferable and the potassium carbonate is more preferable.

The used amount of the base is only required to be 1 to 50 times and preferably 1 to 10 times the molar amount of the compound represented by Formula [11].

The used amount of the compound represented by Formula [12] is not particularly limited, but is only required to be 1 to 10 times the molar amount of the compound represented by Formula [11].

Specific examples of the additive used in this reaction include lithium iodide, sodium iodide, potassium iodide, benzyltriethylammonium iodide, and the like.

The used amount of the additive is only required to be 0 to 10 times the molar amount of the compound represented by Formula [12].

This reaction is only required to carry out at -30°C to 150°C, preferably at 0°C to 100°C for 5 minutes to 48 hours.

(3-2)
The compound represented by Formula [15] can be manufactured in the same method as in the manufacturing method 3 (3-1) using the compound represented by Formula [13] and the compound represented by Formula [14] as raw materials.

(3-3)
The compound represented by Formula [16] can be manufactured by reacting the compound represented by Formula [15] with a reagent that converts a hydroxyl group of the compound represented by Formula [15] into a leaving group, in the presence or absence of a base.

The solvent used in this reaction is not particularly limited as long as the solvent does not affect the reaction. Examples of the solvent include halogenated hydrocarbons, ethers, esters, amides, nitriles, sulfoxides, and aromatic hydrocarbons. These solvents may be used by being mixed together. Preferred examples of the solvent include ethers, and tetrahydrofuran is more preferable.

The used amount of the solvent is not particularly limited, but is only required to be 1 to 500 times (v/w) the amount of the compound represented by Formula *[15].*

Examples of the base used in this reaction include an inorganic base and an organic base. Specifically, examples thereof include potassium carbonate, sodium carbonate, lithium carbonate, potassium phosphate, sodium phosphate, lithium phosphate, triethylamine, N,N-diisopropylethylamine, 4-methylmorpholine, pyridine, 1,8-diazabicyclo[5.4.0]-7-undecene, 4-dimethylaminopyridine, and the like.

Examples of the reagent that converts a hydroxyl group into a leaving group, used for this reaction, include sulfonic halides such as methanesulfonyl chloride and tosyl chloride; and a combination of triphenylphosphine and tetrahalomethane, and a combination of triphenylphosphine and tetrabromomethane is more preferable.

The used amount of the reagent is not particularly limited, but is only required to be 1 to 10 times the molar amount of the compound represented by Formula *[15].*

This reaction is only required to carry out at -30°C to 150°C, preferably at 0°C to 100°C for 5 minutes to 48 hours.

(3-4)
The compound represented by Formula [5A] can be manufactured by reacting the compound represented by Formula [16] with the compound represented by Formula [17] in the presence of a base and in the presence or absence of an additive.

As the compound represented by Formula [17], for example, diethanolamine and the like are known.

The solvent used in this reaction is not particularly limited as long as the solvent does not affect the reaction. Examples of the solvent include halogenated hydrocarbons, alcohols, ethers, esters, amides, nitriles, sulfoxides, and aromatic hydrocarbons. These solvents may be used by being mixed together. Preferred examples of the solvent include alcohols, and ethanol is more preferable.

The used amount of the solvent is not particularly limited, but is only required to be 1 to 500 times (v/w) the amount of the compound represented by Formula [16].

Examples of the base used in this reaction include an inorganic base and an organic base. Specifically, examples thereof include potassium hydroxide, sodium hydroxide, lithium hydroxide, potassium carbonate, sodium carbonate, lithium carbonate, potassium phosphate, sodium phosphate, lithium phosphate, triethylamine, N,N-diisopropylethylamine, 4-methylmorpholine, pyridine, 1,8-diazabicyclo[5.4.0]-7-undecene, 4-dimethylaminopyridine, and the like, and the potassium carbonate is more preferable.

The used amount of the base is only required to be 1 to 50 times and preferably 1 to 10 times the molar amount of the compound represented by Formula [16].

The used amount of the compound represented by Formula [17] is not particularly limited, but is only required to be 1 to 10 times the molar amount of the compound represented by Formula [16].

Specific examples of the additive used in this reaction include lithium iodide, sodium iodide, potassium iodide, benzyltriethylammonium iodide, and the like.

The used amount of the additive is only required to be 0.1 to 10 times the molar amount of the compound represented by Formula [16].

This reaction is only required to carry out at -30°C to 150°C, preferably at 0°C to 100°C for 5 minutes to 48 hours.

*(3-5)*
The compound represented by Formula [5B] can be manufactured in the same method as in the manufacturing method 1 (1-3) using the compound represented by Formula [5A] and the compound represented by Formula [7] as raw materials.

### [Manufacturing method 4]

A method for manufacturing the compound represented by Formula [7A] from the compound represented by Formula [16].

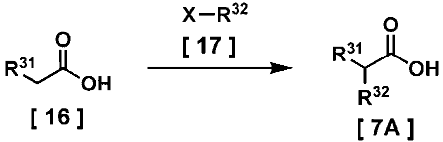

In the formulae, R³ and X have the same meanings as above; and R³¹ and R³² represent hydrocarbon groups in combination having a total number of carbon atoms of 5 to 23.

The compound represented by Formula [7A] can be manufactured by reacting the compound represented by Formula [16] with the compound represented by Formula [17] in the presence of a base and in the presence or absence of an additive.

As the compound represented by Formula [16], for example, octanoic acid and the like are known.

As the compound represented by Formula [17], for example, 1-iodohexane and the like are known.

The solvent used in this reaction is not particularly limited as long as the solvent does not affect the reaction. Examples of the solvent include halogenated hydrocarbons, ethers, esters, amides, nitriles, and aromatic hydrocarbons. These solvents may be used by being mixed together.

Preferred examples of the solvent include ethers, and tetrahydrofuran is more preferable.

The used amount of the solvent is not particularly limited, but is only required to be 1 to 500 times (v/w) the amount of the compound represented by Formula [16].

Examples of the base used in this reaction include an inorganic base and an organic base. Specific examples thereof include sodium hydride, calcium hydride, lithium diisopropylamide, n-butyllithium, 1,8-diazabicyclo[5.4.0]-7-undecene, and 4-dimethylaminopyridine, and these bases may be used in combination.

The used amount of the base is only required to be 1 to 50 times and preferably 1 to 10 times the molar amount of the compound represented by Formula [16].

The used amount of the compound represented by Formula [17] is not particularly limited, but is only required to be 1 to 10 times the molar amount of the compound represented by Formula [16].

Specific examples of the additive used in this reaction include lithium iodide, sodium iodide, potassium iodide, benzyltriethylammonium iodide, and the like.

The used amount of the additive is only required to be 0 to 10 times the molar amount of the compound represented by Formula [17].

This reaction is only required to carry out at -30°C to 150°C, preferably at 0°C to 100°C for 5 minutes to 48 hours.

### [Manufacturing method 5]

A method for manufacturing the compound represented by Formula [7B] from the compound represented by Formula [18].

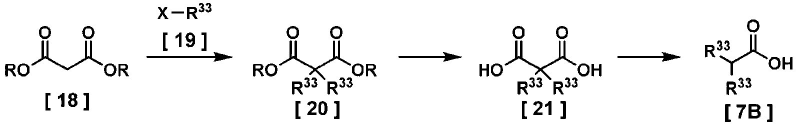

In the formulae, R³ and X have the same meanings as above; R³³ represents a hydrocarbon group having 2 to 11 carbon atoms; and R represents a hydrocarbon group having 1 to 10 carbon atoms.

(5-1)
The compound represented by Formula [20] can be manufactured by reacting the compound represented by Formula [18] with the compound represented by Formula [19] in the presence of a base.

As the compound represented by Formula [18], for example, di-tert-butyl malonate and the like are known.

As the compound represented by Formula [19], for example, 1-bromooctane and the like are known.

The solvent used in this reaction is not particularly limited as long as the solvent does not affect the reaction. Examples of the solvent include halogenated hydrocarbons, ethers, esters, amides, nitriles, sulfoxides, and aromatic hydrocarbons. These solvents may be used by being mixed together. Preferred examples of the solvent include a mixed solvent of sulfoxides and aromatic hydrocarbons, and a mixed solvent of dimethyl sulfoxide and toluene is more preferable.

The used amount of the solvent is not particularly limited, but is only required to be 1 to 500 times (v/w) the amount of the compound represented by Formula [18].

Examples of the base used in this reaction include an inorganic base and an organic base. Specific examples thereof include potassium hydroxide, sodium hydroxide, lithium hydroxide, potassium carbonate, sodium carbonate, lithium carbonate, potassium phosphate, sodium phosphate, lithium phosphate, triethylamine, N,N-diisopropylethylamine, 4-methylmorpholine, pyridine, 1,8-diazabicyclo[5.4.0]-7-undecene, and 4-dimethylaminopyridine, and potassium hydroxide, sodium hydroxide, or lithium hydroxide is more preferable.

The used amount of the base is only required to be 1 to 50 times and preferably 1 to 10 times the molar amount of the compound represented by Formula [18].

The used amount of the compound represented by Formula [19] is not particularly limited, but is only required to be 2 to 10 times the molar amount of the compound represented by Formula [18].

This reaction is only required to carry out at -30°C to 150°C, preferably at 0°C to 100°C for 5 minutes to 48 hours.

(5-2)
The compound represented by Formula [21] can be manufactured by reacting the compound represented by Formula [20] in the presence or absence of an acid and in the presence or absence of a base.

The solvent used in this reaction is not particularly limited as long as the solvent does not affect the reaction. Examples of the solvent include water, halogenated hydrocarbons, ethers, esters, amides, nitriles, sulfoxides, and aromatic hydrocarbons. These solvents may be used by being mixed together. Preferred examples of the solvent include a mixed solvent of water and aromatic hydrocarbons.

The used amount of the solvent is not particularly limited, but is only required to be 0.1 to 10 times (v/w) the amount of the compound represented by Formula [20].

Examples of the acid used in this reaction include an inorganic acid and an organic acid. Specific examples thereof include hydrochloric acid, hydrobromic acid, phosphoric acid, sulfuric acid, formic acid, and trifluoroacetic acid, and trifluoroacetic acid is preferable.

The used amount of the acid is not particularly limited, but is only required to be 1 to 50 times (v/w) the amount of the compound represented by Formula [20].

Examples of the base used in this reaction include an inorganic base and an organic base. Specifically, examples thereof include potassium hydroxide, sodium hydroxide, lithium hydroxide, potassium carbonate, sodium carbonate, lithium carbonate, potassium phosphate, sodium phosphate, lithium phosphate, triethylamine, N,N-diisopropylethylamine, 4-methylmorpholine, pyridine, 1,8-diazabicyclo[5.4.0]-7-undecene, 4-dimethylaminopyridine, and the like.

The used amount of the base is only required to be 2 to 50 times the molar amount of the compound represented by Formula [20].

This reaction is only required to carry out at -30°C to 150°C, preferably at 0°C to 100°C for 5 minutes to 48 hours.

(5-3)
The compound represented by Formula [7B] can be manufactured by reacting the compound represented by Formula [21] in the presence or absence of an acid, in the presence or absence of a base, and in the presence or absence of a solvent.

The solvent used in this reaction is not particularly limited as long as the solvent does not affect the reaction. Examples of the solvent include halogenated hydrocarbons, ethers, esters, amides, nitriles, sulfoxides, and aromatic hydrocarbons. These solvents may be used by being mixed together.

The used amount of the solvent is not particularly limited, but is only required to be 0 to 10 times (v/w) the amount of the compound represented by Formula [21].

Examples of the acid used in this reaction include an inorganic acid and an organic acid. Specific examples thereof include hydrochloric acid, phosphoric acid, sulfuric acid, formic acid, and trifluoroacetic acid.

The used amount of the acid is not particularly limited, but is only required to be 1 to 50 times (v/w) the amount of the compound represented by Formula [21].

Examples of the base used in this reaction include an inorganic base and an organic base. Specifically, examples thereof include potassium hydroxide, sodium hydroxide, lithium hydroxide, potassium carbonate, sodium carbonate, lithium carbonate, potassium phosphate, sodium phosphate, lithium phosphate, triethylamine, N,N-diisopropylethylamine, 4-methylmorpholine, pyridine, 1,8-diazabicyclo[5.4.0]-7-undecene, 4-dimethylaminopyridine, and the like, and the pyridine is more preferable.

The used amount of the base is only required to be 2 to 50 times the molar amount of the compound represented by Formula [21].

This reaction is only required to carry out at -30°C to 250°C, preferably at 0°C to 200°C for 5 minutes to 48 hours.

### [Manufacturing method 6]

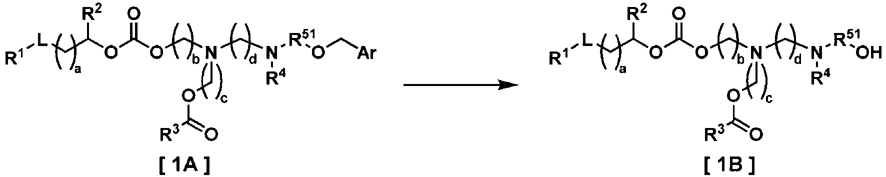

In the formula, R¹, R², R³, R⁴, R⁵, R⁵¹, L, a, b, c, and d have the same meanings as above; and Ar represents an aromatic hydrocarbon group.

The compound represented by Formula [1B] can be manufactured by deprotecting the compound represented by Formula [1A].

This reaction may be performed, for example, according to the method described in T. W. Greene et al., Protective Groups in Organic Synthesis, 4th Edition, pp. 16 to 366, 2007, John Wiley & Sons, INC.

In a case where the compounds used in the above-described manufacturing methods have isomers (for example, an optical isomer, a geometric isomer, a tautomer, and the like), these isomers can also be used.

In addition, in a case where the compounds are in the form of solvates, hydrates, and crystals of various shapes, these solvates, hydrates, and crystals of various shapes can also be used.

In a case where the compounds used in the above-described manufacturing methods have, for example, an amino group, a hydroxyl group, and a carboxyl group, these groups can be protected with ordinary protecting groups in advance, and these protecting groups can be eliminated by known methods after the reaction.

The compounds obtained by the above-described manufacturing methods can be transformed into other compounds by being subjected to known reaction such as condensation, addition, oxidation, reduction, transition, substitution, halogenation, dehydration, or hydrolysis, or by being subjected to these reactions that are appropriately combined.

### <Lipid composition>

In the present invention, a lipid composition containing the compound according to an embodiment of the present invention or a salt thereof can be prepared. In preparing the lipid composition, in addition to the compound according to the embodiment of the present invention, it is possible to use at least one kind of lipid selected from the group consisting of a sterol and a lipid having a nonionic hydrophilic polymer chain. The lipid composition can further contain a neutral lipid. The lipid composition can further contain a nucleic acid.

In the lipid composition according to the embodiment of the present invention, the amount of the compound represented by Formula (1) according to the embodiment of the present invention or a salt thereof mixed in, with respect to the total mass of lipids, is preferably 20 mol% to 80 mol%, more preferably 30 mol% to 70 mol%, and even more preferably 35 mol% to 65 mol%.

### <Sterol>

The lipid composition according to the embodiment of the present invention preferably contains a sterol. In a case where the lipid composition according to the embodiment of the present invention contain a sterol, the fluidity of the membrane can be reduced, and hence the lipid composition can be effectively stabilized.

The sterol is not particularly limited, and examples thereof can include cholesterol, phytosterol (sitosterol, stigmasterol, fucosterol, spinasterol, brassicasterol, and the like), ergosterol, cholestanone, cholestenone, coprostanol, cholesteryl-2'-hydroxyethyl ether, and cholesteryl-4'-hydroxybutyl ether. Among these, cholesterol is preferable.

In the lipid composition according to the embodiment of the present invention, the amount of the sterol mixed in, with respect to the total mass of lipids, is preferably 10 mol% to 70 mol%, more preferably 20 mol% to 65 mol%, and even more preferably 25 mol% to 60 mol%.

### <Neutral lipid>

The lipid composition according to the embodiment of the present invention may contain a neutral lipid. The neutral lipid is not particularly limited, and examples thereof include phosphatidylcholine, phosphatidylethanolamine, sphingomyelin, ceramide, and the like. Among these, phosphatidylcholine is preferable. The neutral lipid may be a single neutral lipid or a combination of a plurality of different neutral lipids.

The phosphatidylcholine is not particularly limited, and examples thereof include soybean lecithin (SPC), hydrogenated soybean lecithin (HSPC), egg yolk lecithin (EPC), hydrogenated egg yolk lecithin (HEPC), dimyristoylphosphatidylcholine (DMPC), dipalmitoylphosphatidylcholine (DPPC), 1,2-distearoyl-sn-glycero-3-phosphocholine (DSPC), 1-palmitoyl-2-oleoylphosphatidylcholine (POPC), dioleoylphosphatidylcholine (DOPC), and the like.

The phosphatidylethanolamine is not particularly limited, and examples thereof include dimyristoylphosphatidylethanolamine (DMPE), dipalmitoylphosphatidylethanolamine (DPPE), distearoylphosphatidylethanolamine (DSPE), dioleoylphosphatidylethanolamine (DOPE), dilinoleoylphosphatidylethanolamine (DLoPE), diphytanoylphosphatidylethanolamine (D(Phy)PE), 1-palmitoyl-2-oleoylphosphatidylethanolamine (POPE), ditetradecylphosphatidylethanolamine, dihexadecylphosphatidylethanolamine, dioctadecylphosphatidylethanolamine, diphytanylphosphatidylethanolamine, and the like.

The sphingomyelin is not particularly limited, and examples thereof include egg yolk-derived sphingomyelin, milk-derived sphingomyelin, and the like.

The ceramide is not particularly limited, and examples thereof include egg yolk-derived ceramide, milk-derived ceramide, and the like.

In the lipid composition according to the embodiment of the present invention, the amount of the neutral lipid mixed in, with respect to the total amount of the constituent lipid components, is preferably 0 mol% or more and 55 mol% or less and more preferably 0 mol% or more and 40 mol% or less.

### <Lipid having nonionic hydrophilic polymer chain>

The lipid composition according to the embodiment of the present invention may contain a lipid having a nonionic hydrophilic polymer chain in an oil phase. In the present invention, in a case where the lipid composition contains the lipid having a nonionic hydrophilic polymer chain in an oil phase, the dispersion of the lipid particles can be effectively stabilized.

The nonionic hydrophilic polymer is not particularly limited, and examples thereof include a nonionic vinyl-based polymer, a nonionic polyamino acid, a nonionic polyester, a nonionic polyether, a nonionic natural polymer, a nonionic modified natural polymer, and a block polymer or a graft copolymer having two or more kinds of these polymers as constitutional units.

Among these nonionic hydrophilic polymers, a nonionic polyether, a nonionic polyester, a nonionic polyamino acid, or a nonionic synthetic polypeptide is preferable, a nonionic polyether or a nonionic polyester is more preferable, a nonionic polyether or a nonionic monoalkoxy polyether is even more preferable, and polyethylene glycol (hereinafter, polyethylene glycol will be also called PEG) is particularly preferable.

The lipid having a nonionic hydrophilic polymer chain is not particularly limited, and examples thereof include PEG-modified phosphoethanolamine, a diacylglycerol PEG derivative, a monoacylglycerol PEG derivative, a dialkylglycerol PEG derivative, a cholesterol PEG derivative, a ceramide PEG derivative, and the like. Among these, the monoacylglycerol PEG derivative or the diacylglycerol PEG derivative is preferable.

The weight-average molecular weight of the PEG chain of the nonionic hydrophilic polymer derivative is preferably 500 to 5,000 and more preferably 750 to 3,000.

The nonionic hydrophilic polymer chain may be branched or may have a substituent such as a hydroxymethyl group.

In the lipid composition according to the embodiment of the present invention, the amount of the lipid having a nonionic hydrophilic polymer chain mixed in, with respect to the total amount of lipids, is preferably 0.1 mol% to 12 mol%, more preferably 0.3 mol% to 6 mol%, and still more preferably 0.5 mol% to 5 mol%.

### <Nucleic acid, protein, peptide, and low-molecular-weight compound>

The lipid composition according to the embodiment of the present invention may contain at least one selected from the group consisting of a nucleic acid, a protein, a peptide, and a low-molecular-weight compound.

Examples of the nucleic acid include plasmid DNA, nanoplasmid DNA, single-stranded DNA, double-stranded DNA, small interfering RNA (siRNA), microRNA (miRNA), mRNA, an antisense oligonucleotide (also referred to as ASO), ribozyme, an aptamer, dsRNA, saRNA, sgRNA, shRNA, tRNA, circular RNA and the like. The lipid composition may contain any of these. In addition, the lipid particles may contain a modified nucleic acid. As the nucleic acid, RNA is particularly preferable, and RNA having a number of bases of 5 to 20,000 is preferable.

In the lipid composition according to the embodiment of the present invention, the mass ratio of the lipids to the nucleic acid is preferably 2 to 1,000, more preferably 3 to 500, still more preferably 5 to 200, and particularly preferably 5 to 100.

Examples of the protein, peptide, and low-molecular-weight molecule include an intracellular protein, an intracellular peptide, a membrane protein, a membrane peptide, a secreted protein, a secreted peptide, a synthetic protein, a synthetic peptide, a natural low-molecular-weight compound, a synthetic low-molecular-weight compound, a compound having an antitumor activity, and the like.

More specifically, examples of the protein include a CRISPR-Cas protein, a gene editing-related protein such as a zinc-finger protein, and TALEN, a hormone such as erythropoietin, an endogenous factor such as VEGF, a cancer antigen protein, an antibody, and the like. Examples of the peptide include a functional domain or a recognition domain of the above-described protein. mRNA or DNA encoding the above-described protein may be contained.

As the peptide, a natural peptide is desirable. In addition, the peptide structure may be linear or may be cyclic. In a case where the peptide is cyclic, examples of a linking portion of the ring include an amide bond, a disulfide bond, and the like.

Examples of the low-molecular-weight compound include an anticancer agent, an antibacterial agent, an antifungal agent, and the like.

These proteins, peptides, and low-molecular-weight compounds may have physiological activity in vivo or may not have physiological activity. Here, the low-molecular-weight compound refers to an organic compound having a molecular weight of about 1,000 or less.

### <Manufacturing method of lipid composition>

The manufacturing method of the lipid composition according to the embodiment of the present invention will be described.

The manufacturing method of the lipid composition is not limited, and for example, the lipid composition can be manufactured by a method in which all of the constituent components of the lipid composition or some of oil-soluble components of the lipid composition are dissolved in an organic solvent or the like to form an oil phase, water-soluble components of the lipid composition are dissolved in water to form a water phase, and then the oil phase and the water phase are mixed together. A micromixer may be used for mixing, or an emulsification using an emulsifying machine such as a homogenizer, an ultrasonic emulsifying machine, a high-pressure injection emulsifying machine, or the like may be performed.

Alternatively, the lipid composition can also be manufactured by a method in which a lipid-containing solution is subjected to evaporation to dryness using an evaporator under reduced pressure or subjected to spray drying using a spray drier, so that a dried mixture containing a lipid is prepared, and then the mixture is added to an aqueous solvent and further emulsified using the aforementioned emulsifying machine or the like.

Examples of a manufacturing method of a lipid composition containing a nucleic acid include a method including:
a step (a) of dissolving constituent components of a lipid composition containing the compound according to the embodiment of the present invention in an organic solvent to obtain an oil phase, and dissolving a nucleic acid in an aqueous solvent to obtain a water phase;
a step (b) of mixing the oil phase and the water phase obtained in the step (a) to obtain a dispersion liquid of lipid particles;
a step (c) of diluting the dispersion liquid of the lipid particles obtained in the step (b);
a step (d) of removing the organic solvent from the dispersion liquid of the lipid particles; and
a step (e) of adjusting the concentration of the dispersion liquid of the lipid particles.

In the step (a), the constituent components of the lipid composition containing the compound according to the embodiment of the present invention are dissolved in an organic solvent (an alcohol such as ethanol, an ester, or the like). The total lipid concentration is not particularly limited, but is generally 1 mmol/L to 100 mmol/L, preferably 5 mmol/L to 50 mmol/L, and more preferably 10 mmol/L to 30 mmol/L.

The water phase can be obtained by dissolving a nucleic acid (for example, siRNA, mRNA, an antisense nucleic acid, or the like) in water or a buffer solution. The concentration of the nucleic acid is not particularly limited, but is preferably 1 to 1,000 µg/mL and more preferably 10 to 500 µg/mL. A component such as a buffer component or an antioxidant for pH adjustment can be added as necessary. The pH of the water phase is preferably 2.0 to 7.0 and more preferably 3.0 to 6.0. Acetic acid, citric acid, malic acid, phosphoric acid, MES, HEPES, or the like is preferably used as a buffer component for adjusting the pH, and as necessary, salts such as sodium chloride and potassium chloride may be added for the purpose of adjusting a ionic strength, or sugars or sugar alcohols, such as sucrose, trehalose, and mannitol may be added for the purpose of adjusting an osmotic pressure.

In the step (b), the oil phase and the water phase may be mixed by any method, and a batch type or an in-line method using a channel device may be used. As the in-line method, a microchannel device is preferably used, and as the microchannel device to be used, a Y-shaped mixer, a T-shaped mixer, a herringbone mixer, a ring micromixer, an impingement jet mixer, or the like can be used. The mixing ratio (volume ratio) of water phase:oil phase is preferably 5:1 to 1:1 and more preferably 4:1 to 2:1.

In the step (c), by mixing the dispersion liquid of lipid particles with the dilution solution, the content of the organic solvent can be reduced and the lipid particles can be stabilized. The dilution solution may be water, but the pH or ionic strength may be adjusted with the dilution solution. The component contained in the dilution solution can be optionally selected depending on the purpose. For example, for the purpose of adjusting the pH, a buffer solution (for example, a citrate buffer solution, a citrate buffered physiological saline, an acetate buffer solution, an acetate buffered physiological saline, a phosphate buffered physiological saline, a Tris buffer solution, an MES buffer solution, a HEPES buffer solution, or the like) may be used. In addition, sodium chloride, potassium chloride, sucrose, trehalose, fructose, mannitol, or the like may be contained for the purpose of adjusting the ionic strength or the osmotic pressure, and a buffer solution to which these additives are further added can also be used.

The dispersion liquid of lipid particles and the dilution solution may be mixed by any method, and a batch type or an in-line method using a channel device may be used. As the channel device used at the time of mixing, a Y-shaped mixer, a T-shaped mixer, or the like can be used. In addition, the time from mixing the oil phase with the water phase to mixing the dilution solution therewith is not particularly limited, but the dilution is preferably performed within 30 seconds and more preferably performed within 10 seconds after mixing the oil phase with the water phase.

The mixing ratio (liquid amount ratio) of the dispersion liquid of lipid particles to the dilution solution is preferably 1:0.5 to 1:10 and more preferably 1:1 to 1:5.

In some embodiments, in the step (c), the dispersion liquid of lipid particles may be mixed with the dilution solution a plurality of times depending on the purpose. In addition, the dilution solutions to be used may be the same as or different from each other. In the dispersion liquid of lipid particles, the particle diameter of the lipid particles may change depending on the pH, and thus the adjustment of the pH of the dispersion liquid is important. Therefore, for example, to adjust the pH of the dispersion liquid of lipid particles after mixing with the dilution solution, a buffer solution having a concentration and a pH suitable for the adjustment or a buffer solution further containing other components may be used.

Furthermore, the plurality of dilution steps may be continuously performed, and the interval between the dilution step and the next dilution step can be optionally set, and for example, the interval may be 10 seconds, 30 seconds, 1 minute, 5 minutes, 10 minutes, 30 minutes, 1 hour, 2 hours, 3 hours, 4 hours, 6 hours, 12 hours, or 24 hours.

In addition, the pH of the dispersion liquid of lipid particles after performing the step (c) is preferably 3.0 to 10.0, more preferably 3.5 to 9.0, and particularly preferably 4.0 to 8.5.

The lipid composition can be subjected to sizing if necessary. The method of sizing is not particularly limited, and the particle size can be reduced by using an extruder or the like.

In addition, the dispersion liquid containing the lipid composition according to the embodiment of the present invention can be frozen or freeze-dried by a general method.

In the step (d), a method of removing the organic solvent from the dispersion liquid of lipid particles is not particularly limited, and a general method can be used. For example, a pH buffer solution such as phosphate buffered physiological saline or a Tris buffer solution can be used as the dialysate, and additives such as any salt or sugar can be added as necessary for the purpose of adjusting the osmotic pressure or protecting the dialysate from freezing.

In the step (e), the concentration of the dispersion liquid of lipid particles obtained in the step (d) can be adjusted. In a case of diluting the dispersion liquid, the dispersion liquid can be diluted to an appropriate concentration by using a solution such as phosphate buffered physiological saline, physiological saline, a Tris buffer solution, or a sucrose-containing Tris buffer solution as a dilution solution. In a case of concentrating the dispersion liquid, the dispersion liquid obtained in the step (d) can be concentrated by ultrafiltration using an ultrafiltration membrane, or the like. It is preferable to use the concentrated dispersion as it is. Alternatively, it is preferable to adjust the concentrated dispersion liquid to a desired concentration by using the aforementioned diluent after the concentration.

In addition, in some embodiments, the organic solvent removal step (step (d)) and the concentration adjustment step (step (e)) can be continuously performed using tangential flow filtration (TFF). In the present step, the organic solvent removal step and the concentration adjustment step may be performed in any order. The organic solvent removal step and the concentration adjustment step may be each performed a plurality of times as necessary.

As the solution that can be used in the dialysis in the step (d) or the dilution in the step (e), an excipient, a freeze protective agent, a buffering agent, or an antioxidant may be added. The excipient or the freeze protective agent is not particularly limited, and examples thereof include sugars and sugar alcohols. Examples of the sugars include sucrose, trehalose, maltose, glucose, lactose, fructose, and the like, and examples of the sugar alcohols include mannitol, sorbitol, inositol, xylitol, and the like. The buffering agent is not particularly limited, and examples thereof include ACES, BES, bicine, CAPS, CHES, DIPSO, EPPS, HEPES, HEPPSO, MES, MOPS, MOPSO, TAPS, TAPSO, TES, tricine, tris, phosphoric acid, acetic acid, citric acid, and the like. Examples of the antioxidant include EDTA, ascorbic acid, tocopherol, and the like.

To make a pharmaceutical composition of the dispersion liquid of lipid particles of the present invention, it is preferable to perform sterile filtration. As a filtration method, a hollow fiber membrane, a reverse osmosis membrane, a membrane filter, or the like can be used to remove insoluble substances from the dispersion liquid of lipid particles. In the present invention, the filtration method is not particularly limited, but it is preferable to filter the dispersion liquid through a filter having a pore diameter capable of sterilization (preferably a filtration sterilization filter with a pore diameter of 0.2 µm). Furthermore, it is preferable that the sterile filtration be performed after Step (d) or Step (e).

In addition, if necessary, the dispersion liquid of lipid particles of the present invention can be frozen or freeze-dried. The dispersion liquid of the lipid particles of the present invention can be frozen or freeze-dried by a general method, and the method is not particularly limited.

### <Regarding lipid composition>

In the present invention, the lipid composition may be lipid particles. The lipid particle means a particle composed of a lipid and includes a composition having any of structures selected from a lipid aggregate in which lipids are aggregated, a micelle, a liposome, a lipid nanoparticle (LNP), or a lipoplex, but the structure of the lipid particle is not limited to these structures as long as the lipid particle is a composition containing a lipid. The liposome has a lipid bilayer structure and has a water phase in the inside, and includes a liposome which has a single bilayer membrane, and a multilayer liposome which has multiple layers stacked together. The present invention may include any of these liposomes.

The form of the lipid particles can be checked by electron microscopy, structural analysis using X-rays, or the like. For example, by a method using Cryo transmission electron microscopy (CryoTEM method), it is possible to check, for example, whether or not a lipid particle is, such as a liposome, a bimolecular lipid membrane structure (lamella structure) and a structure having an inner water layer, and whether or not a lipid particle has a structure having an inner core with a high electron density and packed with constituent components including a lipid. The X-ray small angle scattering (SAXS) analysis also makes it possible to check whether or not a lipid particle has a bimolecular lipid membrane structure (lamella structure).

The particle size of the lipid particles according to the embodiment of the present invention is not particularly limited, and is preferably 10 to 1,000 nm, more preferably 30 to 500 nm, and even more preferably 50 to 250 nm. The particle size of the lipid particles can be measured by a general method (for example, a dynamic light scattering method, a laser diffraction method, or the like).

### <Use of lipid composition>

As an example of the use of the lipid composition according to the embodiment of the present invention, a nucleic acid, a protein, a peptide, or a low-molecular-weight compound can be introduced into a cell by introducing a lipid composition containing a nucleic acid, a protein, a peptide, or a low-molecular-weight compound into the cell. In addition, in a case where the lipid composition according to the embodiment of the present invention contains a nucleic acid, a protein, a peptide, or a low-molecular-weight compound having a pharmaceutical use application, the lipid composition can be administered to a living body as a pharmaceutical composition.

In addition, the lipid composition may be a lipid composition containing only lipid components without nucleic acids, proteins, peptides, or low-molecular-weight compounds. A lipid composition containing a nucleic acid, a protein, a peptide, or a low-molecular-weight compound can also be obtained by preparing a lipid composition containing only a lipid component and then mixing the lipid composition with a nucleic acid, a protein, a peptide, or a low-molecular-weight compound.

In a case where the lipid composition in the present invention is used as a pharmaceutical composition, the lipid composition according to the embodiment of the present invention can be administered to a living body singly or by being mixed with a pharmaceutically acceptable dosing medium (for example, physiological saline or a phosphate buffer solution).

The concentration of the lipid composition in the mixture with a pharmaceutically acceptable dosing medium is not particularly limited, and can be set to 0.05% by mass to 90% by mass in general. In addition, other pharmaceutically acceptable additives, for example, a pH adjusting buffer and an osmotic pressure adjusting agent, may be added to the pharmaceutical composition containing the lipid composition according to the embodiment of the present invention.

The route of administration when the pharmaceutical composition containing the lipid composition according to the embodiment of the present invention is administered is not particularly limited. The nucleic acid drug can be administered by any method. Examples of the administration method include oral administration and parenteral administration (intraarticular administration, intravenous administration, intraarterial administration, subcutaneous administration, intracutaneous administration, intravitreal administration, intraperitoneal administration, intramuscular administration, intravaginal administration, intravesical administration, intrathecal administration, pulmonary administration, rectal administration, colonic administration, buccal administration, nasal administration, intracisternal administration, inhalation, and the like). Parenteral administration is preferable. As the method of administration, intravenous injection, subcutaneous injection, intracutaneous injection, or intramuscular injection is preferable. The pharmaceutical composition containing the lipid composition according to the embodiment of the present invention can also be administered by being directly injected into the affected area.

The dosage form of the lipid composition according to the embodiment of the present invention is not particularly limited. In a case of performing oral administration, the lipid composition according to the embodiment of the present invention can be used in the form of tablets, troches, capsules, pills, suspension, syrup, or the like by being combined with an appropriate excipient. In addition, pharmaceutical preparation suitable for parenteral administration can appropriately contain an antioxidant, a buffer, a bacteriostat, and additives such as an isotonic sterile injection, a suspending agent, a solubilizer, a thickener, a stabilizer, or a preservative.

### <Delivery carrier>

The lipid composition according to the embodiment of the present invention can retain a nucleic acid at a high encapsulation rate and thereby is extremely useful as a delivery carrier for a nucleic acid. According to the delivery carrier using the present invention, the nucleic acid and the like can be introduced into the cells, for example, by mixing the obtained lipid composition with the nucleic acid and the like and transfecting the mixture in vitro, ex vivo, or in vivo. In addition, the delivery carrier using the present invention is also useful as a nucleic acid delivery carrier in nucleic acid drugs. That is, the lipid composition according to the embodiment of the present invention is useful as a composition for nucleic acid delivery in vitro, ex vivo, or in vivo (preferably in vivo).

Next, the present invention is described based on examples, but the present invention is not limited thereto.

### Examples

Unless otherwise specified, for the purification by column chromatography, an automatic purification device ISOLERA (Biotage), a medium pressure separation and purification device Purif-espoir-2 (Shoko Science Co., Ltd.), or a medium pressure liquid chromatograph YFLC W-prep 2XY (Yamazen Corporation) was used.

Unless otherwise specified, as a carrier for silica gel column chromatography, Chromatorex Q-Pack SI 50 (FUJI SILYSIA CHEMICAL LTD.) or HIGH FLASH COLUMN WO01, WO02, WO03, WO04, or WO05 (Yamazen Corporation) was used.

As an NH silica gel, Chromatorex Q-Pack NH 60 (FUJI SILYSIA CHEMICAL LTD.) was used.

NMR spectra were measured using a Bruker AVNEO400 (manufactured by Bruker Corporation) and using tetramethylsilane as an internal standard, and all δ values were shown in ppm.

MS spectra were measured using an ACQUITY SQD LC/MS System (manufactured by Waters Corporation).

### Comparative Example

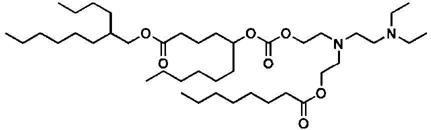

The synthesis was performed according to the method described in WO2019/235635A ([Example 108] (2-butyloctyl 3-ethyl-12-hexyl-6-(2-(octanoyloxy)ethyl)-10-oxo-9,11-dioxa-3,6-diazahexadecan-16-oate).

### [Example 1]

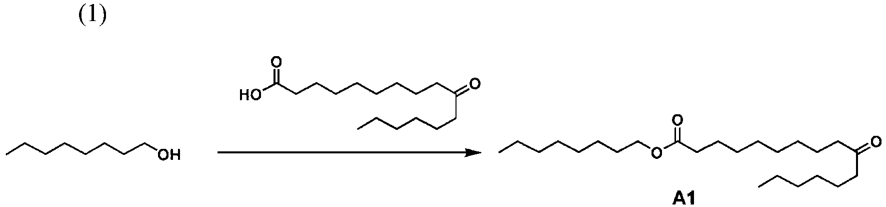

To a 10 mL dichloromethane solution of 1.0 g of 10-oxohexadecanoic acid, 0.64 mL of octan-1-ol, 1.0 g of 1-ethyl-3-(3-dimethylaminopropyl)carbodiimide hydrochloride, 0.9 g of 4-dimethylaminopyridine, and 2.1 mL of triethylamine were added, and the mixture was stirred at room temperature for 6 hours. The solvent was distilled off from the reaction mixture under reduced pressure, ethyl acetate and water were added thereto, and the organic layer was isolated. The organic layer was washed with saturated saline, and anhydrous sodium sulfate was then added thereto to dry the organic layer, and the solvent was distilled off under reduced pressure. The obtained residue was purified by silica gel column chromatography (ethyl acetate-hexane), thereby obtaining 1.1 g of octyl 10-oxohexadecanoate (A1) as white solids.

¹H-NMR (CDCl₃) δ:4.05 (2H, t, J = 6.7 Hz), 2.37 (4H, t, J = 7.4 Hz), 2.28 (2H, t, J = 7.6 Hz), 1.65-1.50 (10H, m), 1.36-1.21 (22H, m), 0.91-0.84 (6H, m).

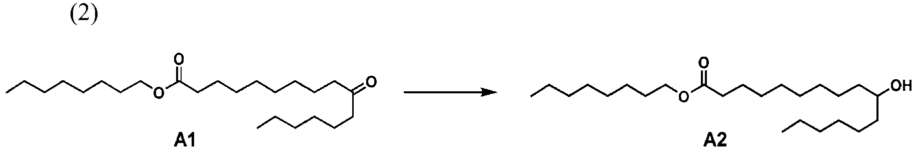

To a mixed solution of 1.1 g of octyl 10-oxohexadecanoate (A1) in 6 mL of tetrahydrofuran and 6 mL of methanol under ice cooling, 0.17 g of sodium borohydride was added, and the mixture was stirred under ice cooling for 0.5 hours. The reaction mixture was added dropwise to ice water, and a 1N hydrochloric acid aqueous solution was added thereto until the solution became acidic. After adding ethyl acetate thereto, the organic layer was isolated. The organic layer was washed with saturated saline, and anhydrous sodium sulfate was then added thereto to dry the organic layer, and the solvent was distilled off under reduced pressure. The obtained residue was purified by silica gel column chromatography (ethyl acetate-hexane), thereby obtaining 1.1 g of octyl 10-hydroxyhexadecanoate (A2) as a colorless oily substance.

¹H-NMR (CDCl₃) δ:24.69 (1H, t, J = 5.5 Hz), 4.08 (2H, d, J = 5.5 Hz), 3.75-3.67 (2H, m), 3.61-3.53 (2H, m), 3.22-3.15 (4H, m), 1.55-1.47 (4H, m), 1.33-1.20 (26H, m), 0.90-0.85 (6H, m).

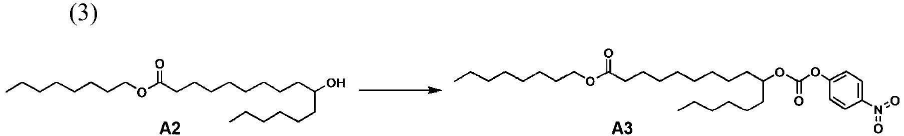

To a 11 mL tetrahydrofuran solution of 1.1 g of octyl 10-hydroxyhexadecanoate (A2), 0.87 g of 4-Nitrophenyl chloroformate and 1.2 mL of triethylamine were added, and the mixture was stirred at room temperature for 2 hours. Ethyl acetate and water were added to the reaction mixture, and the organic layer was isolated. The organic layer was washed with saturated saline, and anhydrous sodium sulfate was then added thereto to dry the organic layer, and the solvent was distilled off under reduced pressure. The obtained residue was purified by silica gel column chromatography (ethyl acetate-hexane), thereby obtaining 1.58 g of octyl 10-(((4-nitrophenoxy)carbonyl)oxy)hexadecanoate (A3) as a colorless oily substance.

¹H-NMR (CDCl₃) δ:8.28 (2H, d, J = 9.2 Hz), 7.38 (2H, d, J = 9.2 Hz), 4.86-4.76 (1H, m), 4.05 (2H, t, J = 6.7 Hz), 2.28 (2H, t, J = 7.5 Hz), 1.77-1.52 (8H, m), 1.47-1.21 (28H, m), 0.95-0.82 (6H, m).

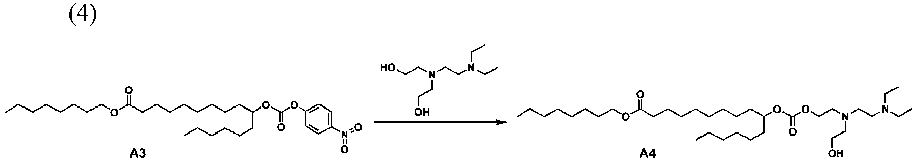

To a 16 mL tetrahydrofuran solution of 1.58 g of octyl 10-(((4-nitrophenoxy)carbonyl)oxy)hexadecanoate (A3), 1.47 g of 2,2'-((2-(diethylamino)ethyl)azanediyl)bis(ethan-1-ol), 1.05 g of 4-dimethylaminopyridine, and 1.2 mL of triethylamine were added, and the mixture was stirred at 80°C for 5 hours. Ethyl acetate and water were added to the reaction mixture, and the organic layer was isolated. The organic layer was washed with saturated saline, and anhydrous sodium sulfate was then added thereto to dry the organic layer, and the solvent was distilled off under reduced pressure. The obtained residue was purified by silica gel column chromatography (methanol-ethyl acetate), and then purified by NH silica gel column chromatography (ethyl acetate-hexane), thereby obtaining 1.05 g of octyl 3-ethyl-12-hexyl-6-(2-hydroxyethyl)-10-oxo-9,11-dioxa-3,6-diazahenicosan-21-oate (A4) as a colorless oily substance.

¹H-NMR (CDCl₃) δ:4.72-4.63 (1H, m), 4.19 (2H, t, J = 5.9 Hz), 4.05 (2H, t, J = 6.7 Hz), 3.53 (2H, t, J = 5.0 Hz), 2.88 (2H, t, J = 6.1 Hz), 2.73-2.64 (4H, m), 2.58-2.50 (4H, m), 2.47 (2H, t, J = 6.0 Hz), 2.28 (2H, t, J = 7.6 Hz), 1.66-1.45 (10H, m), 1.38-1.20 (26H, m), 1.15 (6H, t, J = 7.1 Hz), 0.92-0.83 (6H, m).

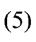

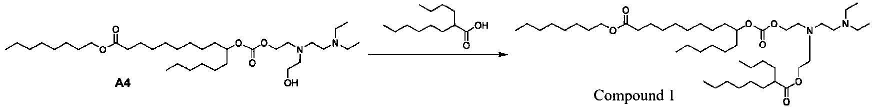

To a 3 mL dichloromethane solution of 300 mg of octyl 3-ethyl-12-hexyl-6-(2-hydroxyethyl)-10-oxo-9,11-dioxa-3,6-diazahenicosan-21-oate (A4), 153 mg of 2-butyloctanoic acid, 191 mg of 1-ethyl-3-(3-dimethylaminopropyl)carbodiimide hydrochloride, 179 mg of 4-dimethylaminopyridine, and 0.4 mL of triethylamine were added, and the mixture was stirred overnight at room temperature. The solvent was distilled off from the reaction mixture under reduced pressure, ethyl acetate and water were added thereto, and the organic layer was isolated. The organic layer was washed with saturated saline, and anhydrous sodium sulfate was then added thereto to dry the organic layer, and the solvent was distilled off under reduced pressure. The obtained residue was purified by silica gel column chromatography (methanol-ethyl acetate), and then purified by NH silica gel column chromatography (ethyl acetate-hexane), thereby obtaining 358 mg of octyl 6-(2-((2-butyloctanoyl)oxy)ethyl)-3-ethyl-12-hexyl-10-oxo-9,11-dioxa-3,6-diazahenicosan-21 -oate (Compound 1) as a colorless oily substance.

¹H-NMR (CDCl₃) δ:4.71-4.63 (1H, m), 4.19-4.09 (4H, m), 4.05 (2H, m), 2.87-2.78 (4H, m), 2.71-2.63 (2H, m), 2.57-2.47 (6H, m), 2.34-2.24 (3H, m), 1.67-1.49 (12H, m), 1.47-1.18 (40H, m), 1.01 (6H, t, J = 7.1 Hz), 0.93-0.83 (12H, m).

MS m/z (M+H): 798.

### [Example 2]

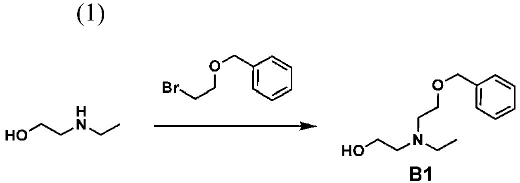

To a 32 mL ethanol solution of 1.6 g of 2-(ethylamino)ethanol, 5.0 g of benzyl 2-bromoethyl ether and 7.4 g of potassium carbonate were added, and the mixture was stirred at room temperature for 15 minutes and then stirred under heating reflux for 12 hours. The insoluble matter in the reaction mixture was removed by filtration, and the filtrate was concentrated under reduced pressure. The obtained residue was purified by NH silica gel column chromatography (ethyl acetate-hexane), thereby obtaining 3.13 g of 2-((2-(benzyloxy)ethyl)(ethyl)amino)ethan-1-ol (B1) as a colorless oily substance.

¹H-NMR (CDCl₃) δ:7.37-7.23 (5H, m), 4.53 (2H, s), 3.58-3.53 (4H, m), 2.76 (2H, t, J = 5.7 Hz), 2.70-2.61 (4H, m), 1.04 (3H, t, J = 7.1 Hz).

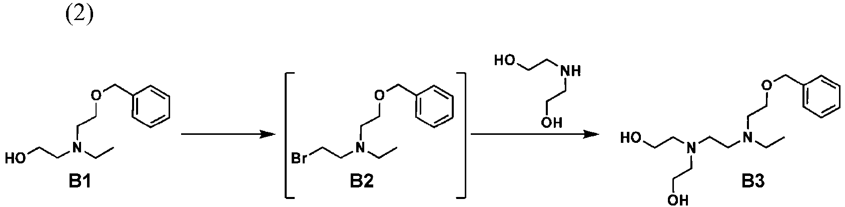

To a 16 mL tetrahydrofuran solution of 1.0 g of 2-((2-(benzyloxy)ethyl)(ethyl)amino)ethan-1-ol (B1), 3.0 g of carbon tetrabromide was added, then 4 mL of a tetrahydrofuran solution of 2.3 g of triphenylphosphine was added dropwise thereto under ice cooling, and the mixture was stirred at room temperature for 1 hour, thereby obtaining a mixture of 2-(benzyloxy)-N-(2-bromoethyl)-N-ethyl ethan-1-amine (B2). 52 mL of ethanol, 0.95 g of diethanolamine, and 1.9 g of potassium carbonate were added to the obtained reaction mixture, and the mixture was stirred at room temperature for 15 minutes and then stirred under heating reflux for 3 hours. The insoluble matter in the reaction mixture was removed by filtration, and the filtrate was concentrated under reduced pressure. The obtained residue was purified by NH silica gel column chromatography (ethyl acetate-hexane), thereby obtaining 1.22 g of 2,2'-((2-((2-(benzyloxy)ethyl)(ethyl)amino)ethyl)azandiyl)bis(ethan-1-ol) (B3) as a light yellow oily substance.

¹H-NMR (CDCl₃) δ:7.39-7.24 (5H, m), 4.53 (2H, s), 3.66-3.51 (6H, m), 2.82-2.55 (14H, m), 1.07 (3H, t, J = 7.0 Hz).

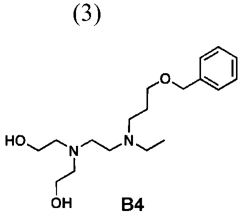

2,2'-((2-((3-(benzyloxy)propyl)(ethyl)amino)ethyl)azanediyl)bis(ethan-1-ol) (B4) was obtained by the same manner as in (1) and (2) of Example 2.

¹H-NMR (CDCl₃) δ:7.38-7.23 (5H, m), 4.49 (2H, s), 3.61-3.54 (4H, m), 3.50 (2H, t, J = 6.1 Hz), 2.73-2.48 (12H, m), 1.87-1.74 (2H, m), 1.06 (3H, t, J = 7.1 Hz).

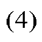

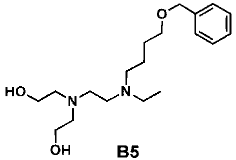

2,2'-((2-((4-(benzyloxy)butyl)(ethyl)amino)ethyl)azanediyl)bis(ethan-1-ol) (B5) was obtained by the same manner as in (1) and (2) of Example 2.

¹H-NMR (CDCl₃) δ:7.36-7.24 (5H, m), 4.49 (2H, s), 3.62-3.54 (4H, m), 3.52-3.45 (2H, m), 2.73-2.45 (12H, m), 1.65-1.53 (4H, m), 7.04 (3H, t, J = 7.0 Hz).

### [Example 3]

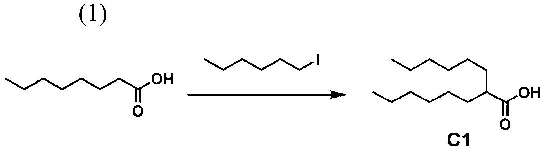

1.5 g of octanoic acid was added portionwise to a mixture of 0.45 g of 60% sodium hydride and 15 mL of tetrahydrofuran under ice cooling, and then the mixture was stirred at the same temperature for 30 minutes. 12 mL of 1 mol/L lithium diisopropylamide (tetrahydrofuran-heptane solution) was added dropwise to the reaction mixture under ice-cooling, the mixture was stirred at room temperature for 30 minutes, then 2.7 g of 1-iodohexane was added dropwise thereto, and the mixture was stirred at 45°C for 2 hours. Ethyl acetate and ice water were added to the reaction mixture, and a 1N hydrochloric acid aqueous solution was added thereto until the reaction mixture became acidic. The organic layer was isolated, then washed with saturated saline, anhydrous sodium sulfate was added thereto to dry the organic layer, and the solvent was distilled off under reduced pressure. The obtained residue was purified by silica gel column chromatography (ethyl acetate-hexane), thereby obtaining 1.33 g of 2-hexyloctanoic acid (C1) as a yellow oily substance.

¹H-NMR (CDCl₃) δ:2.41-2.31 (1H, m), 1.67-1.41 (4H, m), 1.36-1.20 (16H, m), 0.94-0.83 (6H, m).

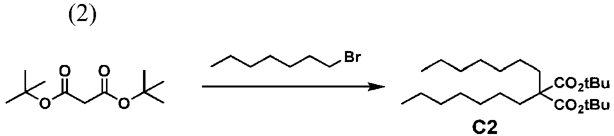

6.6 g of powdered potassium hydroxide was added to a mixture of 50 mL of dimethyl sulfoxide and 30 mL of toluene, 10 mL of a toluene solution of 10 g of di-tert-butyl malonate and 10 mL of a toluene solution of 19.9 g of 1-bromoheptane were added thereto under ice cooling, and the mixture was stirred overnight at 30°C or lower. The reaction mixture was stirred in an ice bath, hydrochloric acid was added thereto for neutralization, and then the organic layer was isolated. The obtained organic layer was washed with water and saturated saline, anhydrous sodium sulfate was added thereto for drying, and the solvent was distilled off under reduced pressure, thereby obtaining 22.9 g of di-tert-butyl 2,2-diheptylmalonate (C2) as a light yellow oily substance.

¹H-NMR (CDCl₃) δ:1.79-1.74 (4H, m), 1.44 (18H, s), 1.32-1.10 (20H, m), 0.87 (6H, t, J = 6.8 Hz).

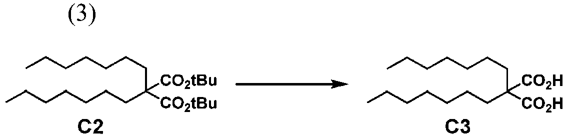

45 mL of trifluoroacetic acid was added to a mixture of 22.9 g of di-tert-butyl 2,2-diheptylmalonate (C2), 23 mL of toluene, and 2.3 mL of water, the mixture was stirred at room temperature for 2 hours, and the solvent was distilled off under reduced pressure. 23 mL of hexane was added to the residue, the mixture was stirred under ice cooling, and then the resulting solid was collected by filtration. The obtained solid was dried to obtain 11.7 g of 2,2-diheptylmalonic acid (C3) as a white solid.

¹H-NMR (CDCl₃) δ:1.97-1.92 (4H, m), 1.32-1.17 (20H, m), 0.87 (6H, t, J = 7.3 Hz).

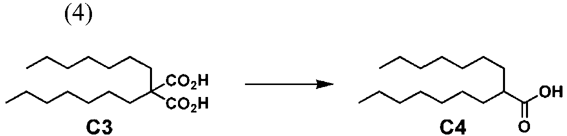

11.7 g of 2,2-diheptylmalonic acid (C3) was stirred at 165°C for 3 hours, thereby obtaining 11.7 g of 2-heptylnonanoic acid (C4) as a light yellow oily substance.

¹H-NMR (CDCl₃) δ:2.39-2.32 (1H, m), 1.96-1.93 (1H, m), 1.68-1.41 (4H, m), 1.36-1.16 (20H, m), 0.89-0.85 (6H, m).

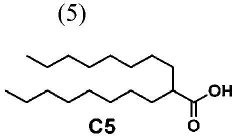

2-Octyldecanoic acid (C5) as a light yellow solid was obtained by the same method as in (2) to (4) of Example 3, except that 1-bromooctane was used instead of 1-bromoheptane in (2) of Example 3.

¹H-NMR (CDCl₃) δ:2.38-2.31 (1H, m), 1.68-1.41 (4H, m), 1.36-1.18 (24H, m), 0.89-0.86 (6H, m).

### [Example 4]

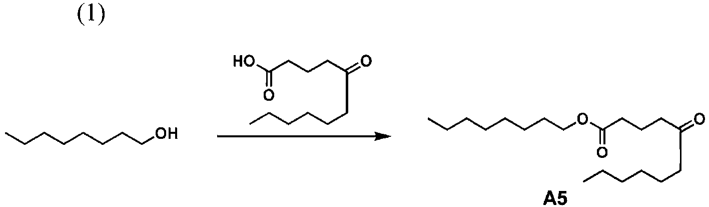

To a 30 mL toluene solution of 4.0 g of 5-oxoundecanoic acid synthesized according to the method described in WO2019/235635A, 2.4 mL of 1-octanol and 130 mg of 4-toluenesulfonic acid monohydrate were added, and the mixture was stirred under heating reflux for 5 hours. After distilling off the solvent in the reaction mixture under reduced pressure, the residue was purified by NH silica gel column chromatography (ethyl acetate-hexane) to obtain 4.6 g of octyl 5-oxoundecanoate (A5) as a light yellow oily substance.

¹H-NMR (CDCl₃) δ:4.05 (2H, t, J = 6.7 Hz), 2.46 (2H, t, J = 7.2 Hz), 2.41-2.28 (4H, m), 1.93-1.83 (2H, m), 1.65-1.49 (4H, m), 1.37-1.20 (16H, m), 0.92-0.83 (6H, m).

(2)
Intermediates (A6) to (A9) in Table 1 were obtained by the same method as in (1) of Example 1 or (1) of Example 4.

**[Table 1]**

| Compound No. | Structure | Physical property data |
|---|---|---|
| A6 | 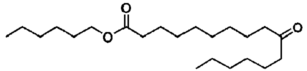 | ¹H-NMR (CDCl₃) δ :4. 05 (2H, t, J=6.7Hz), 2. 37 (4H, t, J=7.4Hz) , 2.28 (2H, t, J=7.6Hz), 1.66-1.50 (8H, m), 1.39-1.21 (20H, m), 0. 92-0. 84 (6H, m). |
| A7 | 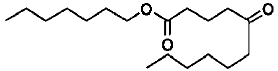 | ¹H-NMR (CDCl₃) δ :4.05 (2H, t, J=6. 7Hz), 2. 46 (2H, t, J=7.2Hz), 2.38 (2H, t, J=7.5Hz), 2.32 (2H, t, J=7.2Hz), 1.93-1.83 (2H, m), 1. 66-1. 49 (4H, m), 1.37-1.20 (14H, m), 0.91-0.84 (6H, m). |
| A8 | 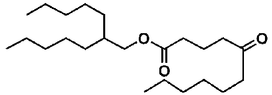 | ¹H-NMR (CDCl₃) δ :3. 97 (2H, d, J=5. 1Hz), 2.47 (2H, t, J=7.2Hz), 2.39 (2H, t, J=7. 2Hz), 2.33 (2H, t, J=7. 2Hz), 1. 95-1. 83 (2H, m), 1. 66-1. 49 (3H, m), 1.36-1.20 (22H, m), 0.92-0.82 (9H, m). |
| A9 | 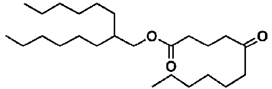 | ¹H-NMR (CDCl₃) δ : 3. 96 (2H, d, J=5. 7Hz), 2.47 (2H, t, J=7.2Hz), 2. 42-2. 29 (4H, m), 1. 93-1. 83 (2H, m), 1. 65-1. 50 (3H, m), 1. 35-1. 18 (26H, m), 0. 92-0. 82 (9H, m). |

### [Example 5]

Intermediates (A10) to (A14) in Table 2 were obtained by the same method as in (2) of Example 1, using Intermediates (A5) to (A9) as raw materials.

**[Table 2]**

| Compound No. | Structure | Physical property data |
|---|---|---|
| A10 | 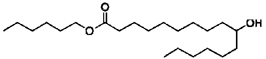 | ¹H-NMR(CDCl₃) δ :4.05 (2H, t, J=6. 7Hz), 3.61-3.52 (1H, m), 2.28 (2H, t, J=7.6Hz), 1.66-1.57 (4H, m), 1.49-1.21 (28H, m), 0.93-0.84 (6H, m). |
| A11 | 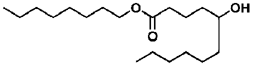 | ¹H-NMR (CDCl₃) δ :4. 06 (2H, t, J=6.7Hz), 3.64-3.54 (1H, m), 2.33 (2H, t, J=7. 2Hz), 1.84-1.21 (26H, m), 0.93-0.83 (6H, m). |
| A12 | 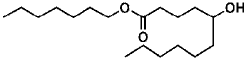 | ¹H-NMR (CDCl₃) δ :4.06 (2H, t, J=6. 7Hz) , 3.63-3.54 (1H, m), 2.33 (2H, t, J=7.2Hz), 1.84-1.21 (24H, m), 0.93-0.84 (6H, m). |
| A13 | 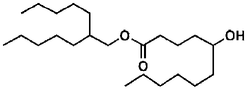 | ¹H-NMR (CDCl₃) δ :3.98 (2H, d, J=6.0Hz), 3.63-3.57 (1H, m), 2.11-2.28 (2H, m), 1.84-1.22 (32H, m), 0.88 (9H, t, J=7.2Hz). |
| A14 | 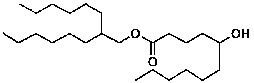 | ¹H-NMR (CDCl₃) δ :3.97 (2H, d, J=5.7Hz), 3.63-3.54 (1H, m), 2.34 (2H, t, J=7.0Hz), 1.83-1.38 (7H, m), 1.36-1.19 (28H, m), 0.92-0.83 (9H, m). |

### [Example 6]

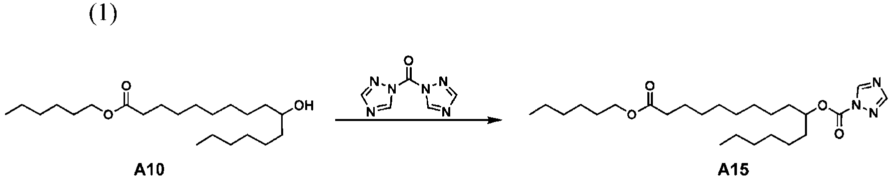

To a 18 mL tetrahydrofuran solution of 1.8 g of hexyl 10-hydroxyhexadecanoate (A10), 1.3 g of 1,1'-carbonyldi(1,2,4-triazole) was added, and the mixture was stirred at room temperature for 6 hours. Ethyl acetate and water were added to the reaction mixture, and the organic layer was isolated. The organic layer was washed with saturated saline, and anhydrous sodium sulfate was then added thereto to dry the organic layer, and the solvent was distilled off under reduced pressure. The obtained residue was purified by silica gel column chromatography (ethyl acetate-hexane) to obtain 2.3 g of 16-(hexyloxy)-16-oxohexadecan-7-yl 1H-1,2,4-triazole-1-carboxylate (A15) as a colorless oily substance.

¹H-NMR (CDCl₃) δ:8.81 (1H, s), 8.07 (1H, s), 5.21-5.11 (1H, m), 4.05 (2H, t, J = 6.7 Hz), 2.28 (2H, t, J = 7.5 Hz), 1.84-1.65 (4H, m), 1.65-1.53 (4H, m), 1.44-1.21 (24H, m), 0.92-0.83 (6H, m).

(2)
Intermediates (A16) to (A19) in Table 3 were obtained by the same method as in (1) of Example 6, using Intermediates (A11) to (A14) as raw materials.

**[Table 3]**

| Compound No. | Structure | Physical property data |
|---|---|---|
| A16 | 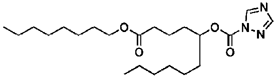 | ¹H-NMR(CDCl₃) δ :8. 82 (1H, s), 8.07 (1H, s), 5.21-5.13 (1H, m), 4.05 (2H, t, J=6.7Hz), 2.35 (2H, t, J=6.9Hz), 1.88-1.67 (6H, m), 1.65-1.50 (2H, m), 1.43-1.20 (18H, m), 0.93-0.82 (6H, m). |
| A17 | 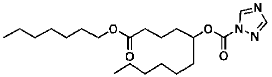 | ¹H-NMR(CDCl₃) δ :8.82 (1H, s), 8.07 (1H, s), 5.22-5.13 (1H, m), 4.05 (2H, t, J=6.7Hz), 2. 35 (2H, t, J=6.9Hz), 1.87-1.67 (6H, m), 1.65-1.50 (2H, m), 1.43-1.19 (16H, m), 0.93-0.79 (6H, m). |
| A18 | 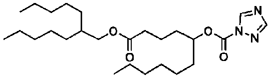 | ¹H-NMR(CDCl₃) δ :8.82 (1H, s), 8.07 (1H, s), 5.22-5.13 (1H, m), 3.97 (2H, d, J=5. 8Hz), 2.36 (2H, t, J=6.9Hz), 1.88-1.49 (7H, m), 1.45-1.17 (24H, m), 0.94-0.82 (9H, m). |
| A19 | 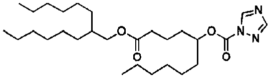 | ¹H-NMR(CDCl₃) δ :8. 82 (1H, s), 8.07 (1H, s), 5.21-5.12 (1H, m), 3.96 (2H, d, J=5. 8Hz), 2.36 (2H, t, J=6.9Hz), 1.88-1.52 (7H, m), 1.44-1.18 (28H, m), 0.90-0.82 (9H, m). |

### [Example 7]

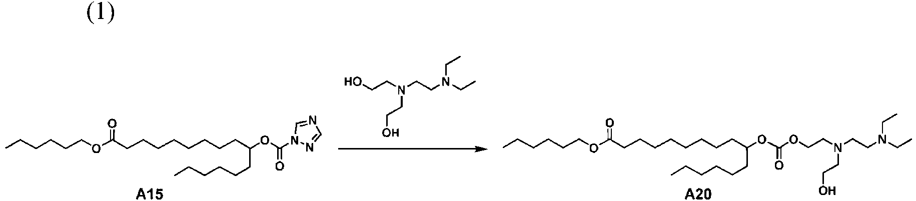

To a 23 mL acetonitrile solution of 2.3 g of 16-(hexyloxy)-16-oxohexadecan-7-yl 1H-1,2,4-triazole-1-carboxylate (A15), 2.1 g of 2,2'-((2-(diethylamino)ethyl)azanediyl)bis(ethan-1-ol) and 2.3 mL of 1,8-diazabicyclo[5.4.0]-7-undecene were added, and the mixture was stirred at 50°C for 3 hours. Ethyl acetate and water were added to the reaction mixture, and the organic layer was isolated. The organic layer was washed with saturated saline, and anhydrous sodium sulfate was then added thereto to dry the organic layer, and the solvent was distilled off under reduced pressure. The obtained residue was purified by silica gel column chromatography (methanol-ethyl acetate), and then purified by NH silica gel column chromatography (ethyl acetate-hexane), thereby obtaining 1.5 g of hexyl 3-ethyl-12-hexyl-6-(2-hydroxyethyl)-10-oxo-9,11-dioxa-3,6-diazahenicosan-21-oate (A20) as a colorless oily substance.

¹H-NMR (CDCl₃) δ:4.71-4.63 (1H, m), 4.19 (2H, t, J = 5.8 Hz), 4.05 (2H, t, J = 6.7 Hz), 3.53 (2H, t, J = 5.0 Hz), 2.88 (2H, t, J = 6.1 Hz), 2.73-2.64 (4H, m), 2.58-2.50 (4H, m), 2.47 (2H, t, J = 5.9 Hz), 2.28 (2H, t, J = 7.6 Hz), 1.70-1.45 (8H, m), 1.41-1.21 (24H, m), 1.02 (6H, t, J = 7.1 Hz), 0.93-0.82 (6H, m).

MS m/z (M+H): 588.

(2)
Intermediates (A21) to (A24) in Table 4 were obtained by the same method as in (1) of Example 7, using Intermediates (A16) to (A19) as raw materials.

**[Table 4]**

| Compound No. | Structure | Physical property data |
|---|---|---|
| A21 | 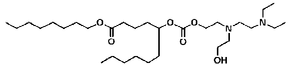 | ¹H-NMR(CDCl₃) δ :4.74-4.62 (1H, m), 4.20 (2H, t, J=6. 1Hz), 4.05 (2H, t, J=6.7Hz), 3.58-3.50 (2H, m), 2. 88 (2H, t, J=6.1Hz), 2.75-2.40 (10H, m), 2.31 (2H, t, J=7.0Hz), 1.75-1.48 (8H, m), 1.38-1.19 (18H, m), 1.11-0.97 (6H, m), 0.92-0.84 (6H, m). |
| A22 | 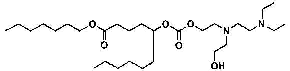 | ¹H-NMR (CDCl₃) δ :4.74-4.65 (1H, m), 4.23-4.16 (2H, m), 4.05 (2H, t, J=6.7Hz), 3.57-3.50 (2H, m), 2.88 (2H, t, J=6. 1Hz), 2.73-2.62 (4H, m), 2.59-2.50 (4H, m), 2.47 (2H, t, J=5.9Hz), 2.31 (2H, t, J=7.0Hz), 1.72-1.47 (8H, m), 1.38-1.20 (16H, m), 1.02 (6H, t, J=7.1Hz), 0.92-0.82 (6H, m). |
| | | MS m/z (M+H) :532. |
| A23 | 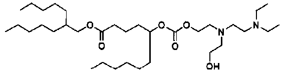 | ¹H-NMR(CDCl₃) δ :4.73-4.65 (1H, m), 4.24-4.14 (2H, m), 3.96 (2H, d, J=5.8Hz), 3.57-3.52 (2H, m), 2.88 (2H, t, J=6.1Hz), 2.74-2.64 (4H, m), 2.62-2.43 (6H, m), 2.32 (2H, t, J=6. 9Hz), 1.74-1.51 (7H, m), 1.36-1.19 (24H, m), 1.08-0.99 (6H, m), 0.92-0.82 (9H, m). |
| | | MS m/z (M+H) :602. |
| A24 | 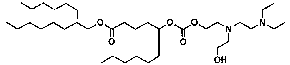 | ¹H-NMR (CDCl₃) δ :4.73-4.65 (1H, m), 4.25-4.17 (2H, m), 3.96 (2H, d, J=5.7Hz), 3. 56-3.50 (2H, m), 2.88 (2H, t, J=6.0Hz), 2.75-2.41 (10H, m), 2.32 (2H, t, J=6.9Hz), 1.73-1.47 (7H, m), 1.35-1.20 (28H, m), 1.12-0.96 (6H, m), 0.92-0.81 (9H, m). |
| | | MS m/z (M+H) :630. |

### [Example 8]

Intermediates (A25) to (A27) in Table 5 were obtained by the same method as in (1) of Example 7, using Intermediate (A16) and Intermediates (B3) to (B5) as raw materials.

**[Table 5]**

| Compound No. | Structure | Physical property data |
|---|---|---|
| A25 | 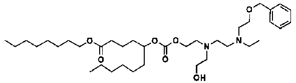 | ¹H-NMR (CDCl₃) δ : 7.37-7.24 (5H, m), 4.73-4.66 (1H, m), 4.51 (2H, s), 4.23-4.13 (2H, m), 4.05 (2H, t, J=6. 7Hz), 3.64-3.48 (4H, m), 2.85 (2H, t, J=6.0Hz), 2.80-2.47 (10H, m), 2.30 (2H, t, J=7.0Hz), 1.77-1.48 (8H, m), 1.38-1.19 (18H, m) , 1.10-0.97 (3H, m), 0.92-0.80 (6H, m). |
| | | MS m/z (M+H) : 652. |
| A26 | 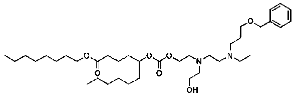 | ¹H-NMR(CDCl₃) δ:7.37-7.22 (5H, m), 4.72-4.65 (1H, m), 4.49 (2H, s), 4.21-4.17 (2H, m), 4.04 (2H, t, J=6.7Hz) , 3.60-3. 45 (4H, m), 2.86 (2H, t, J=5.9Hz), 2.74-2.44 (10H, m), 2.30 (2H, t, J=6.9Hz), 1.79-1.43 (10H, m), 1.37-1.18 (18H, m), 1.10-0.97 (3H, m), 0.93-0.83 (6H, m). |
| | | MS m/z (M+H) : 666. |
| A27 | 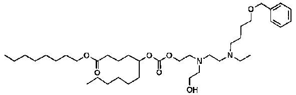 | ¹H-NMR (CDCl₃) δ : 7.37-7.23 (5H, m), 4.74-4.63 (1H, m), 4.49 (2H, s), 4.25-4.16 (2H, m), 4.05 (2H, t, J=6. 7Hz), 3.59-3.43 (4H, m), 2.87 (2H, t, J=6.0Hz), 2.74-2.61 (4H, m), 2.60-2.39 (6H, m), 2.31 (2H, t, J=7.0Hz), 1.74-1.45 (8H, m), 1.37-1.19 (22H, m), 1.07-0.97 (3H, m), 0.93-0.82 (6H, m). |
| | | MS m/z (M+H) : 680. |

### [Example 9]

Intermediates (A20) to (A27), Intermediates (Cl), (C4), and (C5), and 3-heptyldecanoic acid described in WO2019/235635A as raw materials were used to obtain the compound group shown in Table 6, including hexyl 3-ethyl-12-hexyl-6-(2-((2-heptyloctanoyl)oxy)ethyl)-10-oxo-9,11-dioxa-3,6-diazahenicosan-2 1-oate (compound 2), heptyl 3-ethyl-6-(2-((2-heptylnonanoyl)oxy)ethyl)-12-hexyl-10-oxo-9,11-dioxa-3,6-diazahexadecan-16-oate (Compound 3), heptyl 3-ethyl-12-hexyl-6-(2-((2-octyldecanoyl)oxy)ethyl)-10-oxo-9,11-dioxa-3,6-diazahexadecan-1 6-oate (Compound 4), heptyl 3-ethyl-12-hexyl-6-(2-((2-hexyloctanoyl)oxy)ethyl)-10-oxo-9,11-dioxa-3,6-diazahexadecan-1 6-oate (Compound 5), heptyl 3-ethyl-12-hexyl-6-(2-((3-heptyldecanoyl)oxy)ethyl)-10-oxo-9,11-dioxa-3,6-diazahexadecan-16-oate (Compound 6), octyl 3-ethyl-6-(2-((2-heptylnonanoyl)oxy)ethyl)-12-hexyl-10-oxo-9,11-dioxa-3,6-diazahexadecan-16-oate (Compound 7), octyl 3-ethyl-6-(2-((3-heptyldecanoyl)oxy)ethyl)-12-hexyl-10-oxo-9,11-dioxa-3,6-diazahexadecan-16-oate (Compound 8), 2-pentylheptyl 3-ethyl-6-(2-((3-heptyldecanoyl)oxy)ethyl)-12-hexyl-10-oxo-9,11-dioxa-3,6-diazahexadecan-16-oate (Compound 9), 2-hexyloctyl 3-ethyl-6-(2-((3-heptyldecanoyl)oxy)ethyl)-12-hexyl-10-oxo-9,11-dioxa-3,6-diazahexadecan-16-oate (Compound 10), octyl 5-ethyl-8-(2-((3-heptyldecanoyl)oxy)ethyl)-14-hexyl-12-oxo-1-phenyl-2,11,13-trioxa-5,8-diaz adodecane-18-oate (Compound 11), octyl 6-ethyl-9-(2-((3-heptyldecanoyl)oxy)ethyl)-15-hexyl-13-oxo-1-phenyl-2,12,14-trioxa-6,9-daza nonadecane-19-oate (Compound 12), and octyl 7-ethyl-10-(2-((3-heptyldecanoyl)oxy)ethyl)-16-hexyl-14-oxo-1-phenyl-2,13,15-trioxa-7,10-d azaicosane-20-oate (Compound 13), by the same method as in (5) of Example 1.

**[Table 6]**

| Compound No. | Structure | Physical property data |
|---|---|---|
| 2 | 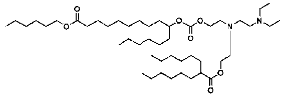 | ¹H-NMR(CDCl₃) δ :4.72-4.62 (1H, m), 4.19-4.08 (4H, m), 4.05 (2H, t, J=6.7Hz), 2.88-2.77 (4H, m), 2.70-2.62 (2H, m), 2.57-2.47 (6H, m), 2.35-2.24 (3H, m), 1.65-1.17 (52H, m), 1.01 (6H, t, J=7.1Hz), 0.93-0.82 (12H, m). |
| | | MS m/z (M+H) :798. |
| 3 | 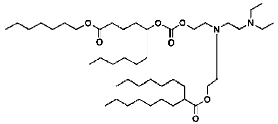 | 1H-NMR(CDCl₃) δ :4.72-4.64 (1H, m) , 4.20-4.10 (4H, m), 4.05 (2H, t, J=6.7Hz), 2.88-2.77 (4H, m), 2.72-2.62 (2H, m), 2.58-2.46 (6H, m), 2.36-2.25 (3H, m), 1.73-1.18 (48H, m), 1.01 (6H, t, J=7.1Hz), 0.92-0.82 (12H, m). |
| | | MS m/z (M+H) :770. |
| 4 | 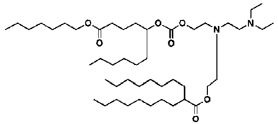 | ¹H-NMR (CDCl₃) δ : 4.72-4.64 (1H, m), 4.21-4.09 (4H, m), 4.05 (2H, t, J=6.7Hz), 2.87-2.76 (4H, m), 2.71-2.63 (2H, m), 2.58-2. 47 (6H, m), 2.35-2.25 (3H, m), 1.73-1.18 (52H, m), 1.01 (6H, t, J=7.1Hz), 0.93-0.83 (12H, m). |
| | | MS m/z (M+H) : 798. |
| 5 | 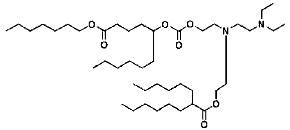 | ¹H-NMR(CDCl₃) δ :4.73-4.64 (1H, m), 4.20-4.08 (4H, m), 4.05 (2H, t, J=6.7Hz), 2.87-2.77 (4H, m), 2.7-2.63 (2H, m), 2.59-2.46 (6H, m), 2.35-2.26 (3H, m), 1.74-1.17 (44H, m), 1.01 (6H, t, J=7.1Hz), 0.93-0.82 (12H, m). |
| | | MS m/z (M+H) :742. |
| 6 | 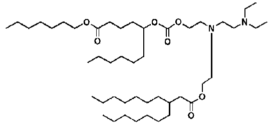 | ¹H-NMR(CDCl₃) δ :4.72-4.64 (1H, m), 4.19-4.09 (4H, m), 4.05 (2H, t, J=6.7Hz), 2.87-2.76 (4H, m), 2.74-2.46 (8H, m), 2.31 (2H, t, J=7.0Hz), 2. 22 (2H, d, J=6.9Hz), 1.88-1.78 (1H, m), 1.70-1.50 (8H, m), 1.39-1.18 (40H, m), 1.07-0.96 (6H, m), 0.92-0.81 (12H, m). |
| | | MS m/z (M+H) :784. |
| 7 | 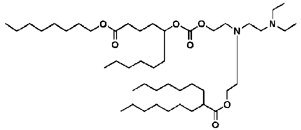 | ¹H-NMR(CDCl₃) δ :4.73-4.63 (1H, m), 4.23-4.08 (4H, m), 4.05 (2H, t, J=6.7Hz), 2.88-2.76 (4H, m), 2.75-2.43 (8H, m), 2.36-2.25 (3H, m), 1.74-1.17 (50H, m), 1.09-0.95 (6H, m), 0.93-0.81 (12H, m). |
| | | MS m/z (M+H) :784. |
| 8 | 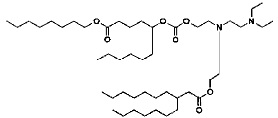 | ¹H-NMR (CDCl₃) δ :4.73-4.64 (1H, m), 4.20-4.09 (4H, m), 4.05 (2H, t, J=6.7Hz), 2. 89-2.77 (4H, m), 2.73-2.42 (8H, m), 2.31 (2H, t, J=7.0Hz), 2.22 (2H, d, J=6.8Hz) , 1.88-1.77 (1H, m), 1.71-1.50 (8H, m), 1.38-1.19 (42H, m), 1.10-0.96 (6H, m), 0.92-0.83 (12H, m). |
| | | MS m/z (M+H) :798. |
| 9 | 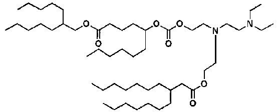 | ¹H-NMR(CDCl₃) δ :4.73-4.64 (1H, m), 4.20-4.08 (4H, m), 3.6 (2H, d, J=5.8Hz) , 2.88-2.77 (4H, m), 2.74-2. 44 (8H, m), 2.32 (2H, t, J=6.9Hz), 2.22 (2H, d, J=6.8Hz), 1.88-1.78 (1H, m), 1.68-1.49 (7H, m), 1.38-1.19 (48H, m), 1.08-0.95 (6H, m), 0.93-0.82 (15H, m). |
| | | MS m/z (M+H) :854. |
| 10 | 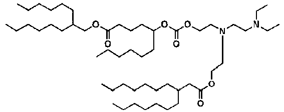 | ¹H-NMR (CDCl₃) δ :4.73-4.65 (1H, m), 4.21-4.07 (4H, m), 3.96 (2H, d, J=5.7Hz), 2.89-2. 77 (4H, m), 2.72-2.44 (8H, m), 2.32 (2H, t, J=6. 9Hz), 2.22 (2H, d, J=6.8Hz), 1.87-1.77 (1H, m), 1.71-1.47 (7H, m), 1.36-1.18 (52H, m), 1.09-0.95 (6H, m), 0.92-0.82 (15H, m). |
| | | MS m/z (M+H) :882. |
| 11 | 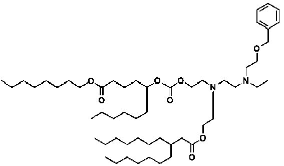 | ¹H-NMR (CDCl₃) δ :7.37-7. 24 (5H, m), 4. 72-4. 63 (1H, m), 4. 52 (2H, s), 4. 17-4. 00 (6H, m), 3. 53 (2H, t, J=5. 9Hz), 2. 86-2. 74 (4H, m), 2. 73-2. 62 (4H, m), 2.61-2.52 (4H, m), 2.30 (2H, t, J=7.0Hz), 2.21 (2H, d, J=6.8Hz), 1.87-1.76 (1H, m), 1.71-1.49 (8H, m), 1. 37-1. 16 (42H, m), 1. 01 (3H, t, J=6. 9Hz), 0.93-0.82 (12H, m). |
| | | MS m/z (M+H) :904. |
| 12 | 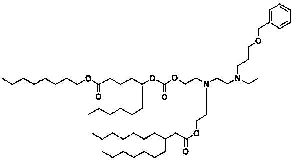 | ¹H-NMR (CDCl₃) δ :7.38-7. 23 (5H, m), 4. 72-4. 64 (1H, m), 4.49 (2H, s), 4. 21-4. 07 (4H, m), 4.04 (2H, t, J=6.7Hz), 3.54-3.47 (2H, m), 2.86-2.73 (4H, m), 2.68-2.59 (2H, m), 2.57-2.45 (6H, m), 2.30 (2H, t, J=6.9Hz), 2.21 (2H, d, J=6.9Hz)1.86-1.48 (11H, m), 1. 37-1. 19 (42H, m), 1.00 (3H, t, J=6. 6Hz), 0.93-0.82 (12H, m). |
| | | MS m/z (M+H) :918. |
| 13 | 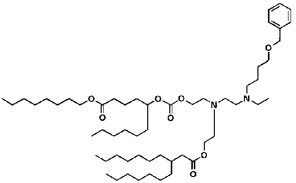 | ¹H-NMR(CDCl₃) δ :7.37-7.22 (5H, m), 4.73-4.62 (1H, m), 4.50(2H, s), 4.19-4.08 (4H, m), 4.05 (2H, t, J=6.7Hz), 3.48 (2H, t, J=6.3Hz), 2.86-2.75 (4H, m), 2.69-2.59 (2H, m), 2.56-2.37 (6H, m), 2.30 (2H, t, J=7.0Hz), 2.21 (2H, d, J=6.9H), 1.87-1.77 (1H, m), 1.72-1.45 (8H, m), 1.40-1.19 (46H, m), 0.99 (3H, t, J=6.9Hz), 0.93-0.83 (12H, m). |
| | | MS m/z (M+H) :932. |

### [Example 10]

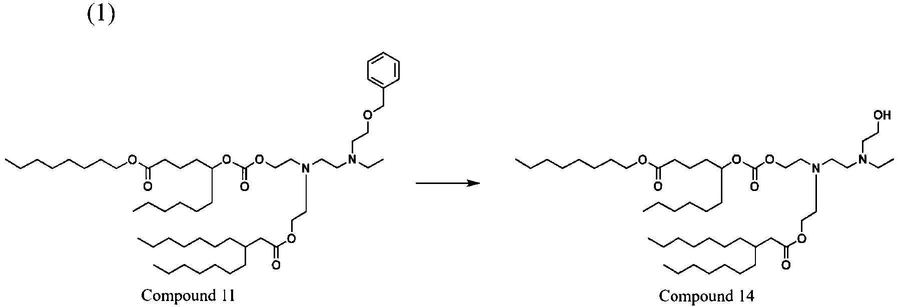

To a 5 mL methanol solution of 240 mg of octyl 5-ethyl-8-(2-((3-heptyldecanoyl)oxy)ethyl)-14-hexyl-12-oxo-1-phenyl-2,11,13-trioxa-5,8-diaz adodecane-18-oate (Compound 11), 72 mg of 10% palladium on carbon (55% water wet) and 167 mg of ammonium formate were added, and the mixture was stirred under reflux for 3 hours. The insoluble matter was removed by filtration through Celite, and the filtrate was concentrated under reduced pressure. The obtained residue was purified by NH silica gel column chromatography (ethyl acetate-hexane) to obtain 147 mg of octyl 3-ethyl-6-(2-((3-heptyldecanoyl)oxy)ethyl)-12-hexyl-1-hydroxy-10-oxo-9,11-dioxa-3,6-diazah exadecan-16-oate (Compound 14) as a colorless oily substance.

¹H-NMR (CDCl₃) δ:4.74-4.64 (1H, m), 4.24-4.11 (4H, m), 4.05 (2H, t, J = 6.7 Hz), 3.62-3.48 (2H, m), 2.89-2.77 (4H, m), 2.76-2.45 (8H, m), 2.31 (2H, t, J = 6.9 Hz), 2.22 (2H, d, J = 6.8 Hz), 1.87-1.78 (1H, m), 1.76-1.47 (8H, m), 1.38-1.17 (42H, m), 1.12-0.98 (3H, m), 0.94-0.82 (12H, m).

MS m/z (M+H): 814.

(2)
Octyl 4-ethyl-7-(2-((3-heptyldecanoyl)oxy)ethyl)-13-hexyl-1-hydroxy-11-oxo-10,12-dioxa-4,7-diaza heptadecan-17-oate (Compound 15), and octyl 5-ethyl-8-(2-((3-heptyldecanoyl)oxy)ethyl)-14-hexyl-1-hydroxy-12-oxo-11,13-dioxa-5,8-diaza octadecan-18-oate (Compound 16) which were the compounds in Table 7 were obtained by the same method as in (1) of Example 10.

**[Table 7]**

| Compound No. | Structure | Physical property data |
|---|---|---|
| 15 | 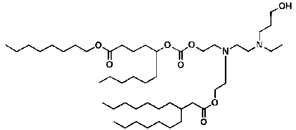 | ¹H-NMR(CDCl₃) δ :4.72-4.65 (1H, m), 4.22-4.10 (4H, m), 4.05 (2H, t, J=6.7Hz), 3. 83-3. 73 (2H, m), 2.88-2.41 (12H, m), 2.31 (2H, t, J=7.0Hz), 2.22 (2H, d, J=6.8Hz), 1.85-1.78 (1H, m), 1.74-1.46 (10H, m), 1.38-1.17 (42H, m), 1.11-0. 99 (3H, m), 0.93-0.82 (12H, m). |
| | | MS m/z (M+H) :828. |
| 16 | 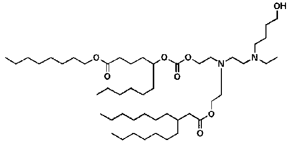 | ¹H-NMR (CDCl₃) δ :4.73-4.64 (1H, m), 4.20-4.08 (4H, m), 4.05 (2H, t, J=6.7Hz), 3.62-3.50 (2H, m), 2.87-2.65 (6H, m), 2.61-2.39 (6H, m), 2.31 (2H, t, J=6.9Hz), 2.22 (2H, d, J=6.8Hz), 1.86-1.78 (1H, m), 1.74-1.48 (10H, m), 1.37-1.17 (44H, m), 1.12-0.99 (3H, m), 0.93-0.82 (12H, m). |
| | | MS m/z (M+H) :842. |

### [Example 11]

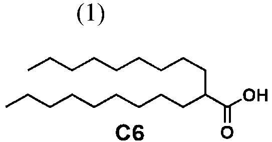

2-Nonylundecanoic acid (C6) as a light yellow solid was obtained by the same method as in (2) to (4) of Example 3, except that 1-bromononane was used instead of 1-bromoheptane in (2) of Example 3.

¹H-NMR (CDCl₃) δ: 2.39-2.28 (1H, m), 1.70-1.55 (2H, m), 1.54-1.39 (2H, m), 1.37-1.16 (28H, m), 0.93-0.81 (6H, m).

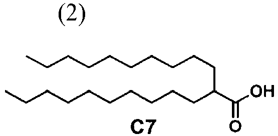

2-Decyldodecanoic acid (C7) as a light yellow solid was obtained by the same method as in (2) to (4) of Example 3, except that 1-bromodecane was used instead of 1-bromoheptane in (2) of Example 3.

¹H-NMR (CDCl₃) δ: 2.40-2.28 (1H, m), 1.70-1.54 (2H, m), 1.54-1.39 (2H, m), 1.38-1.17 (32H, m), 0.93-0.81 (6H, m).

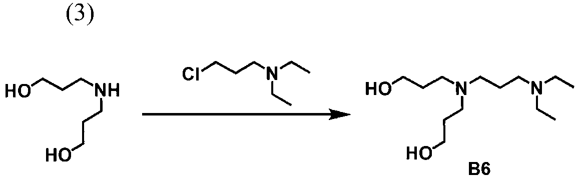

To a 20 mL acetonitrile solution of 2.0 g of 3,3'-azanediylbis(propan-1-ol), 3.2 g of 3-chloro-N,N-diethylpropan-1-amine and 5.2 g of N,N-diisopropylethylamine were added, and the mixture was stirred at 60°C for 5 hours. The solvent was distilled off under reduced pressure, and the obtained residue was purified by NH silica gel column chromatography (ethyl acetate-methanol) to obtain 3.1 g of 3,3'-((3-(diethylamino)propyl)azanediyl)bis(propan-1-ol) (B6) as a light yellow oily substance.

¹H-NMR (CDCl₃) δ: 3.74 (4H, t, J = 5.6 Hz), 2.62 (4H, t, J = 6.3 Hz), 2.53 (4H, q, J = 7.1 Hz), 2.48-2.39 (4H, m), 1.89-1.54 (8H, m), 1.02 (6H, t, J = 7.1 Hz).

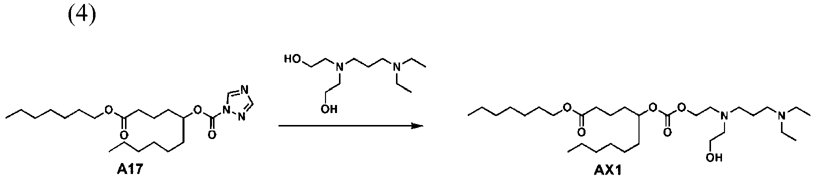

Heptyl 3-ethyl-13-hexyl-7-(2-hydroxyethyl)-11-oxo-10,12-dioxa-3,7-diazaheptadecan-17-oate (AX1) was obtained by the same method as in (1) of Example 7, using Intermediate (A17) as a raw material, except that 2,2'-((3-(diethylamino)propyl)azanediyl)bis(ethan-1-ol) described in WO2022/230964A was used instead of 2,2'-((2-(diethylamino)ethyl)azanediyl)bis(ethan-1-ol).

¹H-NMR (CDCl₃) δ: 4.74-4.62 (1H, m), 4.22-4.11 (2H, m), 4.05 (2H, t, J = 6.8 Hz), 3.60-3.53 (2H, m), 2.76 (2H, t, J = 6.1 Hz), 2.66-2.40 (10H, m), 2.35-2.27 (2H, m), 1.77-1.47 (8H, m), 1.38-1.20 (18H, m), 1.02 (6H, t, J = 7.2 Hz), 0.93-0.82 (6H, m).

MS m/z (M+H): 546.

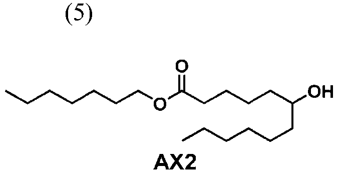

Heptyl 6-hydroxydodecanoate (AX2) was obtained in the same manner as in (1) of Example 84 described in WO2019/235635A, except that monomethyl adipate was used instead of 10-methoxy-10-oxodecanoic acid, and 1-heptanol was used instead of 2-butyloctan-1-ol.

¹H-NMR (CDCl₃) δ: 4.06 (2H, t, J = 6.8 Hz), 3.65-3.53 (1H, m), 2.32 (2H, t, J = 7.4 Hz), 1.74-1.20 (27H, m), 0.94-0.82 (6H, m).

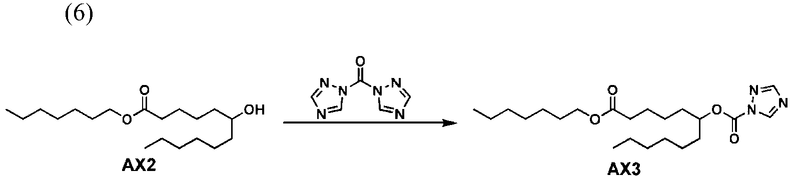

1-(Heptyloxy)-1-oxododecan-6-yl 1H-1,2,4-triazole-1-carboxylate (AX3) was obtained by the same method as in (1) of Example 6, using Intermediate (AX2) as a raw material.

¹H-NMR (CDCl₃) δ: 8.82 (1H, s), 8.07 (1H, s), 5.21-5.12 (1H, m), 4.04 (2H, t, J = 6.8 Hz), 2.31 (2H, t, J = 7.4 Hz), 1.87-1.50 (10H, m), 1.43-1.19 (18H, m), 0.93-0.81 (6H, m).

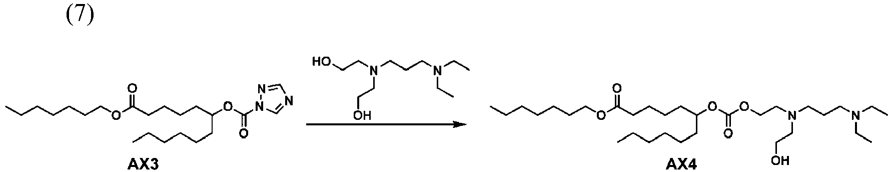

Heptyl 3-ethyl-13-hexyl-7-(2-hydroxyethyl)-11-oxo-10,12-dioxa-3,7-diazaoctadecan-18-oate (AX4) was obtained by the same method as in (4) of Example 11, using Intermediate (AX3) as a raw material.

¹H-NMR (CDCl₃) δ: 4.74-4.63 (1H, m), 4.23-4.11 (2H, m), 4.05 (2H, t, J = 6.8 Hz), 3.60-3.53 (2H, m), 2.76 (2H, t, J = 6.1 Hz), 2.66-2.40 (10H, m), 2.30 (2H, t, J = 7.5 Hz), 1.71-1.47 (10H, m), 1.45-1.20 (19H, m), 1.02 (6H, t, J = 7.1 Hz), 0.93-0.82 (6H, m).

MS m/z (M+H): 560.

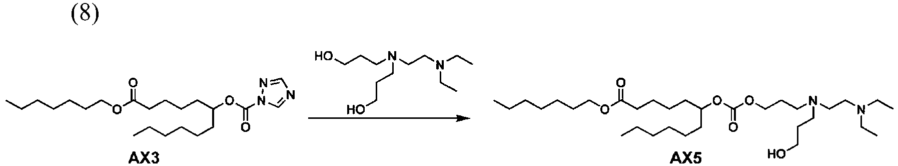

Heptyl 3-ethyl-13-hexyl-6-(3-hydroxypropyl)-11-oxo-10,12-dioxa-3,6-diazaoctadecan-18-oate (AX5) was obtained by the same method as in (1) of Example 7, using Intermediate (AX3) as a raw material, except that 3,3'-((2-(diethylamino)ethyl)azanediyl)bis(propan-1-ol) described in WO2022/230964A was used instead of 2,2'-((2-(diethylamino)ethyl)azanediyl)bis(ethan-1-ol).

¹H-NMR (CDCl₃) δ: 5.83 (1H, brs), 4.74-4.63 (1H, m), 4.23-4.11 (2H, m), 4.05 (2H, t, J = 6.7 Hz), 3.72 (2H, t, J = 5.3 Hz), 2.63-2.46 (12H, m), 2.30 (2H, t, J = 7.5 Hz), 1.91-1.79 (2H, m), 1.73-1.46 (10H, m), 1.45-1.20 (18H, m), 1.02 (6H, t, J = 7.2 Hz), 0.93-0.83 (6H, m).

MS m/z (M+H): 538.

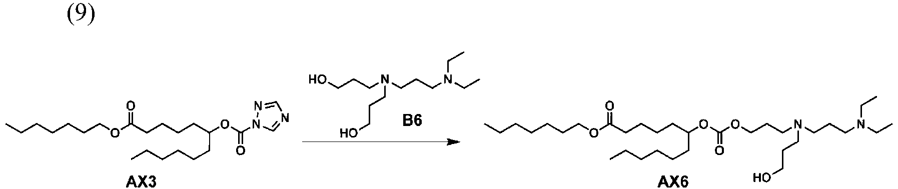

Heptyl 3-ethyl-14-hexyl-7-(3-hydroxypropyl)-12-oxo-11,13-dioxa-3,7-diazanonadecan-19-oate (AX6) was obtained by the same method as in (1) of Example 7, using Intermediate (AX3) as a raw material, except that Intermediate (B6) was used instead of 2,2'-((2-(diethylamino)ethyl)azanediyl)bis(ethan-1-ol).

¹H-NMR (CDCl₃) δ: 4.99 (1H, brs), 4.74-4.63 (1H, m), 4.22-4.11 (2H, m), 4.05 (2H, t, J = 6.8 Hz), 3.81-3.74 (2H, m), 2.68-2.60 (2H, m), 2.59-2.36 (10H, m), 2.30 (2H, t, J = 7.5 Hz), 1.92-1.80 (2H, m), 1.73-1.46 (12H, m), 1.45-1.20 (18H, m), 1.01 (6H, t, J = 7.2 Hz), 0.92-0.84 (6H, m).

MS m/z (M+H): 589.

### [Example 12]

Intermediate (A22), (AX1), (AX4), (AX5), (AX6), Intermediates (C1), (C4), (C5), (C6), (C7), 2-hexyldecanoic acid, and 3-heptyldecanoic acid described in WO2019/235635A as raw materials were used to obtain the compound group in Table 8, including heptyl 3-ethyl-12-hexyl-6-(2-((2-nonylundecanoyl)oxy)ethyl)-10-oxo-9,11-dioxa-3,6-diazahexadecan -16-oate (Compound 17), heptyl 6-(2-((2-decyldodecanoyl)oxy)ethyl)-3-ethyl-12-hexyl-10-oxo-9,11-dioxa-3,6-diazahexadecan -16-oate (Compound 18), heptyl 3-ethyl-12-hexyl-6-(2-((2-hexyldecanoyl)oxy)ethyl)-10-oxo-9,11-dioxa-3,6-diazahexadecan-1 6-oate (Compound 19), heptyl 3-ethyl-13-hexyl-7-(2-((2-hexyloctanoyl)oxy)ethyl)-11-oxo-10,12-dioxa-3,7-diazaheptadecan-17-oate (Compound 20), heptyl 3-ethyl-13-hexyl-7-(2-((2-hexyloctanoyl)oxy)ethyl)-11-oxo-10,12-dioxa-3,7-diazaoctadecan-1 8-oate (Compound 21), heptyl 3-ethyl-7-(2-((2-heptylnonanoyl)oxy)ethyl)-13-hexyl-11-oxo-10,12-dioxa-3,7-diazaoctadecan-18-oate (Compound 22), heptyl 3-ethyl-13-hexyl-7-(2-((2-hexyloctanoyl)oxy)ethyl)-11-oxo-10,12-dioxa-3,7-diazaoctadecan-1 8-oate (Compound 23), heptyl 3-ethyl-13-hexyl-7-(2-((2-hexyloctanoyl)oxy)ethyl)-11-oxo-10,12-dioxa-3,7-diazaoctadecan-1 8-oate (Compound 24), heptyl 3-ethyl-7-(2-((3-heptyldecanoyl)oxy)ethyl)-13-hexyl-11-oxo-10,12-dioxa-3,7-diazaoctadecan-18-oate (Compound 25), heptyl 3-ethyl-13-hexyl-6-(3-((2-hexyloctanoyl)oxy)propyl)-11-oxo-10,12-dioxa-3,6-diazaoctadecan-18-oate (Compound 26), Heptyl 3-ethyl-6-(3-((2-heptylnonanoyl)oxy)propyl)-13-hexyl-11-oxo-10,12-dioxa-3,6-diazauctadeca n-18-oate (Compound 27), heptyl 3-ethyl-13-hexyl-6-(3-((2-octyldecanoyl)oxy)propyl)-11-oxo-10,12-dioxa-3,6-diazauctadecan-18-oate (Compound 28), heptyl 3-ethyl-13-hexyl-6-(3-((2-nonylundecanoyl)oxy)propyl)-11-oxo-10,12-dioxa-3,6-diazauctadec an-18-oate (Compound 29), heptyl 3-ethyl-13-hexyl-6-(3-((2-hexyldecanoyl)oxy)propyl)-11-oxo-10,12-dioxa-3,6-diazauctadecan -18-oate (Compound 30), heptyl 3-ethyl-6-(3-((3-heptyldecanoyl)oxy)propyl)-13-hexyl-11-oxo-10,12-dioxa-3,6-diazauctadeca n-18-oate (Compound 31), heptyl 3-ethyl-14-hexyl-7-(3-((2-hexyloctanoyl)oxy)propyl)-12-oxo-11,13-dioxa-3,7-diazanonadecan -19-oate (Compound 32), heptyl 3-ethyl-7-(3-((2-heptylnonanoyl)oxy)propyl)-14-hexyl-12-oxo-11,13-dioxa-3,7-diazanonadeca n-19-oate (Compound 33), heptyl 3-ethyl-14-hexyl-7-(3-((2-octyldecanoyl)oxy)propyl)-12-oxo-11,13-dioxa-3,7-diazanonadecan -19-oate (Compound 34), heptyl 3-ethyl-14-hexyl-7-(3-((2-nonylundecanoyl)oxy)propyl)-12-oxo-11,13-dioxa-3,7-diazanonade can-19-oate (Compound 35), heptyl 3-ethyl-14-hexyl-7-(3-((2-hexyldecanoyl)oxy)propyl)-12-oxo-11,13-dioxa-3,7-diazanonadeca n-19-oate (Compound 36), and heptyl 3-ethyl-7-(3-((3-heptyldecanoyl)oxy)propyl)-14-hexyl-12-oxo-11,13-dioxa-3,7-diazanonadeca n-19-oate (Compound 37), by the same method as in (5) of Example 1.

**[Table 8]**

| Compound No. | Structure | Physical property data |
|---|---|---|
| 17 | 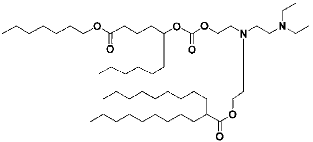 | ¹H-NMR(CDCl₃) δ : 4. 72-4. 64 (1H, m), 4. 20-4. 08 (4H, m), 4.05 (2H, t, J=6. 8Hz), 2.89-2.75 (4H, m), 2.74-2.21 (11H, m), 1. 74-1. 49 (14H, m), 1. 48-1. 16 (42H, m), 1. 12-0.95 (6H, m), 0. 93-0. 82 (12H, m). |
| | | MS m/z (M+H) :826. |
| 18 | 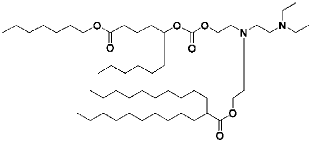 | ¹H-NMR (CDCl₃) δ : 4.72-4.65 (1H, m), 4. 19-4. 09 (4H, m), 4.05 (2H, t, J-6.7Hz), 2. 88-2. 75 (4H, m), 2. 73-2. 43 (8H, m), 2. 35-2. 25 (3H, m), 1. 73-1. 17 (60H, m), 1. 11-0. 95 (6H, m), 0. 94-0. 82 (12H, m). |
| | | MS m/z (M+H) :854. |
| 19 | 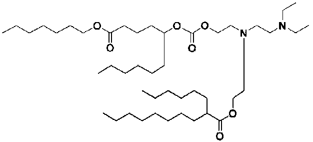 | 1H-NMR(CDCl₃) δ : 4.73-4.65 (1H, m), 4. 22-4. 10 (4H, m), 4. 05 (2H, L, J=6. 7Hz), 2. 87-2. 76 (4H, m), 2. 72-2. 63 (2H, m), 2. 57-2. 47 (6H, m), 2. 36-2. 26 (3H, m), 1. 75-1. 50 (14H, m), 1. 48-1. 20 (34H, m), 1. 06-0. 98 (6H, m), 0.93-0.82 (12H, m). |
| | | MS m/z (M+H) :770. |
| 20 | 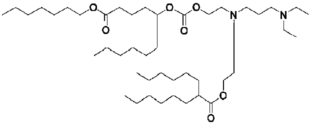 | ¹H-NMR(CDCl₃) δ : 4. 73-4. 65 (1H, m), 4.22-4.09 (4H, m), 4. 05 (2H, t, J=6. 7Hz), 2. 85-2. 73 (4H, m), 2. 60-2. 46 (6H, m), 2. 44-2. 38 (2H, m, 2. 35-2. 26 (3H, m), 1. 73-1. 18 (46H, m), 1. 05-0. 98 (6H, m), 0. 93-0. 82 (12H, m). |
| | | MS m/z (M+H) : 756. |
| 21 | 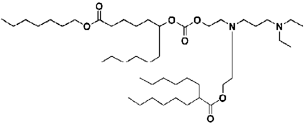 | ¹H-NMR (CDCl₃) δ : 4. 72-4. 63 (1H, m), 4. 20-4. 10 (4H, m), 4. 05 (2H, t, J=6. 7Hz), 2. 85-2. 74 (4H, m), 2. 60-2. 49 (6H, m), 2. 47-2. 39 (2H, m), 2. 36-2. 26 (3H, m), 1. 74-1. 18 (48H, m), 1. 06-0. 98 (6H, m), 0. 92-0. 83 (12H, m). |
| | | MS m/z (M+H) :770. |
| 22 | 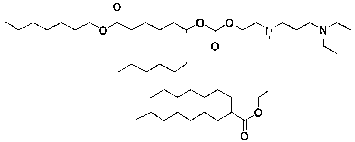 | ¹H-NMR (CDCl₃) δ : 4. 71-4. 63 (1H, m), 4. 21-4. 09 (4H, m), 4.05 (2H, t, J=6.7Hz), 2.85-2.74 (4H, m), 2.60-2.48 (6H, m), 2.46-2.39 (2H, m), 2. 35-2. 26 (3H, m), 1. 73-1. 18 (52H, m), 1. 07-0. 98 (6H, m), 0.92-0.83 (12H, m). |
| | | MS m/z (M+H) :798. |
| 23 | 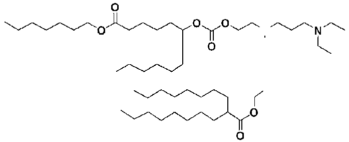 | ¹H-NMR(CDCl₃) δ : 4. 71-4.63 (1H, m), 4.21-4.09 (4H, m), 4.05 (2H, t, J=6. 7Hz), 2.84-2.73 (4H, m), 2. 59-2. 47 (6H, m), 2. 45-2. 39 (2H, m), 2. 34-2. 26 (3H, m), 1. 68-1. 19 (56H, m), 1. 06-0. 97 (6H, m), 0. 92-0.84 (12H, m). |
| | | MS m/z (M+H) :826. |
| 24 | 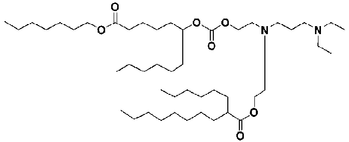 | ¹H-NMR (CDCl₃) δ : 4. 71-4. 64 (1H, m), 4. 21-4. 09 (4H, m), 4.05 (2H, t, J=6. 7Hz), 2. 85-2. 74 (1H, m), 2. 60-2. 47 (6H, m), 2. 45-2. 38 (2II, m), 2. 35-2. 26 (3H, m), 1. 69-1. 18 (52H, m), 1.05-0.97 (6H, m), 0.93-0.83 (12H, m). |
| 25 | 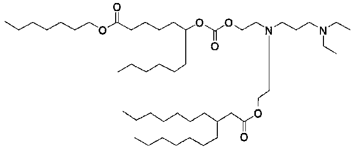 | ¹H-NMR (CDCl₃) δ : 4.72-4.63 (1H, m), 4. 21-4. 08 (4H, m), 4. 05 (2H, t, J=6. 7Hz) 2. 84-2. 73 (4H, m), 2. 60-2. 47 (6H, m), 2. 44-2. 38 (2H, m), 2.29 (2H, t, J=7.5Hz), 2.22 (2H, d, J=6.9Hz), 1.88-1.77 (1H, m), 1.69-1.20 (52H, m), 1.06-0. 97 (6H, m), 0. 93-0. 82 (12H, m). |
| | | MS m/z (M+H) :812. |
| 26 | 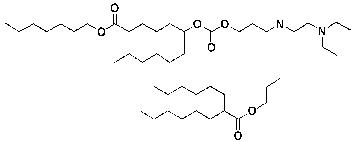 | ¹H-NMR (CDCl₃) δ : 4.71-4. 63 (1H, m), 4. 21-4. 02 (6H, m), 2. 59-2. 46 (12H, m), 2. 34-2. 26 (3H, m), 1. 84-1. 70 (4H, m), 1. 69-1. 19 (46H, m), 1. 06-0. 98 (6H, m), 0. 92-0.83 (12H, m). |
| | | MS m/z (M+H) :784. |
| 33 | 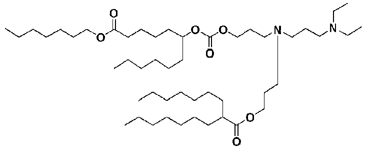 | ¹H-NMR (CDCl₃) δ : 4. 72-4.63 (1H, m), 4.20-4.01 (6H, m), 2. 55-2. 37 (12H, m), 2. 35-2. 26 (3H, m), 1. 84-1. 69 (4H, m), 1. 68-1. 18 (52H, m), 1.05-0.96 (6H, m), 0.93-0.82 (12H, m). |
| | | MS m/z (M+H) :798. |
| 34 | 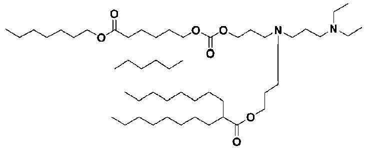 | ¹H-NMR(CDCl₃) δ : 4.71-4. 64 (1H, m), 4.21-4.02 (6H, m), 2. 55-2. 38 (12H, m), 2. 34-2. 25 (3H, m), 1. 84-1. 70 (4H, m), 1. 68-1. 17 (56H, m), 1.05-0. 97 (6H, m), 0. 94-0. 82 (12H, m). |
| | | MS m/z (M+H) :854. |
| 35 | 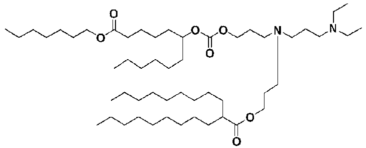 | ¹H-NMR(CDCl₃) δ : 4. 72-4.63 (1H, m), 4.21-4.02 (6H, m), 2. 55-2. 38 (12H, m), 2. 35-2. 26 (3H, m), 1.84-1.70 (4H, m), 1.69-1.18 (60H, m), 1.05-0.98 (6H, m), 0.93-0.83 (12H, m). |
| | | MS m/z (M+H):882. |
| 36 | 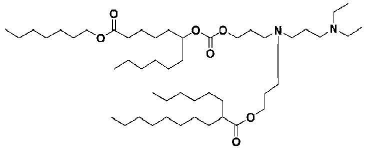 | ¹H-NMR (CDCl₃) δ : 4. 72-4.63 (1H, m), 4.20-4.02 (6H, m), 2. 56-2. 37 (12H, m), 2. 34-2. 26 (3H, m), 1. 85-1. 69 (4H, m), 1. 68-1. 19 (52H, m), 1.05-0.96 (6H, m), 0.93-0.83 (12H, m). |
| | | MS m/z (M+H) :826. |
| 37 | 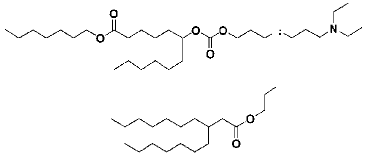 | ¹H-NMR (CDCl₃) δ : 4. 72-4.63 (1H, m), 4.21-4.02 (6H, m), 2. 56-2. 37 (12H, m), 2.29 (2H, t, J=7.5Hz), 2.21 (2H, d, J=6.9Hz), 1.87-1.70 (5H, m), 1.68-1.17 (52H, m), 1.05-0.97 (6H, m), 0.92-0.82 (12H, m). |
| | | MS m/z (M+H):840. |
| 27 | 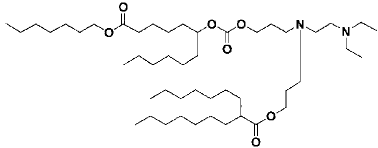 | ¹H-NMR (CDCl₃) & : 4.71-4. 64 (1H, m), 4. 22-4. 02 (6H, m), 2. 56-2. 45 (12H, m), 2. 34-2. 25 (3H, m), 1. 85-1. 70 (4H, m), 1. 68-1. 19 (50H, m), 1.05-0. 98 (6H, m), 0. 92-0. 83 (12H, m). |
| | | MS m/z (M+H) :812. |
| 28 | 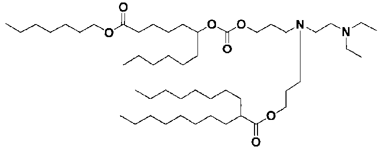 | ¹H-NMR (CDCl₃) δ : 4. 71-4. 63 (1H, m), 4. 21-4. 02 (6H, m), 2. 57-2. 44 (12H, m), 2. 35-2. 25 (3H, m), 1. 84-1. 71 (4H, m), 1. 68-1. 18 (54H, m), 1. 05-0. 98 (6H, m), 0. 92-0. 83 (12H, m). |
| | | MS m/z (M+H) :840. |
| 29 | 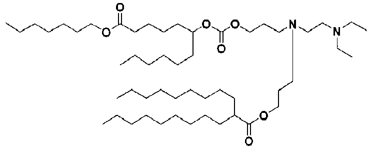 | ¹H-NMR (CDCl₃) δ : 4. 71-4. 63 (1H, m), 4. 21-4. 02 (6H, m), 2. 58-2. 46 (12H, m), 2. 33-2. 25 (3H, m), 1. 84-1. 71 (4H, m), 1. 67-1. 19 (54H, m), 1. 05-0. 98 (6H, m), 0. 92-0. 83 (12H, m). |
| | | MS m/z (M+H) :868. |
| 30 | 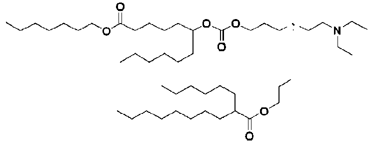 | ¹H-NMR (CDCl₃) δ : 4. 72-4. 63 (1H, m), 4. 21-4. 02 (6H, m), 2. 58-2. 46 (12H, m), 2. 34-2. 25 (3H, m), 1. 84-1. 71 (4H, m), 1. 68-1. 18 (50H, m), 1. 06-0. 98 (6H, m), 0. 92-0. 83 (12H, m). |
| | | MS m/z (M+H) :812. |
| 31 | 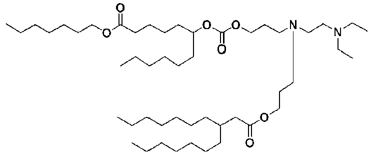 | ¹H-NMR(CDCl₃) δ : 4. 72-4. 64 (1H, m), 4. 20-4. 12 (2H, m), 4. 12-4.02 (4H, m), 2.58-2.44 (12H, m), 2.29 (2H, t, J=7.5Hz), 2.21 (2H, d, J=6.8Hz), 1.88-1.70 (5H, m), 1.67-1.19 (50H, m), 1.06-0.98 (6H, m), 0. 93-0.83 (12H, m). |
| | | MS m/z (M+H) :826. |
| 32 | 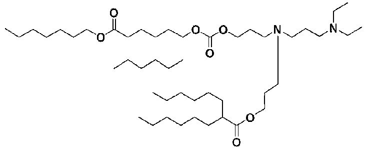 | ¹H-NMR(CDCl₃) δ : 4. 72-4. 63 (1H, m), 4. 21-4. 03 (6H, m), 2. 55-2. 38 (12H, m), 2. 35-2. 25 (3H, m), 1. 84-1. 69 (4H, m), 1. 68-1. 19 (48H, m), 1. 06-0. 96 (6H, m), 0. 93-0. 81 (12H, m). |

### Test Example 1: Preparation of mRNA-encapsulating lipid nanoparticles and measurement of reporter protein expression rate in mice

### <Preparation of Erythropoietin (EPO) mRNA-encapsulating lipid nanoparticles>

The compounds shown in Table 9, 1,2-distearoyl-sn-glycero-3-phosphocholine (product name: COATSOME (R) MC-8080; NOF corporation) (hereinafter, DSPC), cholesterol (product name: Cholesterol HP; Nippon Seika Co., Ltd.), and 1,2-dimyristoyl-rac-glycero-3-(methylpolyoxyethylene 2000) (hereinafter, DMG-PEG2000) (product name: SUNBRIGHT (R) GM-020; NOF corporation) were dissolved in ethanol at a molar ratio (mol%) of compounds shown in Table 9:1,2-distearoyl-sn-glycero-3-phosphocholine:cholesterol:DMG-PEG2000 = 50:10:38.5:1.5 such that the total lipid concentration was 12.5 mmol/L, thereby obtaining an oil phase.

EPO mRNA (product name: CleanCap EPO mRNA (5moU); TriLink) was diluted with 50 mmol/L citrate buffer having a pH 4.0 such that the weight ratio of total lipid concentration to mRNA concentration is about 20:1, thereby obtaining a water phase. Then, the water phase and the oil phase were mixed together using NanoAssemblr (Precision NanoSystems) such that the volume ratio of water phase:oil phase was 3:1, and the mixed solution was 2-fold diluted with phosphate buffered physiological saline (PBS) or water, thereby obtaining a dispersion liquid of mRNA lipid nanoparticles. The dispersion liquid was dialyzed with 20 mM Tris buffer solution containing 8% sucrose using a dialysis cassette (Slide-A-Lyzer G2, MWCO: 10 kD, Thermo Fisher Scientific) to remove ethanol, thereby obtaining EPO mRNA-encapsulating lipid nanoparticles.

### <Measurement of particle diameter and polydispersity index (PdI)>

The particle diameter and the polydispersity index (PdI) of the mRNA-encapsulating lipid nanoparticles were obtained by measuring a dispersion liquid of lipid nanoparticles diluted with phosphate buffered physiological saline (PBS) using a particle size analyzer NanoSAQLA (Otsuka Electronics Co., Ltd.). The results are shown in Table 9.

### <Evaluation of encapsulation rate of mRNA>

### (Quantification of total mRNA concentration)

EPO mRNA was diluted with MilliQ water to prepare a diluted sample in 2-fold dilution series from 100 µg/mL to 3.1 µg/mL, and a standard solution for calibration was prepared. 50 µL of the standard solution for calibration or the mRNA lipid nanoparticles was mixed with 450 µL of methanol to prepare a measurement solution.

The absorbance of each measurement solution at 260 nm and 330 nm was measured using a UV plate reader (Multiskan Go, Thermo Fisher Scientific), the absorbance at 330 nm was subtracted from the absorbance at 260 nm, and the result was used as the absorbance of each measurement solution. The total water phase nucleic acid concentration was calculated from the calibration curve using the absorbance of each sample measurement solution.

### (Quantification of mRNA concentration in outer water phase)

The concentration of the outer water phase nucleic acid was quantified by a standard addition method using a QuanT-iT RiboGreen RNA Assay Kit (Thermo Fisher Scientific). First, a 20× TE buffer included in the above kit was diluted with water, thereby obtaining a 1× TE buffer. TE represents Tris/EDTA (ethylenediaminetetraacetic acid). EPO mRNA was diluted with a TE buffer such that the final concentration was 0 to 400 ng/mL to prepare a nucleic acid diluted series. 10 µL of mRNA lipid nanoparticles diluted 5-fold with TE buffer and 90 µL of a nucleic acid dilution series were mixed in a 96-well plate, 100 µL of a Ribogreen reagent diluted 200-fold with TE buffer was added to each well, and then fluorescence (excitation wavelength: 485 nm, fluorescence wavelength: 535 nm) was measured using a fluorescence plate reader (Infinite 200 Pro M nano +, TECAN). From the obtained results, the outer water phase nucleic acid concentration of each measurement solution was calculated according to the standard addition method.

### (Calculation of encapsulation rate)

By using the quantification results of the total mRNA concentration and the mRNA concentration in the outer water phase that were obtained through the above steps, the mRNA encapsulation rate of the mRNA lipid nanoparticles was calculated according to the following Equation. The results are shown in Table 9.

mRNA encapsulation rate (%) = (total mRNA concentration-mRNA concentration in outer water phase)/total mRNA concentration × 100

**[Table 9]**

| Particle diameter, polydispersity index (PdI), nucleic acid encapsulation rate of nucleic acid-encapsulating lipid nanoparticles | | | |
|---|---|---|---|
| Compounds used | Particle diameter (nm) | PdI | Nucleic acid encapsulation rate |
| Comparative Example | 161 | 0.07 | 50% |
| Compound 1 | 112 | 0.03 | 89% |
| Compound 2 | 103 | 0.03 | 90% |
| Compound 3 | 105 | 0.06 | 84% |
| Compound 4 | 85 | 0.12 | 90% |
| Compound 5 | 118 | 0.07 | 79% |
| Compound 6 | 98 | 0.11 | 77% |
| Compound 7 | 82 | 0.12 | 79% |
| Compound 8 | 89 | 0.12 | 78% |
| Compound 9 | 77 | 0.13 | 85% |
| Compound 10 | 75 | 0.10 | 86% |
| Compound 17 | 79 | 0.15 | 95% |
| Compound 18 | 79 | 0.13 | 95% |
| Compound 19 | 91 | 0.09 | 94% |
| Compound 20 | 137 | 0.10 | 87% |
| Compound 21 | 129 | 0.12 | 89% |
| Compound 22 | 95 | 0.10 | 94% |
| Compound 23 | 85 | 0.11 | 95% |
| Compound 24 | 98 | 0.13 | 94% |
| Compound 25 | 102 | 0.16 | 94% |

### <Measurement of EPO enzyme activity>

The dispersion liquid of mRNA lipid nanoparticles prepared in above <Preparation of EPO mRNA-encapsulating lipid nanoparticles> was intravenously administered to ICR mice at a mRNA dosage of 0.1 mg/kg. 6 hours after the administration, blood was collected from the caudal vena cava, thereby obtaining plasma. The human EPO enzyme activity was quantified by using the obtained blood plasma and Erythropoietin (EPO) Human Elisa Kit (Abcam.plc). The quantitative value is described as a relative amount of EPO protein in a case where Comparative Example is set to 1.

The results are shown in Table 10.

**[Table 10]**

| EPO expression level of nucleic acid-encapsulating lipid nanoparticles | |
|---|---|
| Compounds used | Relative amount of EPO protein (with respect to Comparative Example 1) |
| Comparative Example | 1.00 |
| Compound 2 | 1.61 |
| Compound 3 | 3.16 |
| Compound 4 | 3.09 |
| Compound 5 | 2.28 |
| Compound 6 | 4.91 |
| Compound 7 | 3.82 |
| Compound 8 | 3.79 |
| Compound 9 | 2.30 |
| Compound 10 | 1.72 |
| Compound 17 | 1.91 |
| Compound 18 | 1.30 |
| Compound 19 | 4.31 |
| Compound 20 | 1.68 |
| Compound 21 | 1.65 |
| Compound 22 | 6.09 |
| Compound 23 | 4.71 |
| Compound 24 | 6.54 |
| Compound 25 | 4.77 |

In comparison with the nucleic acid lipid composition in Comparative Examples, the nucleic acid lipid composition according to the embodiment of the present invention showed a higher EPO protein expression rate.

### <Measurement of EPO enzyme activity (dose dependency)>

The dispersion liquid of mRNA lipid nanoparticles prepared in above <Preparation of EPO mRNA-encapsulating lipid nanoparticles> was intravenously administered to ICR mice at a mRNA dosage of 0.1, 0.5, and 1 mg/kg. 6 hours after the administration, blood was collected from the caudal vena cava, thereby obtaining plasma. The human EPO enzyme activity was quantified by using the obtained blood plasma and Erythropoietin (EPO) Human Elisa Kit (Abcam.plc). The quantitative value is described as a relative amount of EPO protein in a case where the amount of EPO protein at the time of 0.1 mg/kg administration in Comparative Example is set to 1.

### The results are shown in Table 11 and Fig. 1.

In comparison with the nucleic acid lipid composition in Comparative Examples, the nucleic acid lipid composition according to the embodiment of the present invention showed to express the EPO protein in a dose-dependent manner.

**[Table 11]**

| Compounds used | Relative amount of EPO protein (with respect to Comparative Example 1 at 0.1 mg/kg) | | |
|---|---|---|---|
| | 0.1mg/kg | 0.5mg/kg | 1.0mg/kg |
| Comparative Example | 1.00 | 0.25 | 1.10 |
| Compound 3 | 1.91 | 17.26 | 27.22 |
| Compound 23 | 4.97 | 25.74 | 43.74 |
| Compound 24 | 4.65 | 26.81 | 45.73 |

## Claims

1. A compound represented by Formula (1) or a salt thereof, 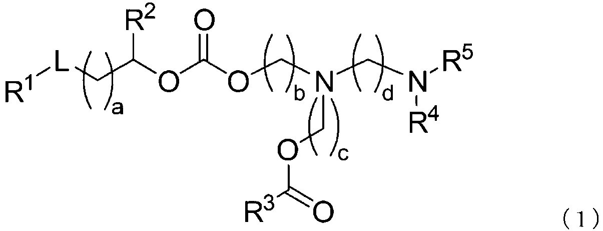
in the formula, R¹ represents a hydrocarbon group having 1 to 24 carbon atoms,
R² represents a hydrocarbon group having 1 to 6 carbon atoms,
R³ represents a hydrocarbon group having 6 to 24 carbon atoms and having a branched chain,
R⁴ and R⁵ each independently represent a hydrocarbon group having 1 to 6 carbon atoms, which may be substituted, and substituents on the hydrocarbon group having 1 to 6 carbon atoms, which may be substituted, represented by R⁴ and R⁵ each independently represent -O-R⁶, where R⁶ represents a hydrogen atom or a hydrocarbon group having 1 to 18 carbon atoms,
L is absent or represents *-O-C(O)- or *-C(O)O-, where * represents a bonding position to R¹,
a represents an integer of 1 to 12,
b represents an integer of 1 to 4,
c represents an integer of 1 to 4, and
d represents an integer of 1 to 4.

2. The compound or a salt thereof according to claim 1,
wherein R¹ represents a hydrocarbon group having 6 to 14 carbon atoms,
R² represents a hydrocarbon group having 6 carbon atoms,
R³ represents a hydrocarbon group having 11 to 17 carbon atoms and having a branched chain,
R⁴ and R⁵ each independently represent a hydrocarbon group having 2 to 4 carbon atoms which may be substituted, and substituents on the hydrocarbon group having 2 to 4 carbon atoms which may be substituted, represented by R⁴ and R⁵, each independently represent -O-R⁶, where R⁶ represents a hydrogen atom or a benzyl group,
L represents *-O-C(O)-, where * represents a bonding position to R¹,
a represents an integer of 3 to 8,
b represents 2,
c represents 2, and
d represents 2.

3. The compound or a salt thereof according to claim 2,
wherein R¹ represents a linear hydrocarbon group having 6 to 8 carbon atoms.

4. A compound or a salt thereof selected from the following compounds:
octyl 6-(2-((2-butyloctanoyl)oxy)ethyl)-3-ethyl-12-hexyl-10-oxo-9,11-dioxa-3,6-diazahenicosan-21 -oate 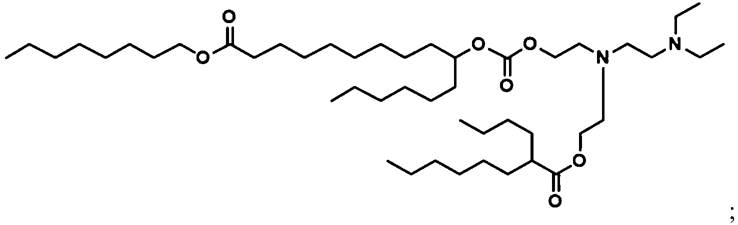
hexyl 3-ethyl-12-hexyl-6-(2-((2-hexyloctanoyl)oxy)ethyl)-10-oxo-9,11-dioxa-3,6-diazahenicosan-21 -oate 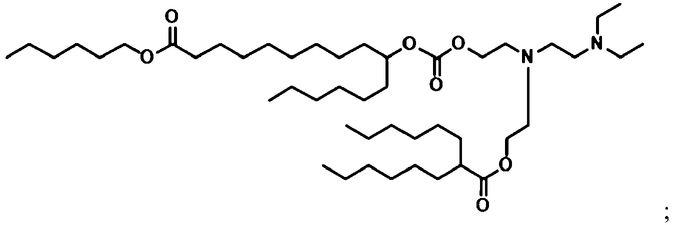
heptyl 3-ethyl-6-(2-((2-heptylnonanoyl)oxy)ethyl)-12-hexyl-10-oxo-9,11-dioxa-3,6-diazahexadecan-16-oate 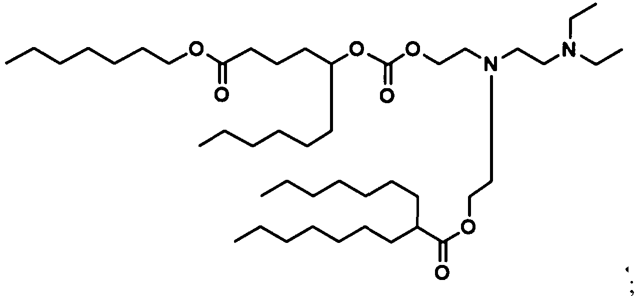
heptyl 3-ethyl-12-hexyl-6-(2-((2-octyldecanoyl)oxy)ethyl)-10-oxo-9,11-dioxa-3,6-diazahexadecan-1 6-oate 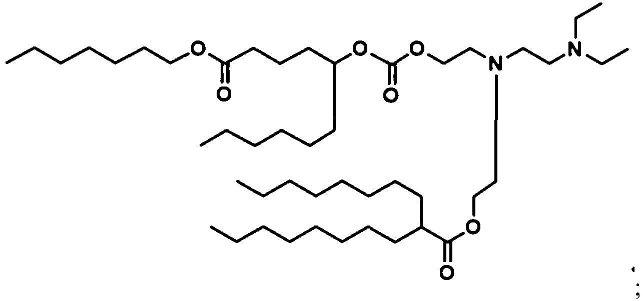
heptyl 3-ethyl-12-hexyl-6-(2-((2-hexyloctanoyl)oxy)ethyl)-10-oxo-9,11-dioxa-3,6-diazahexadecan-1 6-oate 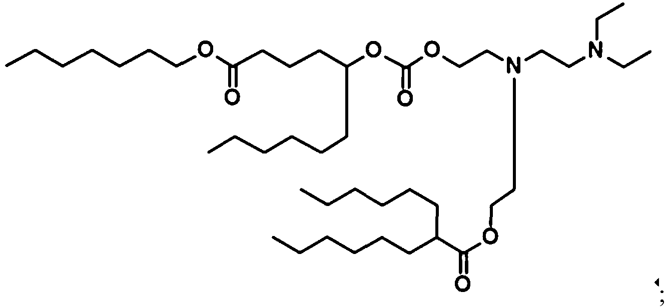
heptyl 3-ethyl-6-(2-((3-heptyldecanoyl)oxy)ethyl)-12-hexyl-10-oxo-9,11-dioxa-3,6-diazahexadecan-16-oate 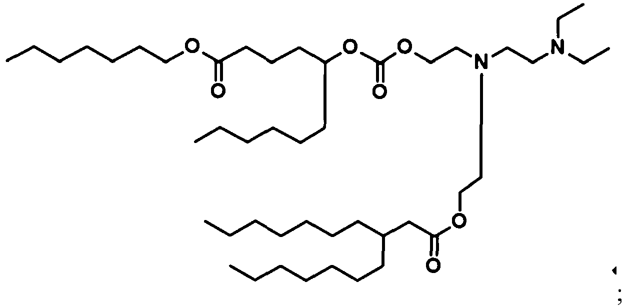
octyl 3-ethyl-6-(2-((2-heptylnonanoyl)oxy)ethyl)-12-hexyl-10-oxo-9,11-dioxa-3,6-diazahexadecan-16-oate 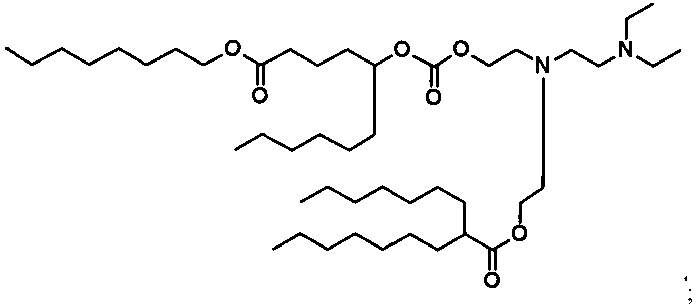
octyl 3-ethyl-6-(2-((3-heptyldecanoyl)oxy)ethyl)-12-hexyl-10-oxo-9,11-dioxa-3,6-diazahexadecan-16-oate 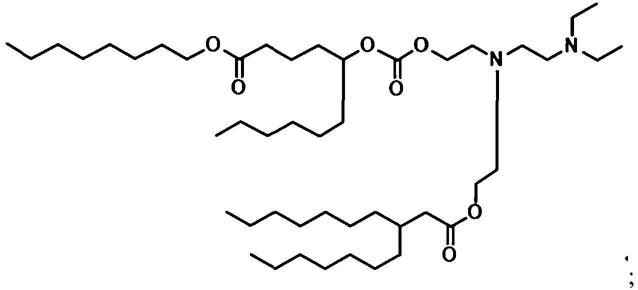
2-pentylheptyl 3-ethyl-6-(2-((3-heptyldecanoyl)oxy)ethyl)-12-hexyl-10-oxo-9,11-dioxa-3,6-diazahexadecan-16-oate 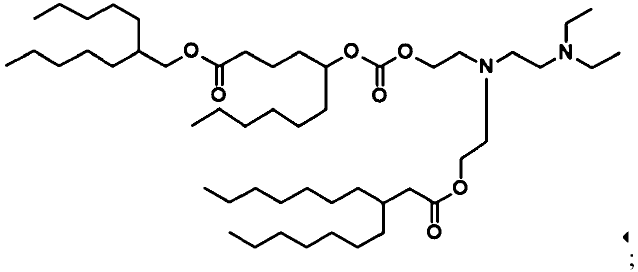
2-hexyloctyl 3-ethyl-6-(2-((3-heptyldecanoyl)oxy)ethyl)-12-hexyl-10-oxo-9,11-dioxa-3,6-diazahexadecan-16-oate 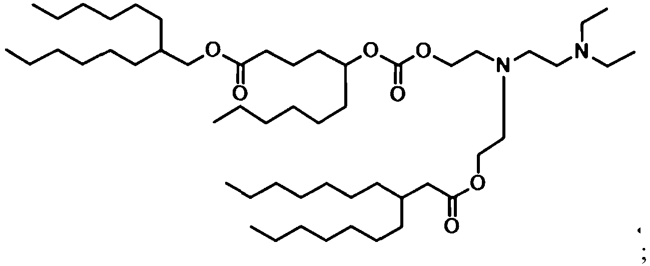
octyl 5-ethyl-8-(2-((3-heptyldecanoyl)oxy)ethyl)-14-hexyl-12-oxo-1-phenyl-2,11,13-trioxa-5,8-diaz aoctadecan-18-oate 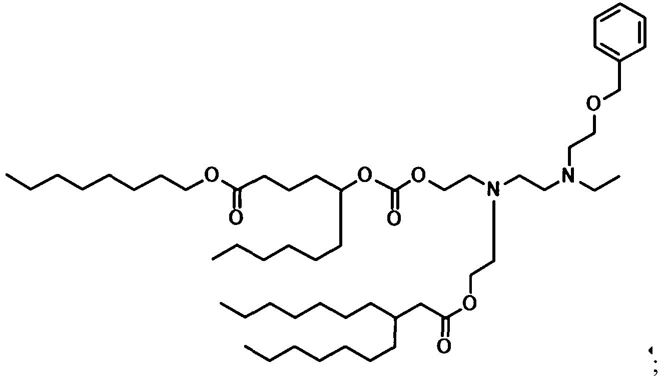
octyl 6-ethyl-9-(2-((3-heptyldecanoyl)oxy)ethyl)-15-hexyl-13-oxo-1-phenyl-2,12,14-trioxa-6,9-diaz anonadecan-19-oate 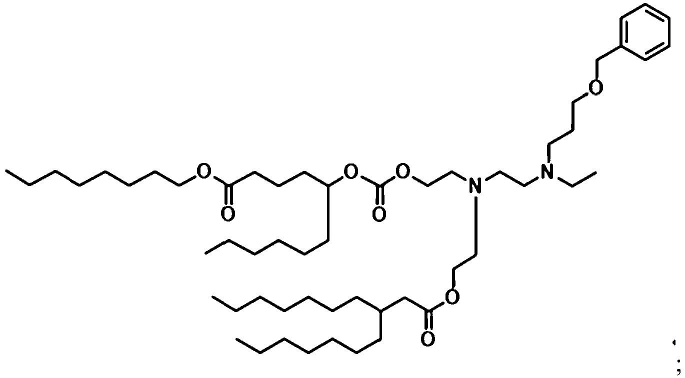
octyl 7-ethyl-10-(2-((3-heptyldecanoyl)oxy)ethyl)-16-hexyl-14-oxo-1-phenyl-2,13,15-trioxa-7,10-di azaicosan-20-oate 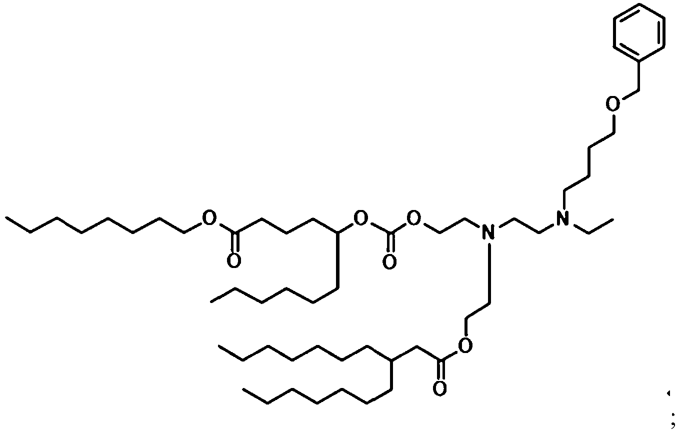
octyl 3-ethyl-6-(2-((3-heptyldecanoyl)oxy)ethyl)-12-hexyl-1-hydroxy-10-oxo-9,11-dioxa-3,6-diazah exadecan-16-oate 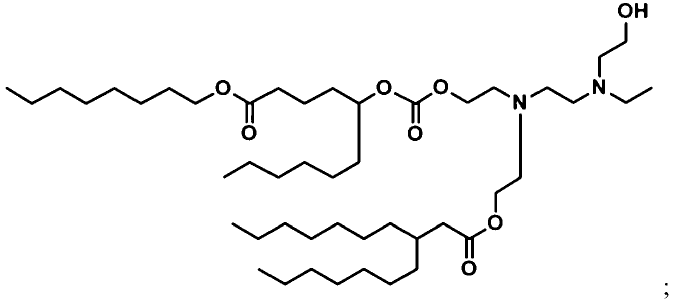
octyl 4-ethyl-7-(2-((3-heptyldecanoyl)oxy)ethyl)-13-hexyl-1-hydroxy-11-oxo-10,12-dioxa-4,7-diaza heptadecan-17-oate 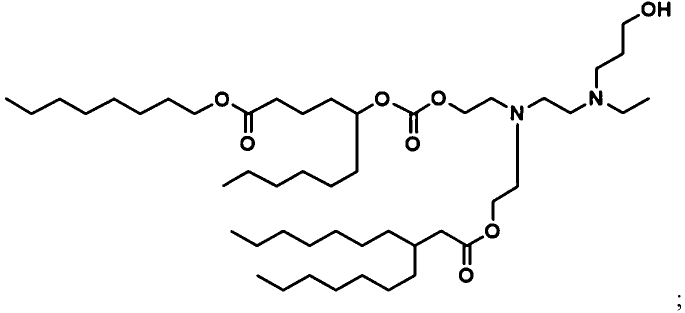
octyl 5-ethyl-8-(2-((3-heptyldecanoyl)oxy)ethyl)-14-hexyl-1-hydroxy-12-oxo-11,13-dioxa-5, 8-diaza octadecan-18-oate 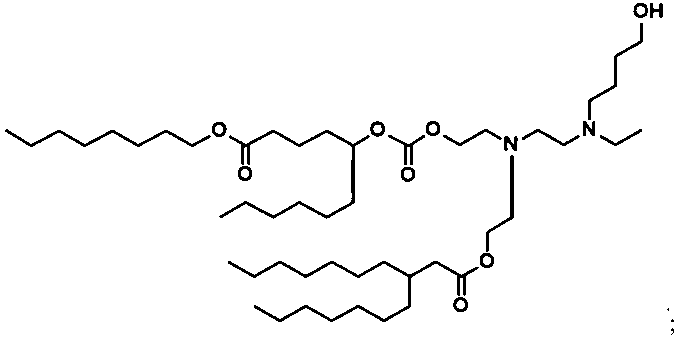
heptyl 3-ethyl-12-hexyl-6-(2-((2-nonylundecanoyl)oxy)ethyl)-10-oxo-9,11-dioxa-3,6-diazahexadecan -16-oate 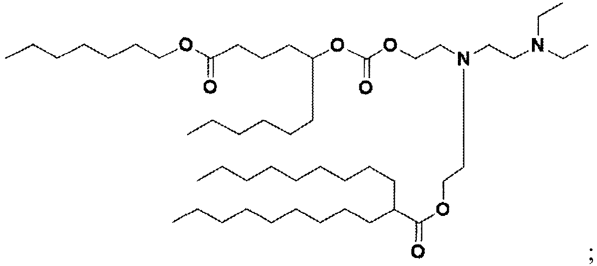
heptyl 6-(2-((2-decyldodecanoyl)oxy)ethyl)-3-ethyl-12-hexyl-10-oxo-9,11-dioxa-3,6-diazahexadecan -16-oate 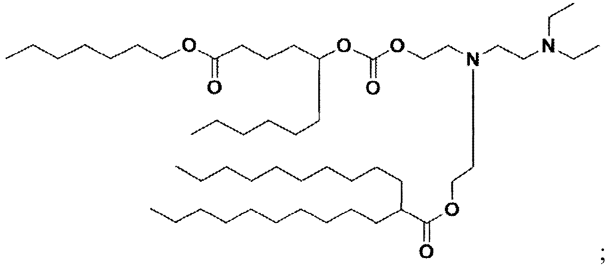
heptyl 3-ethyl-12-hexyl-6-(2-((2-hexyldecanoyl)oxy)ethyl)-10-oxo-9,11-dioxa-3,6-diazahexadecan-1 6-oate 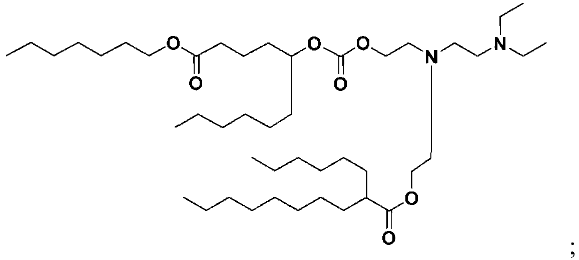
heptyl 3-ethyl-13-hexyl-7-(2-((2-hexyloctanoyl)oxy)ethyl)-11-oxo-10,12-dioxa-3,7-diazaheptadecan-17-oate 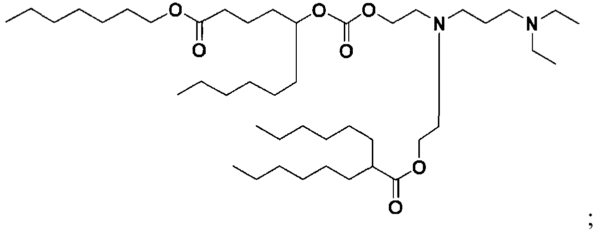
heptyl 3-ethyl-13-hexyl-7-(2-((2-hexyloctanoyl)oxy)ethyl)-11-oxo-10,12-dioxa-3,7-diazaoctadecan-1 8-oate 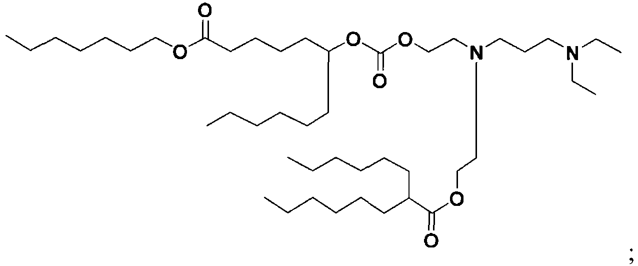
heptyl 3-ethyl-7-(2-((2-heptylnonanoyl)oxy)ethyl)-13-hexyl-11-oxo-10,12-dioxa-3,7-diazaoctadecan-18-oate 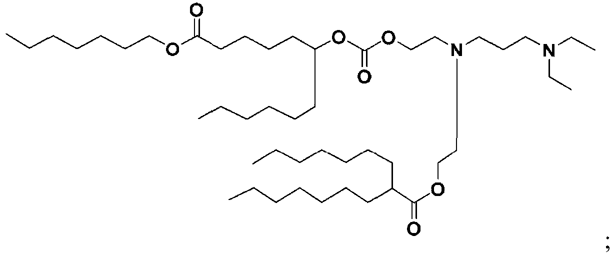
heptyl 3-ethyl-13-hexyl-7-(2-((2-octyldecanoyl)oxy)ethyl)-11-oxo-10,12-dioxa-3,7-diazaoctadecan-1 8-oate 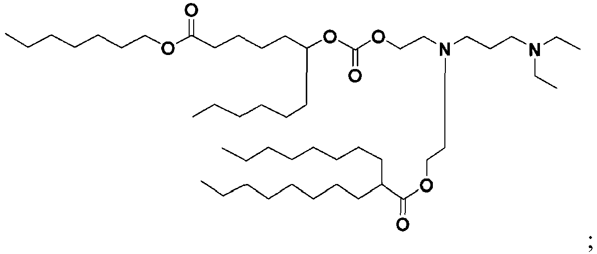
heptyl 3-ethyl-13-hexyl-7-(2-((2-hexyldecanoyl)oxy)ethyl)-11-oxo-10,12-dioxa-3,7-diazaoctadecan-18-oate 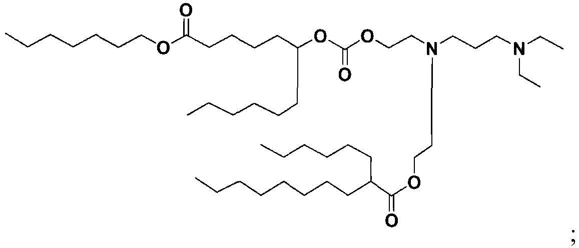
heptyl 3-ethyl-7-(2-((3-heptyldecanoyl)oxy)ethyl)-13-hexyl-11-oxo-10,12-dioxa-3,7-diazaoctadecan-18-oate 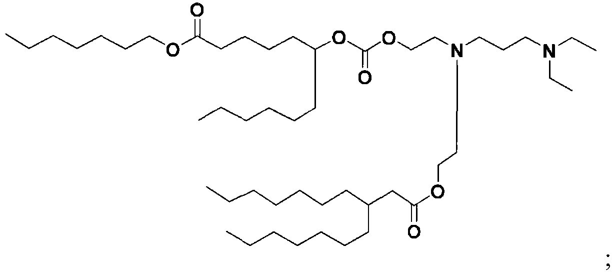
heptyl 3-ethyl-13-hexyl-6-(3-((2-hexyloctanoyl)oxy)propyl)-11-oxo-10,12-dioxa-3,6-diazaoctadecan-18-oate 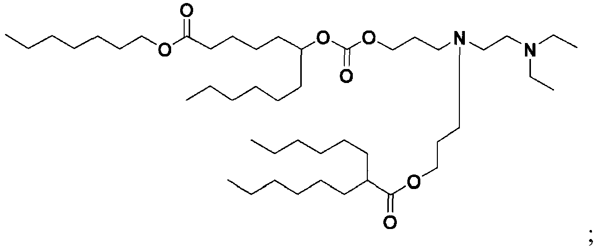
heptyl 3-ethyl-6-(3-((2-heptylnonanoyl)oxy)propyl)-13-hexyl-11-oxo-10,12-dioxa-3,6-diazaoctadeca n-18-oate 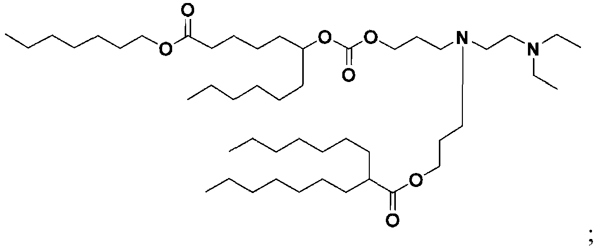
heptyl 3-ethyl-13-hexyl-6-(3-((2-octyldecanoyl)oxy)propyl)-11-oxo-10,12-dioxa-3,6-diazaoctadecan-18-oate 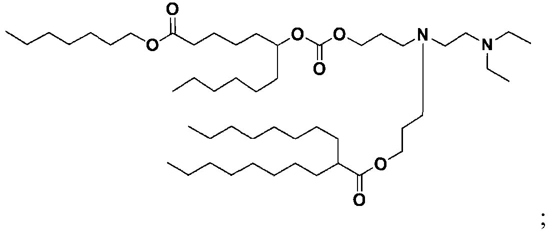
heptyl 3-ethyl-13-hexyl-6-(3-((2-nonylundecanoyl)oxy)propyl)-11-oxo-10,12-dioxa-3,6-diazaoctadec an-18-oate 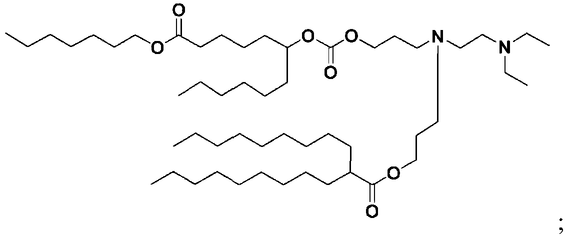
heptyl 3-ethyl-13-hexyl-6-(3-((2-hexyldecanoyl)oxy)propyl)-11-oxo-10,12-dioxa-3,6-diazaoctadecan -18-oate 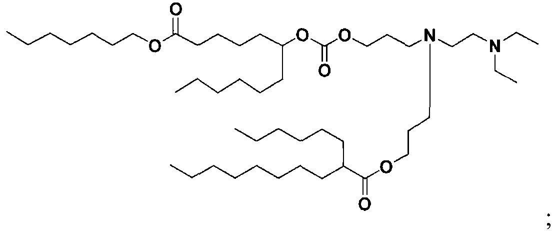
heptyl 3-ethyl-6-(3-((3-heptyldecanoyl)oxy)propyl)-13-hexyl-11-oxo-10,12-dioxa-3,6-diazaoctadeca n-18-oate 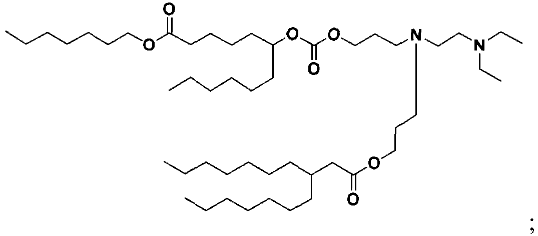
heptyl 3-ethyl-14-hexyl-7-(3-((2-hexyloctanoyl)oxy)propyl)-12-oxo-11,13-dioxa-3,7-diazanonadecan -19-oate 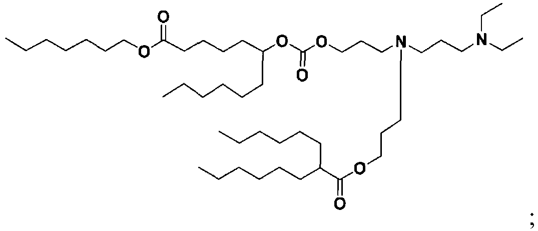
heptyl 3-ethyl-7-(3-((2-heptylnonanoyl)oxy)propyl)-14-hexyl-12-oxo-11,13-dioxa-3,7-diazanonadeca n-19-oate 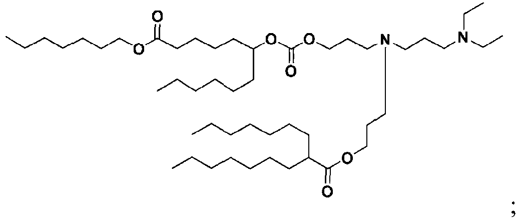
heptyl 3-ethyl-14-hexyl-7-(3-((2-octyldecanoyl)oxy)propyl)-12-oxo-11,13-dioxa-3,7-diazanonadecan -19-oate 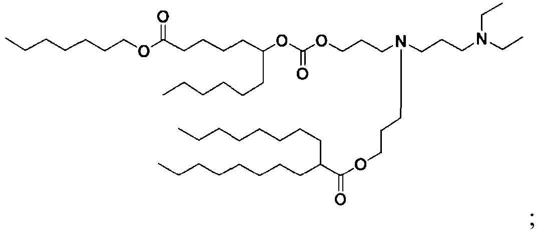
heptyl 3-ethyl-14-hexyl-7-(3-((2-nonylundecanoyl)oxy)propyl)-12-oxo-11,13-dioxa-3,7-diazanonade can-19-oate 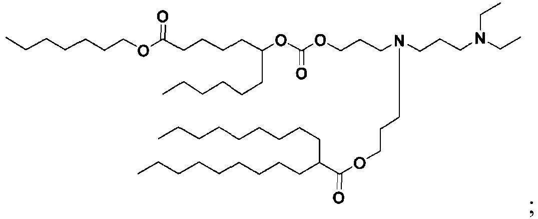
heptyl 3-ethyl-14-hexyl-7-(3-((2-hexyldecanoyl)oxy)propyl)-12-oxo-11,13-dioxa-3,7-diazanonadeca n-19-oate 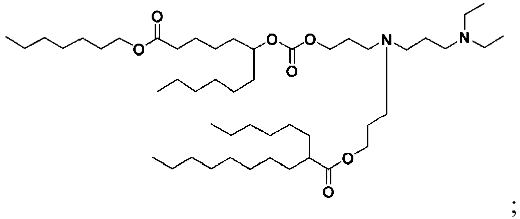 and
heptyl 3-ethyl-7-(3-((3-heptyldecanoyl)oxy)propyl)-14-hexyl-12-oxo-11,13-dioxa-3,7-diazanonadeca n-19-oate

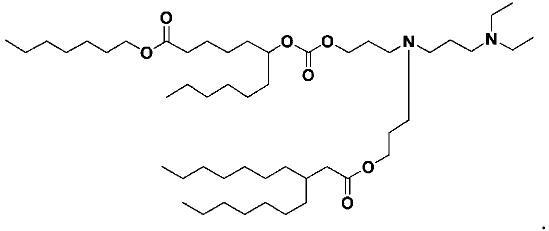

5. A lipid composition comprising:
the compound or a salt thereof according to any one of claims 1 to 4; and
a lipid.

6. The lipid composition according to claim 5,
wherein the lipid is at least one lipid selected from the group consisting of a neutral lipid and a lipid having a nonionic hydrophilic polymer chain.

7. The lipid composition according to claim 5 or 6, further comprising:
a sterol.

8. The lipid composition according to any one of claims 5 to 7, further comprising:
at least one selected from the group consisting of a nucleic acid, a protein, a peptide, and a low-molecular-weight compound.

9. A pharmaceutical composition comprising:
the lipid composition according to any one of claims 5 to 8.

10. A delivery carrier comprising:
the lipid composition according to any one of claims 5 to 8.
